(19) Europäisches Patentamt
European Patent Office
Office européen des brevets

(11) EP 4 755 400 A1

(12) EUROPEAN PATENT APPLICATION
published in accordance with Art. 153(4) EPC

(43) Date of publication:
**10.06.2026 Bulletin 2026/24**

(21) Application number: **24847809.1**

(22) Date of filing: **13.06.2024**

(51) International Patent Classification (IPC):
***A61K 47/68*** *(2017.01)* ***A61P 35/00*** *(2006.01)*

(52) Cooperative Patent Classification (CPC):
**A61K 45/00; A61K 47/54; A61K 47/68; A61P 29/00; A61P 35/00; A61P 37/02; C07H 1/00; C07H 17/02; C07K 16/28**

(86) International application number:
**PCT/CN2024/098950**

(87) International publication number:
**WO 2025/025846 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.08.2023 CN 202310961666**

(71) Applicant: **Novocodex Biopharmaceuticals Co., Ltd.**
**Shaoxing, Zhejiang 312366 (CN)**

(72) Inventors:
- **HAN, Nianhe**
  **Shanghai 201203 (CN)**
- **AN, Deqiang**
  **Shanghai 201203 (CN)**
- **LI, Jianshi**
  **Shanghai 201203 (CN)**
- **MIAO, Zhongyi**
  **Shanghai 201203 (CN)**
- **SONG, Liwei**
  **Shanghai 201203 (CN)**
- **ZHU, Hongju**
  **Shanghai 201203 (CN)**

(74) Representative: **Studio Torta S.p.A.**
**Via Viotti, 9**
**10121 Torino (IT)**

(54) **ANTIBODY-DRUG CONJUGATE CONTAINING GLYCOSYLPYRIDINE LINKER**

(57) An antibody-drug conjugate containing a glycosylpyridine linker. Specifically, the present invention relates to a glycosylpyridine linker, and an antibody-drug conjugate containing the glycosylpyridine linker, a preparation method therefor, and a medical use thereof, particularly a use in the preparation of a drug for treating cancers, autoimmune diseases, and inflammatory diseases. By means of the specific glycosylpyridine linker, the hydrophilicity of the antibody-drug conjugate is enhanced, and the antibody-drug conjugate is controlled to release an active drug under the intracellular enzymatic action.

EP 4 755 400 A1

## Description

### FIELD OF THE INVENTION

The present invention relates to a novel glycosylpyridine linker and an antibody-drug conjugate containing the same, as well as a method for preparing the same and a use thereof in the preparation of a medicament for treating cancer, autoimmune diseases, and inflammatory diseases.

### BACKGROUND OF THE INVENTION

Antibody-Drug Conjugates (ADCs) are a class of novel targeted therapeutic drugs, mainly applied in therapeutic fields such as cancer and autoimmunity. Their basic design concept originated from the "magic bullet" and "drug targeting" concepts first proposed by Paul Ehrlich in 1913, which involve delivering drugs to affected area via appropriate carriers. However, restricted by antibody and highly active cytotoxic drug technologies, it was not until 2000 that the first antibody-drug conjugate (Mylotarg™) for the treatment of acute myeloid leukemia (AML) was approved by the FDA for marketing. To date, 12 types of ADC drugs have been successively approved for marketing, indicating that the application of antibody-drug conjugates in the field of tumor therapy has entered a stage of rapid development.

Antibody-drug conjugates typically consist of three parts of an antibody or other type of ligand, a highly active cytotoxic drug, and a linker that conjugates the antibody with the cytotoxic drug. Their mechanism of action is as follows. The antibody specifically recognizes and binds to a cell surface antigen; the formed conjugate enters the cell via endocytosis, simultaneously carrying the highly active cytotoxic drug into the cell; the antibody is enzymatically degraded or the linker itself cleaves, and the highly active cytotoxic drug is released in an appropriate form to kill the target cell.

Based on the release mechanism of the cytotoxic drug within the cell, linkers can be divided into non-cleavable linkers and cleavable linkers. The vast majority of currently marketed ADC drugs employ cleavable linkers. According to the cleavage mechanism, cleavable linkers can be further divided into the following main types of acid cleavage mechanism linkers (e.g., hydrazones), cysteine/glutathione cleavage mechanism linkers (e.g., disulfides), and enzyme cleavage mechanism linkers (e.g., peptides). Among them, enzyme cleavage mechanism linkers are favored for their stability and specificity of the cleavage mechanism.

Cytotoxic drugs possess certain hydrophobicity, and conjugating them to an antibody typically results in increased hydrophobicity of the antibody-drug conjugate (relative to the antibody). This increased hydrophobicity not only easily leads to ADC molecular aggregation (formation of multimers), but also causes accelerated clearance of the ADC *in vivo.* To reduce the hydrophobicity of ADC drugs, hydrophilic linkers are usually employed to reduce or offset the adverse effects brought by the hydrophobicity of cytotoxic drugs on ADC drugs.

To meet the development of the ADC drug, there is an urgent need to develop linkers with novel structures, cleavage mechanisms, and hydrophilicity, thereby constructing ADC drugs with better developability.

### SUMMARY OF THE INVENTION

The present invention aims to provide a novel glycosylpyridine linker and to construct antibody-drug conjugates based on this linker.

After years of in-depth research, the inventors have developed a novel glycosylpyridine linker. The ADC drug constructed with this linker, after binding to a cell surface antigen and entering into the cell by endocytosis, can be degraded under the action of beta-glucuronidase and release the cytotoxic drug, achieving the effect of killing the cells. This linker possesses good chemical stability and will not cleave during *in vivo* circulation, thereby avoiding increased off-target toxicity. Simultaneously, this linker has good hydrophilicity, which can greatly improve the hydrophilicity of the ADC drug and the concomitant *in vivo* metabolic stability. This linker technology is universal and can be applied to the vast majority of ADC drugs, thus possessing broad prospects.

Specifically, the present invention provides an antibody-drug conjugate comprising a "glycosylpyridine linker", wherein the glycosylpyridine linker comprises a glycosyl (Su) substituted pyridine group. The pyridine group possesses two conjugatable groups, one of which can be conjugated to an antibody or other type of ligand via other groups or directly, and the other can be connected to a cytotoxic drug via other groups or directly (as shown in the formula below).

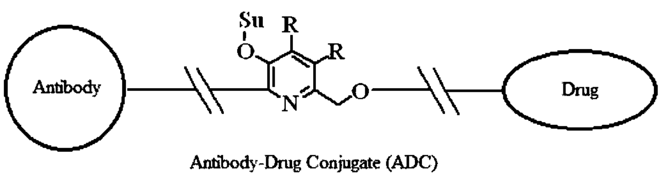

Antibody-Drug Conjugate (ADC)

The obtained antibody-drug conjugate can be used to deliver the drugs to the target cell populations, such as tumor cells. The antibody-drug conjugate can specifically bind to cell surface proteins, and the obtained conjugate is subsequently endocytosed by the cell. In the cell, glycoside hydrolases (GH) selectively hydrolyze the Su-O group to remove the sugar molecule. The residual oxyanion on the pyridine ring undergoes electron rearrangement with the pyridine ring, releasing the drug connected to the para-position in the form of an active drug to produce efficacy (see the reaction scheme below).

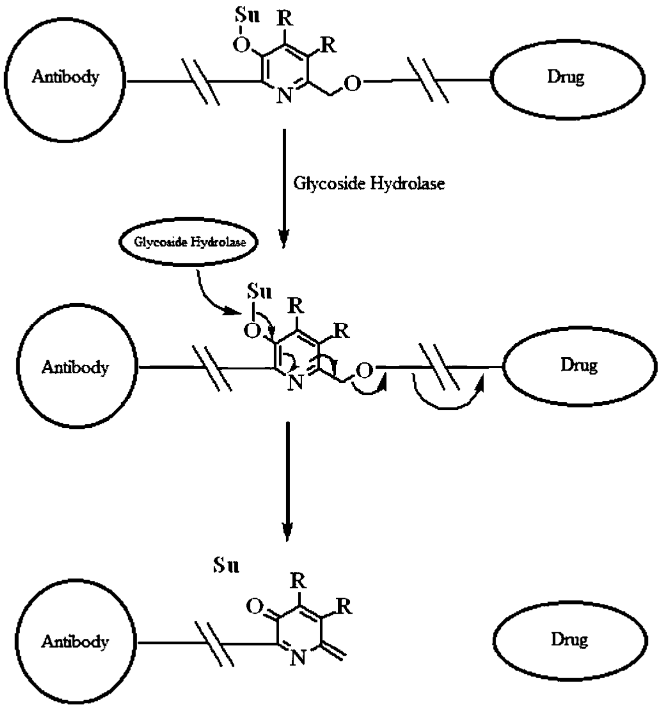


Antibodies for constructing ADC drugs include chimeric antibodies, humanized antibodies, human antibodies; antibody fragments capable of binding to antigens; antibody Fc fusion proteins; proteins; or other types of ligands. The drug is a highly active drug, including but not limited to Maytansinoids, Auristatins, Calicheamicins, Doxorubicins, Duocarmycins and CC-1065, Pyrrolobenzodiazepine dimers (PBD dimers), Camptothecins, Tubulysins, and Amanitins.

Compared with traditional antibody-drug conjugates, the antibody-drug conjugates prepared by the method of the present invention have increased hydrophilicity, and the *in vivo* circulation stability is improved to a great extent, thereby substantially enhancing the developability of the product.

Therefore, an object of the present invention is to provide an antibody-drug conjugate of formula I,

$$L\left(A\left(G_g-W_w-D\right)_p\right)_q \quad \text{I}$$

or a pharmaceutically acceptable salt or hydrate thereof,
wherein,
L is an antibody, an antibody fragment, or another type of ligand;
A is a linker fragment, connected to L and G simultaneously;
G is a glycosyl-substituted pyridine, as shown in formula II:

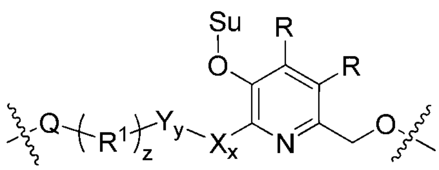
II

wherein,
Su is a sugar molecule;
each R is independently selected from the group consisting of hydrogen, CN, halogen, $NO_2$ and $C_1$-$C_4$ alkyl;

X and Y are each independently selected from the group consisting of $-CR^2R^3-$, $-C(=O)NR^4-$, $-NR^5C(=O)-$ and -O-;
Q is selected from the group consisting of $-NR^6-$ and -O-;
$R^1$ is selected from the group consisting of $C_1$-$C_6$ alkylene, and $C_1$-$C_6$ alkylene containing O in the skeleton;
$R^2$, $R^3$, $R^4$, $R^5$, and $R^6$ are each independently selected from the group consisting of hydrogen and $C_1$-$C_4$ alkyl;
x, y, and z are each independently selected from the group consisting of 0 and 1;
g is selected from the group consisting of 1 and 2;
W is an optional self-immolative linker;
w is selected from the group consisting of 0 and 1;
D is a drug;
p is an integer selected from the group consisting of 1 to 4;
q is an integer selected from the group consisting of 1 to 8.

In an embodiment, the antibody-drug conjugate of formula I according to the present invention is an antibody-drug conjugate of formula III:

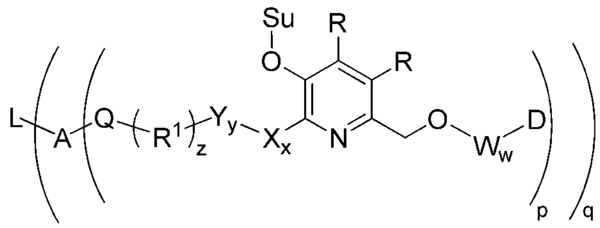

III

wherein, L, A, Q, $R^1$, R, X, Y, W, D, Su, x, y, z, and w are as defined in formula I.

In another embodiment, the antibody-drug conjugate of formula I according to the present invention is an antibody-drug conjugate of formula IV or formula V:

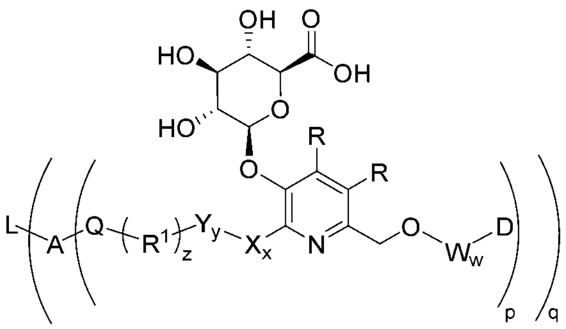

IV

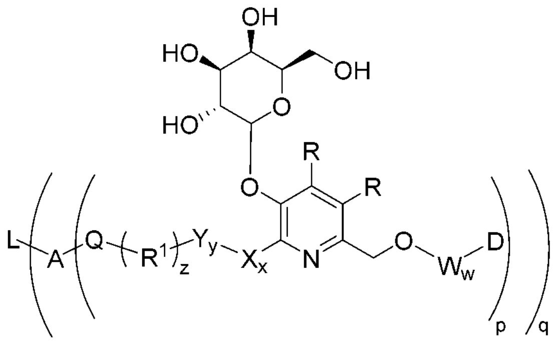

V

wherein, L, A, Q, $R^1$, R, X, Y, W, D, x, y, z, and w are as defined in formula I.

In another embodiment, the antibody-drug conjugate of formula I according to the present invention is an antibody-drug conjugate of formula VI or formula VII:

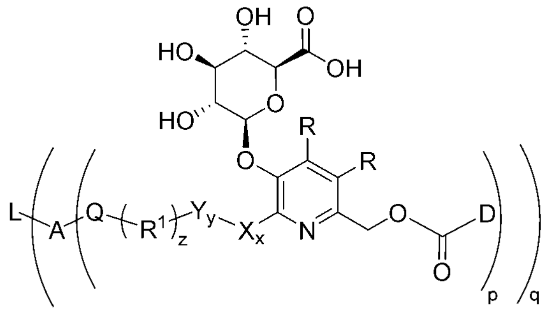

VI

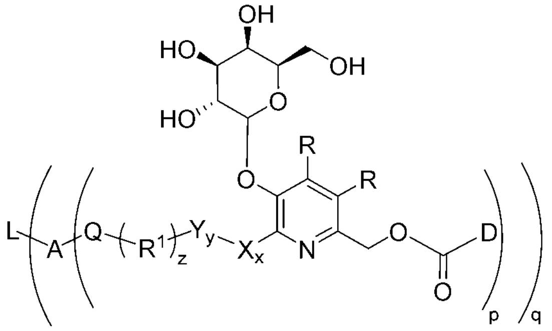

VII

wherein, L, A, Q, $R^1$, R, X, Y, D, x, y, and z are as defined in formula I.

In a preferred embodiment, in the formula I, III, IV, V, VI, or VII according to the present invention,

x is 0;
y is 1;
Y is selected from the group consisting of -C(=O)$NR^4$-, -$NR^5$C(=O)-, and -O-;
$R^4$ and $R^5$ are each independently selected from the group consisting of hydrogen and $C_1$-$C_4$ alkyl.

In another preferred embodiment, in the formula I, III, IV, V, VI, or VII according to the present invention, x is 1;

y is 1;
X is -$CR^2R^3$-;
Y is selected from the group consisting of -C(=O)$NR^4$-, -$NR^5$C(=O)-, and -O-;
$R^2$ and $R^3$ are each independently selected from the group consisting of hydrogen and $C_1$-$C_4$ alkyl, preferably hydrogen;
$R^4$ and $R^5$ are each independently selected from the group consisting of hydrogen and $C_1$-$C_4$ alkyl.

In another preferred embodiment, in the formula I, III, IV, V, VI, or VII according to the present invention, $R^1$ is $C_1$-$C_6$ alkylene, preferably $C_1$-$C_6$ linear alkylene, more preferably $C_2$-$C_4$ linear alkylene; z is 1.

In another preferred embodiment, in the formulae I, III, IV, V, VI, or VII according to the present invention, Q is -$NR^6$-; $R^6$ is each independently selected from the group consisting of hydrogen and $C_1$-$C_4$ alkyl.

In another preferred embodiment, in the formula I, III, IV, V, VI, or VII according to the present invention, each R is independently selected from the group consisting of hydrogen, halogen, and $C_1$-$C_4$ alkyl, preferably hydrogen.

In another preferred embodiment, in the formula I, III, IV, V, VI, or VII according to the present invention, G has the structure shown below:

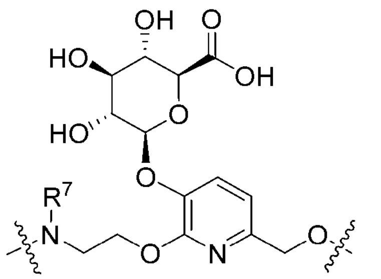

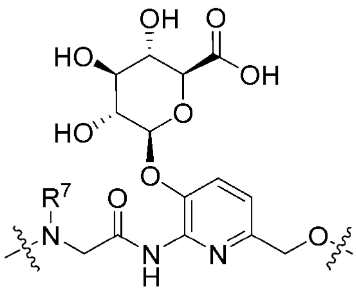

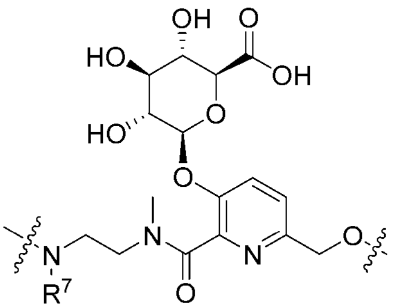

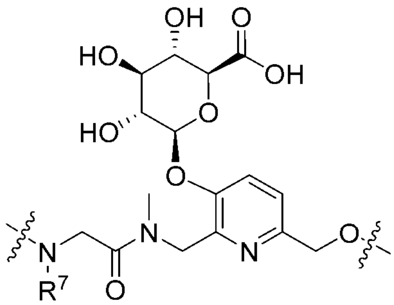

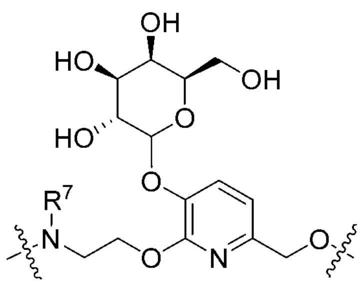

wherein, $R^7$ is selected from the group consisting of hydrogen, $C_{1-4}$ alkyl such as methyl, and a water-soluble group such as polyethylene glycol.

In another preferred embodiment, in the formula I, III, IV, V, VI, or VII according to the present invention, g is 1.

In another preferred embodiment, in the formula I, III, IV, V, VI, or VII according to the present invention, A is a linker covalently connected to both the antibody and G simultaneously.

In another preferred embodiment, in the formula I, III, IV, V, VI, or VII according to the present invention, A is selected from the group consisting of the following structures:

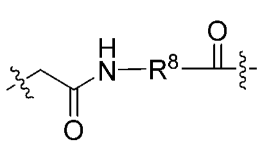
,
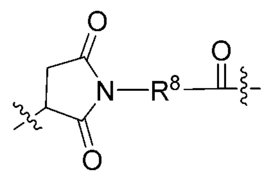
,
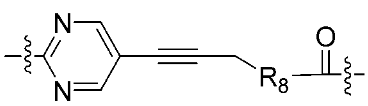
,
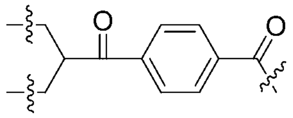
,
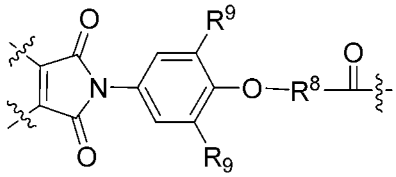
,
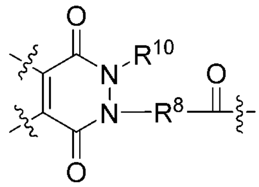
,

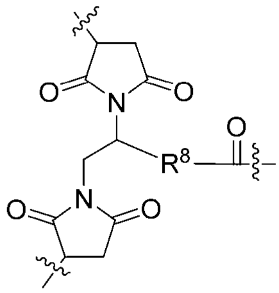,
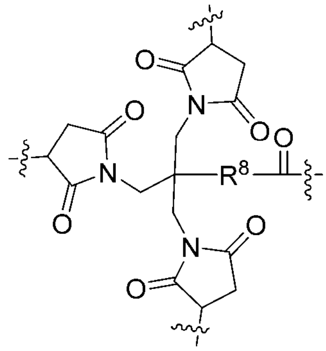,
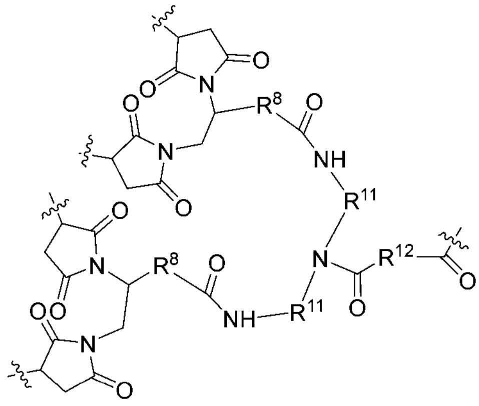

wherein, $R^8$ is selected from the group consisting of $C_1$-$C_6$ alkylene, and $C_1$-$C_6$ alkylene containing O in the skeleton;
$R^9$ is selected from the group consisting of H and halogen;
$R^{10}$ is $C_1$-$C_4$ alkyl;
$R^{11}$ and $R^{12}$ are each independently selected from the group consisting of $C_1$-$C_6$ alkylene.

In another preferred embodiment, in the formula I, III, IV, V, VI, or VII according to the present invention, L is a ligand such as humanized antibody, chimeric antibody, or fully human antibody or antibody fragment, antibody Fc fusion protein.

In another preferred embodiment, in the formula I, III, IV, V, VI, or VII according to the present invention, L is a ligand such as an antibody, antibody fragment, or antibody Fc fusion protein, against a cell surface receptor or tumor-associated antigen.

In another preferred embodiment, in the formula I, III, IV, V, VI, or VII according to the present invention, L is a humanized anti-CD30 monoclonal antibody or a humanized anti-CD79b monoclonal antibody.

In another preferred embodiment, in the formula I, III, IV, V, VI, or VII according to the present invention, L is Ab1, which has a heavy chain shown in SEQ ID NO: 1 and a light chain shown in SEQ ID NO: 2.

In another preferred embodiment, in the formula I, III, IV, V, VI, or VII according to the present invention, L is Ab2, which has a heavy chain shown in SEQ ID NO: 3 and a light chain shown in SEQ ID NO: 4.

In another preferred embodiment, in the formula I, III, IV, V, VI, or VII according to the present invention, D is an antitumor drug, a drug for treating autoimmune diseases, or an anti-inflammatory drug; said antitumor drug is, for example, a tubulin inhibitor, a DNA alkylating agent, a DNA minor groove binder, or a DNA topoisomerase inhibitor.

In another preferred embodiment, in the formula I, III, IV, V, VI, or VII according to the present invention, D is Monomethyl Auristatin E (MMAE), SN-38, or Exatecan.

Another object of the present invention is to provide a compound of formula (A) or a pharmaceutically acceptable salt thereof,

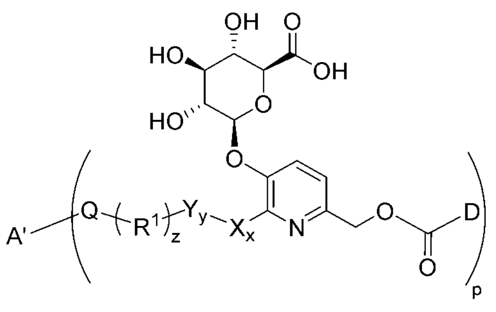

(A)

wherein,

A' is a linker fragment;
each R is independently selected from the group consisting of hydrogen, CN, halogen, $NO_2$ and $C_1$-$C_4$ alkyl, preferably hydrogen, halogen, $C_1$-$C_4$ alkyl, more preferably hydrogen;
X and Y are each independently selected from the group consisting of $-CR^2R^3-$, $-C(=O)NR^4-$, $-NR^5C(=O)-$ and $-O-$;

Q is selected from the group consisting of $-NR^6-$ and -O-, preferably $-NR^6-$;
$R^1$ is selected from the group consisting of $C_1$-$C_6$ alkylene and $C_1$-$C_6$ alkylene containing O in the skeleton, preferably $C_1$-$C_6$ alkylene;
$R^2$ and $R^3$ are each independently selected from the group consisting of hydrogen and $C_1$-$C_4$ alkyl, preferably hydrogen;
$R^4$ and $R^5$ are each independently selected from the group consisting of hydrogen and $C_1$-$C_4$ alkyl;
$R^6$ is selected from the group consisting of hydrogen and $C_1$-$C_4$ alkyl;
x is 0 or 1;
y is 0 or 1, preferably 1;
z is 0 or 1, preferably 1;
D is a drug molecule;
p is an integer selected from the group consisting of 1 to 4, preferably 1 or 2.

In a preferred embodiment, in the compound of formula (A) or the pharmaceutically acceptable salt thereof according to the present invention, A' is selected from the group consisting of the following structures:

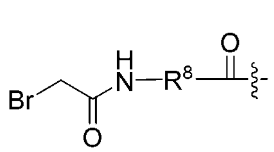
,
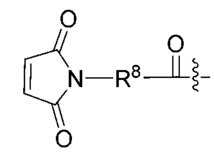
,
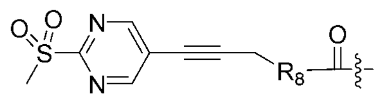
,

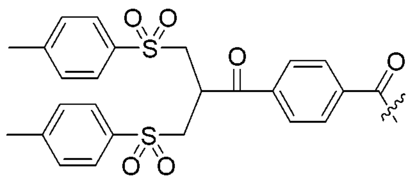
,
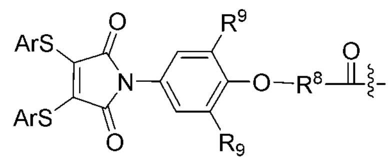
,
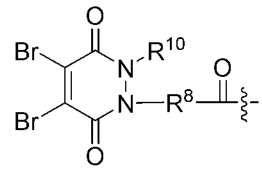
,

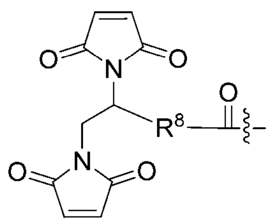
,
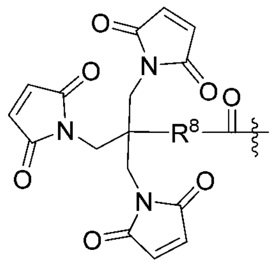
,
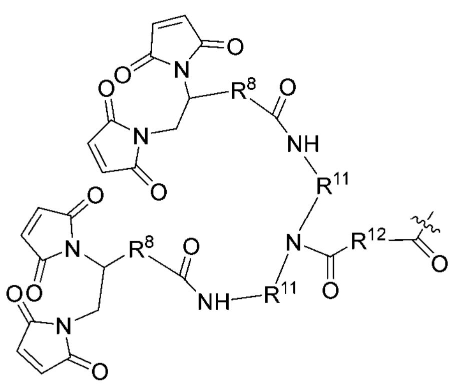

wherein,

$R^8$ is selected from the group consisting of $C_1$-$C_6$ alkylene and $C_1$-$C_6$ alkylene containing O in the skeleton;
$R^9$ is selected from the group consisting of H and halogen;
$R^{10}$ is selected from the group consisting of $C_1$-$C_4$ alkyl;
$R^{11}$ and $R^{12}$ are each independently selected from the group consisting of $C_1$-$C_6$ alkylene.

In another preferred embodiment, the compound of formula (A) or the pharmaceutically acceptable salt thereof according to the present invention is selected from the group consisting of the following structures:

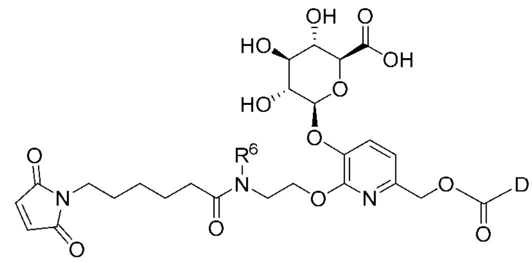

,

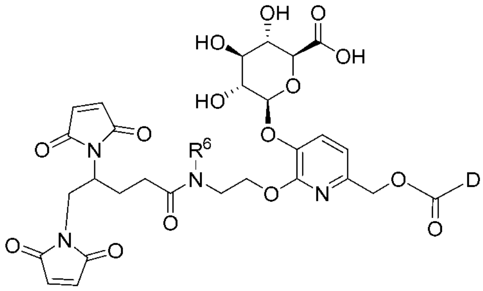

,

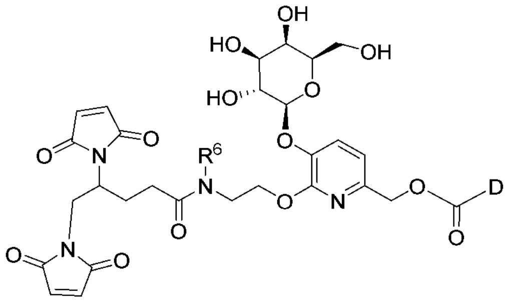

,

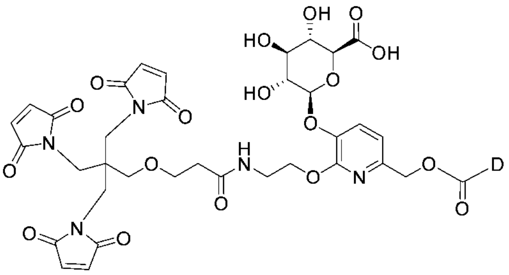

,

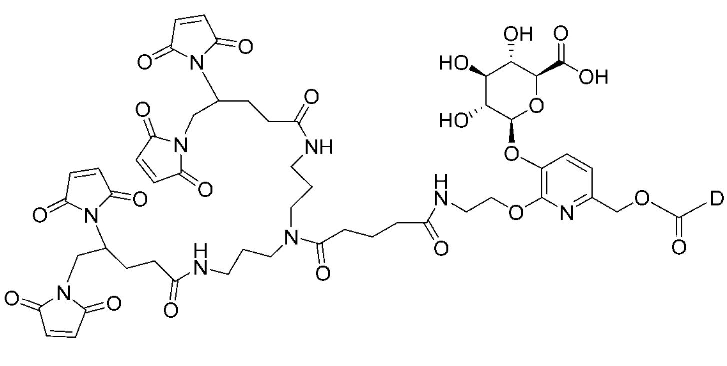

,

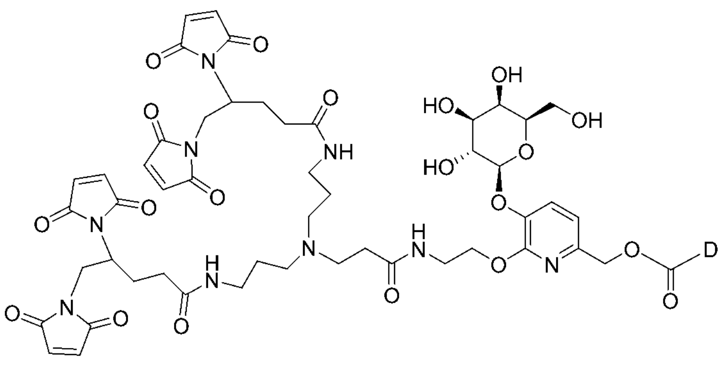

,

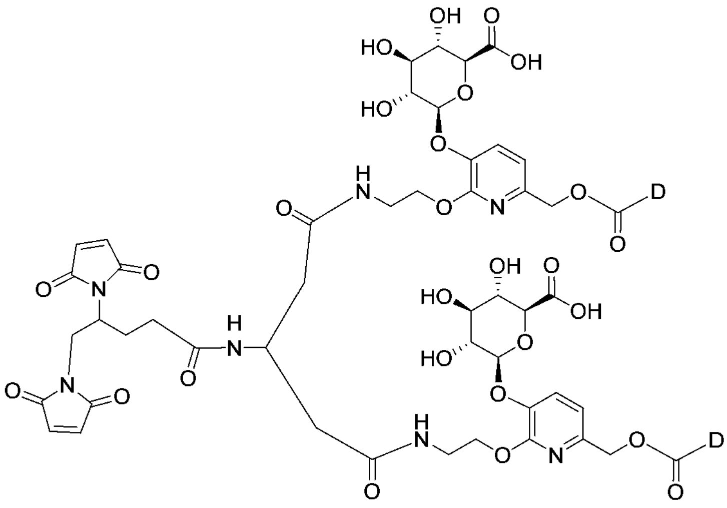

,

wherein, $R^5$, $R^6$ and D are as defined in formula (A).

In another preferred embodiment, in the compound of formula (A) or the pharmaceutically acceptable salt thereof according to the present invention, D is an antitumor drug, a drug for treating autoimmune diseases, or an anti-inflammatory drug, the antitumor drug is, for example, a tubulin inhibitor, a DNA alkylating agent, a DNA minor groove binder, or a DNA topoisomerase inhibitor; preferably, D is Monomethyl Auristatin E (MMAE), SN-38, or Exatecan.

In another preferred embodiment, the compound of formula (A) or the pharmaceutically acceptable salt thereof according to the present invention is selected from the group consisting of the following structures:

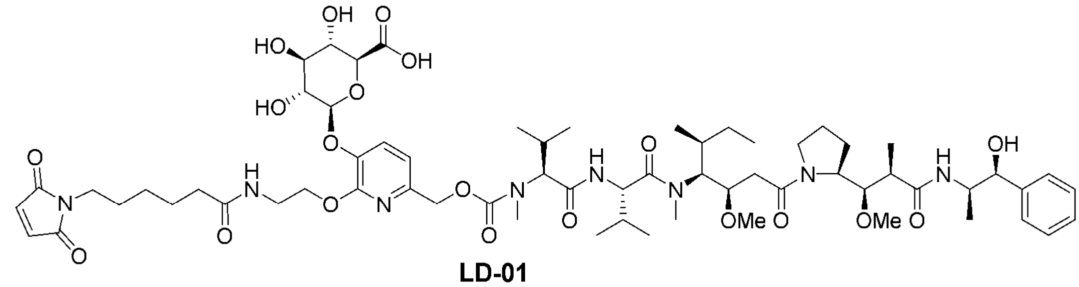

**LD-01**

,

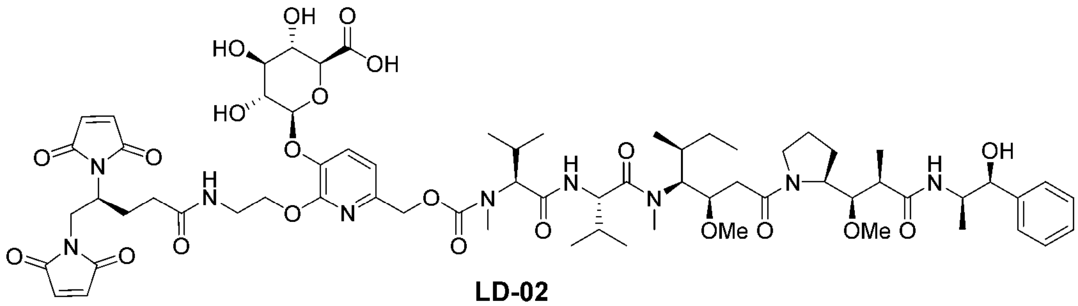

**LD-02**

,

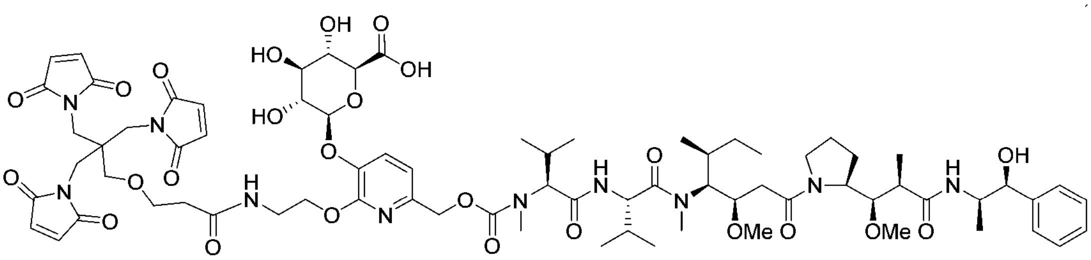

**LD-03**

,

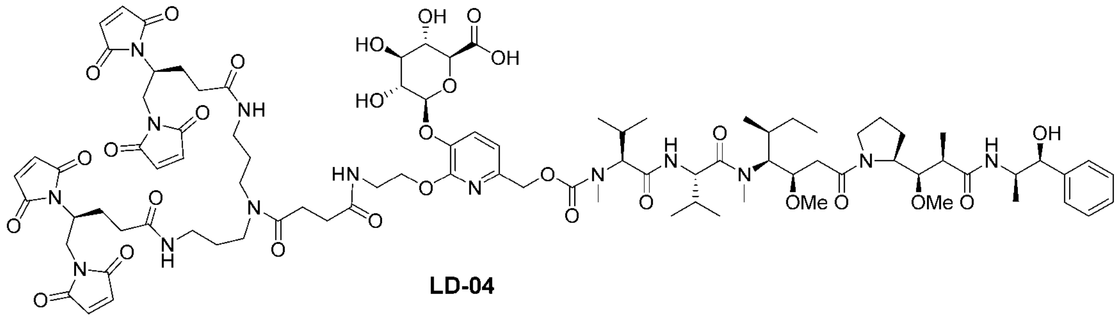

LD-04

,

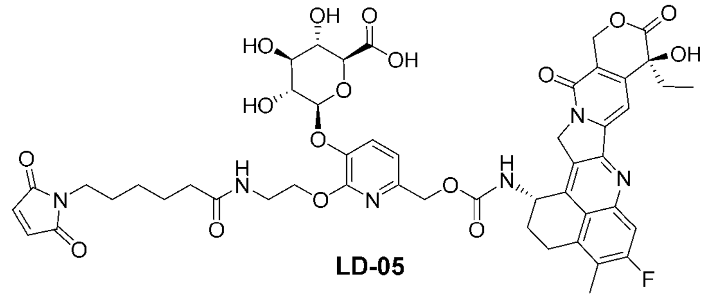

LD-05

,

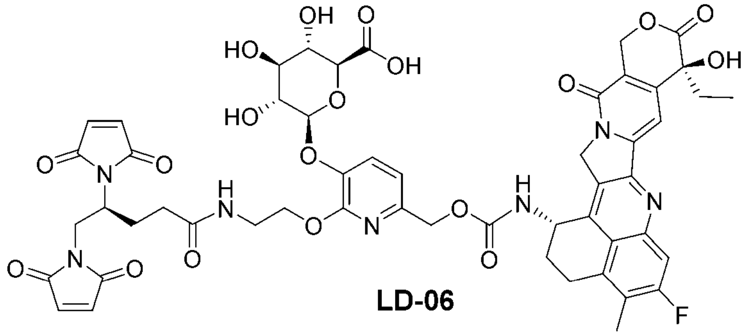

LD-06

,

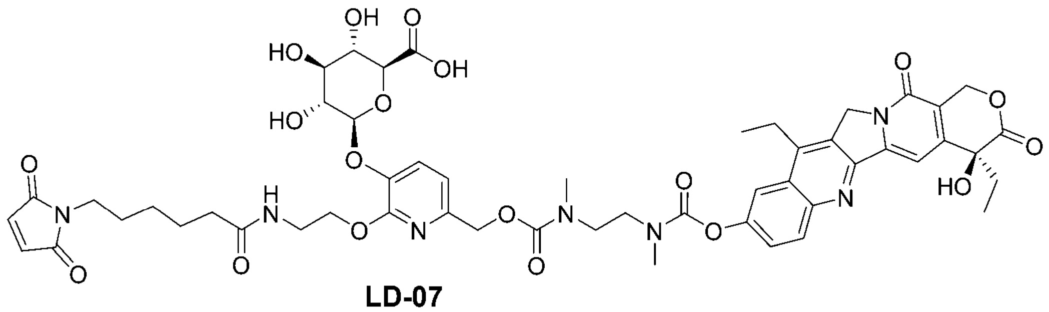

LD-07

,

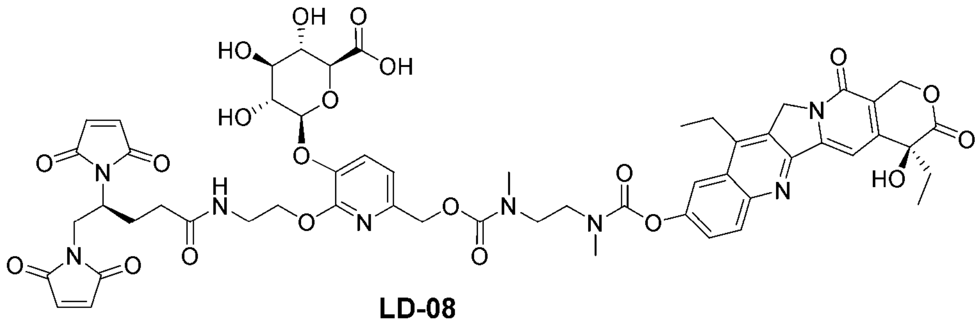

**LD-08**

,

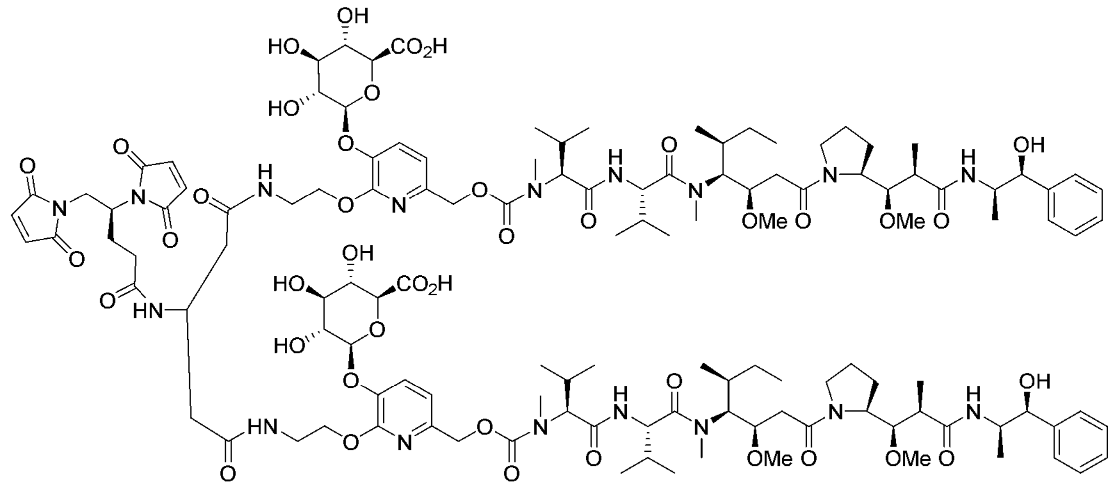

**LD-09**

,

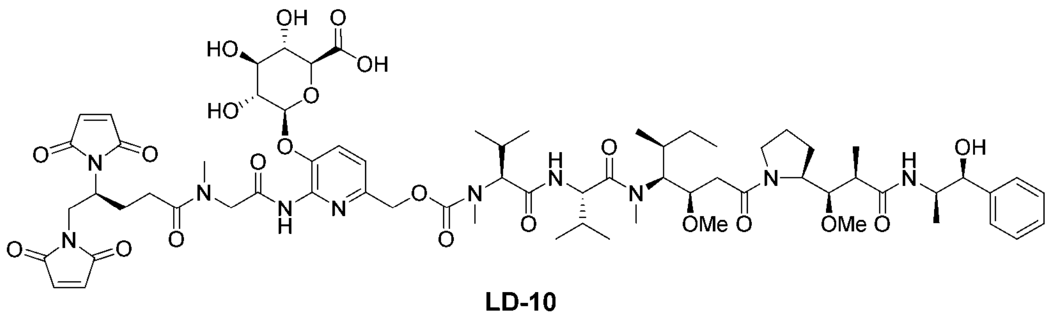

**LD-10**

,

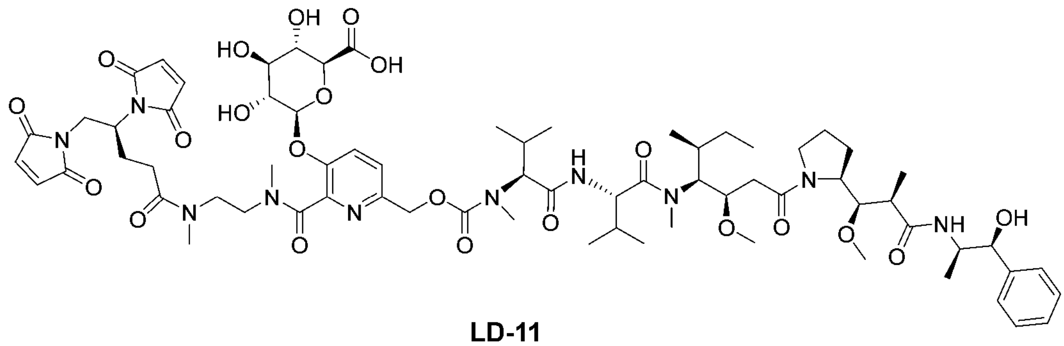

**LD-11**

,

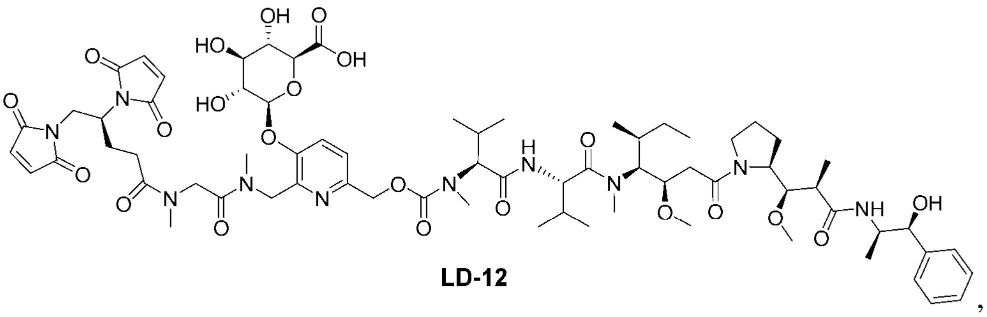

**LD-12**

,

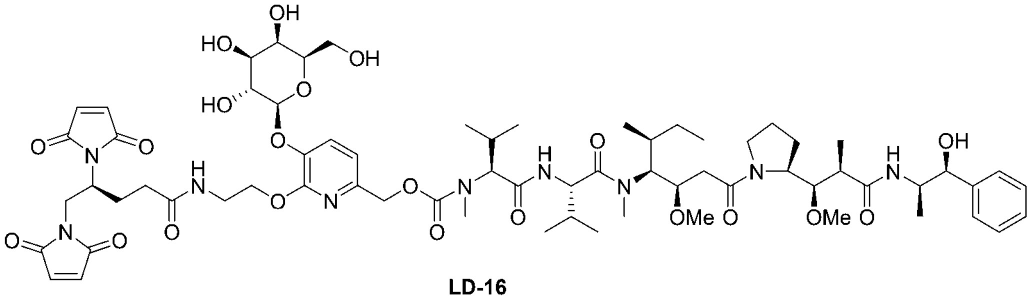

**LD-16**

,

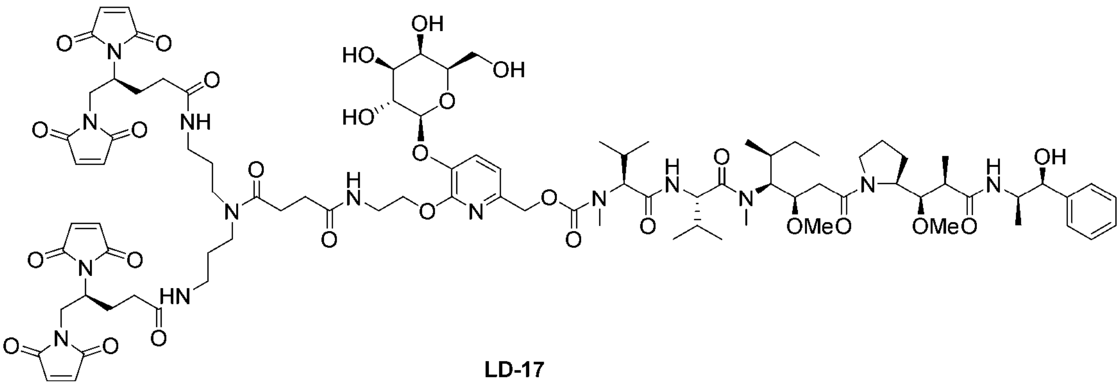

**LD-17**

.

The present invention also provides a pharmaceutical composition comprising the antibody-drug conjugate of formula **I, III,** IV, V, VI or VII according to the present invention and a pharmaceutically acceptable carrier.

The present invention also provides a use of the antibody-drug conjugate of formula **I, III,** IV, V, VI, or VII according to the present invention or the pharmaceutical composition comprising the same in the preparation of a medicament for treating cancer, autoimmune diseases, and inflammatory diseases.

The present invention also provides the antibody-drug conjugate of formula **I, III,** IV, V, VI, or VII according to the present invention or the pharmaceutical composition comprising the same, for use in treating cancer, autoimmune diseases, and inflammatory diseases.

The present invention also provides a method for treating cancer, autoimmune diseases, and inflammatory diseases comprising administering to a subject an effective amount of the antibody-drug conjugate of formula I, III, IV, V, VI, or VII according to the present invention or the pharmaceutical composition comprising the same.

<u>Definition of Terms</u>

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms, an alkyl having 1 to 4 carbon atoms or an alkyl having 1 to 3 carbon atoms. Non-limiting examples include methyl, ethyl, n-propyl, isopropyl, n-butyl, isobutyl, *tert-butyl, sec-butyl,* n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. The alkyl may be substituted or unsubstituted. When substituted, the substituent(s) may be substituted at any available connection point. The substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkylene" refers to a divalent alkyl group with the alkyl as defined above, which has 1 to 20 (for example 1, 2, 3, 4, 5, 6, 7, 8, 9, 10, 11, 12, 13, 14, 15, 16, 17, 18, 19 or 20 ) carbon atoms (i.e., $C_{1-20}$ alkylene). The alkylene is preferably an alkylene having 1 to 12 carbon atoms (i.e., $C_{1-12}$ alkylene), more preferably an alkylene having 1 to 6 carbon atoms (i.e., $C_{1-6}$ alkylene), and further preferably an alkylene having 1 to 4 carbon atoms (i.e., $C_{1-6}$ alkylene). Non-limiting examples of the alkylene include, but are not limited to, methylene ($-CH_2-$), 1,1-ethylidene ($-CH(CH_3)-$), 1,2-ethylidene ($-CH_2CH_2-$), 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$) and the like. The alkylene may be substituted or unsubstituted, and when substituted, it may be substituted at any available connection point. The substituent(s) may be one or more selected from the group consisting of alkyl, alkenyl, alkynyl, alkoxy, haloalkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkenyl" refers to an alkyl consisting of at least two carbon atoms and at least one carbon-carbon double bond, and preferably an alkenyl having 2 to 4 carbon atoms, such as ethenyl, 1-propenyl, 2-propenyl, 1-, 2- or 3-butenyl and the like. The alkenyl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynyl" refers to an alkyl consisting of at least two carbon atoms and at least one carbon-carbon triple bond, and preferably an alkynyl having 2 to 4 carbon atoms or preferably an alkynyl having 3 to 4 carbon atoms, such as ethynyl, propynyl, butynyl and the like. The alkynyl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon group, in which the cycloalkyl ring has 3 to 20 carbon atoms, preferably 3 to 12 carbon atoms, and more preferably 3 to 6 carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. The polycyclic cycloalkyl includes spiro cycloalkyl, fused cycloalkyl and bridged cycloalkyl. The cycloalkyl ring may be fused to an aryl, heteroaryl or heterocyclyl ring, in which the ring linking to the parent structure is cycloalkyl. Non-limiting examples include indanyl, tetrahydronaphthyl, benzocycloheptyl, tetrahydrobenzofuranyl, tetrahydrobenzoxazolyl, tetrahydrobenzoisoxazolyl, cyclopentathienyl, tetrahydrobenzothiazolyl, and the like. The cycloalkyl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a saturated or partially unsaturated monocyclic or polycyclic hydrocarbon group comprising 3 to 20 ring atoms, in which one or more ring atoms are heteroatoms selected from the group consisting of N, O and $S(O)_m$ (wherein m is an integer from 0 to 2), but excluding -O-O-, -O-S- or -S-S- from the ring, the remaining ring atoms being carbon atoms. Preferably, the heterocyclyl comprises 4 to 12 ring atoms, 1 to 4 atoms of which are heteroatoms; and more preferably 7 to 12 ring atoms, 1 to 4 atoms of which are heteroatoms. Non-limiting examples of the monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. The polycyclic heterocyclyl includes spiro heterocyclyl, fused heterocyclyl and bridged heterocyclyl. The heterocyclyl ring may be fused to an aryl, heteroaryl or cycloalkyl ring, in which the ring linking to the parent structure is heterocyclyl. The heterocyclyl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered full-carbon monocyclic or fused polycyclic (i.e., rings sharing a pair of adjacent carbon atoms) group having a conjugated $\pi$-electron system, and preferably a 6 to 10 membered aryl, for example, phenyl and naphthyl. The aryl is more preferably phenyl. The aryl ring may be fused to a heteroaryl, heterocyclyl or cycloalkyl ring, in which the ring linking to the parent structure is an aryl ring. Non-limiting examples thereof include:

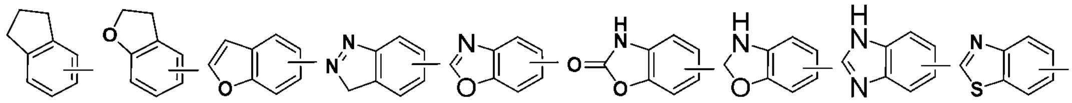

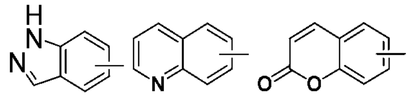 and 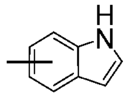.

The aryl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a heteroaromatic system having 5 to 14 ring atoms, which comprises 1 to 4 heteroatoms selected from the group consisting of O, S and N. The heteroaryl is preferably a 5 to 10 membered heteroaryl having 1 to 3 heteroatoms, and more preferably a 5 or 6 membered heteroaryl having 1 to 2 heteroatoms, for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring may be fused to an aryl, heterocyclyl or cycloalkyl ring, in which the ring linking to the parent structure is a heteroaryl ring. Non-limiting examples thereof include:

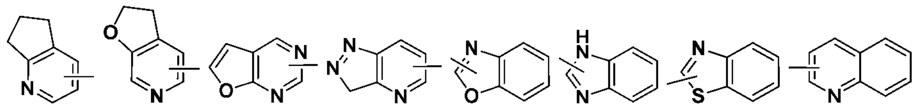 and 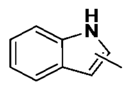.

The heteroaryl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Optional" or "optionally" means that the event or circumstance described subsequently can, but need not, occur, and such description includes the situation in which the event or circumstance may or may not occur. For example, "the heterocyclic group optionally substituted with an alkyl" means that an alkyl group can be, but need not be, present, and such description includes the situation of the heterocyclic group being substituted with an alkyl and the heterocyclic group being not substituted with an alkyl.

"Substituted" refers to one or more hydrogen atoms in a group, preferably up to 5, more preferably 1 to 3 hydrogen atoms, independently substituted with a corresponding number of substituents. It goes without saying that the substituents only exist in their possible chemical position. The person skilled in the art is able to determine whether the substitution is possible or impossible by experiments or theory without paying excessive efforts. For example, it may be unstable that an amino or a hydroxy having free hydrogen is bound to a carbon atoms having unsaturated bonds (such as olefinic).

A "pharmaceutical composition" refers to a mixture of one or more of the compounds according to the present invention or physiologically/pharmaceutically acceptable salts or prodrugs thereof and other chemical components such as physiologically/pharmaceutically acceptable carriers and excipients. The purpose of a pharmaceutical composition is to facilitate administration of a compound to an organism and the absorption of the active ingredient and thus displaying biological activity.

"Pharmaceutically acceptable salts" refers to salts of the compounds of the invention that are safe and effective in mammals and have the desired biological activity.

A "carrier" refers to a carrier or diluent that does not cause significant irritation to the organism and does not eliminate the biological activity and characteristics of the compound.

The term "Drug/Antibody Ratio (DAR)" as used herein refers to the number of drugs that are conjugated to each antibody molecule. Because antibody-drug conjugate samples contain multiple components with different DAR values, the concepts of "average DAR value" and "DAR value distribution" are more suitable for describing the composition of antibody drug conjugates. The average DAR value is the ratio of the total number of drug molecules in a sample to the total number of antibodies, and theDAR value distribution refers to the content distribution of each component with different DAR values in the sample.

As used herein, the term "antibody" or "antibody unit" includes within its scope any fragments of an antibody that binds to or reactively associates with or complexes with a receptor, antigen or other receptor units associated with a given target-cell population. An antibody can be any protein or protein-like molecule that binds to, complexes with, or reacts with

a part of a cell population to be therapeutically or otherwise biologically modified.

Antibody that makes up the ADCs of the invention preferably retains the antigen binding capability of their native, wild type counterparts. Thus, antibodies of the invention are capable of binding (preferably specifically) to antigens. Such antigens include, for example, tumor-associated antigens (TAA), cell surface receptor proteins and other cell surface molecules, cell survival regulatory factors, cell proliferation regulatory factors, molecules associated with tissue development or differentiation (e.g., known or suspected to contribute function), lymphokines, cytokines, molecules involved in cell cycle regulation, molecules involved in vasculogenesis and molecules associated with angiogenesis (e.g. known or suspected to contribute function). The tumor-associated antigen may be a cluster differentiation factor (i.e., a CD protein). Antigens that bind to the antibodies of the present invention may be one or a subset of the above categories, wherein the other subset(s) of said category comprise other molecules/antigens that have a distinct characteristic (with respect to the antigen of interest).

Antibodies used in ADCs include, but not limited to, antibodies against cell surface receptors and tumor-associated antigens. Such tumor-associated antigens are well known in the art, and can be prepared according to the methods or information which is well known in the art for the preparation of antibodies. In order to develop effective objects at cellular level that can be used in the diagnosis and treatment of cancer, the researchers sought to find transmembrane or otherwise tumor-associated polypeptides that are specifically expressed on the surface of one or more particular type(s) of cancer cell as compared to the other one or more normal non-cancerous cell(s). Often, such tumor-associated polypeptides are more abundantly overexpressed on the surface of the cancer cells as compared to the non-cancerous cells. The identification of such tumor-associated factors can greatly enhance the specific targeting properties of antibodies for cancer therapy.

Examples of tumor-associated antigen include, but are not limited to, Tumor-Associated Antigens (1)-(36) listed below. For convenience, information relating to these antigens, all of which are known in the art, is listed below and includes names, alternative names, Genbank accession numbers. Nucleic acid and protein sequences corresponding to the tumor-associated antigens are available in public databases such as GenBank. Tumor-associated antigens targeted by antibodies include all amino acid sequence variants and isoforms possessing at least about 70%, 80%, 85%, 90%, or 95% sequence homology relative to the sequences identified in the cited references, or exhibit substantially the same biological properties or characteristics as a tumor-associated antigen having a sequence found in the cited references.

Tumor-Associated Antigens (1)-(36):

(1) BMPR1B (bone morphogenetic protein receptor-type IB, Genbank accession no. NM_001203);
(2) E16 (LAT1, SLC7A5, Genbank accession no. NM_003486);
(3) STEAP1 (six transmembrane epithelial antigen of prostate, Genbank accession no. NM_012449);
(4) 0772P (CA125, MUC16, Genbank accession no. AF361486);
(5) MPF (MPF, MSLN, SMR, megakaryocyte potentiating factor, mesothelin, Genbank accession no. NM_005823);
(6) Napi3b (NAPI-3B, NPTIIb, SLC34A2, solute carrier family 34 (sodium phosphate) member 2, type II sodium-dependent phosphate transporter 3b, Genbank accession no. NM_006424);
(7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878);
(8) PSCA hlg (2700050C12Rik, C530008016Rik, RIKEN cDNA 2700050C12, RIKEN cDNA 2700050C12 gene, Genbank accession no. AY358628);
(9) ETBR (Endothelin type B receptor, Genbank accession no. AY275463);
(10) MSG783 (RNF124, hypothetical protein FLJ20315, Genbank accession no. NM_017763);
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138);
(12) TrpM4 (BR22450, FLJ20041, TRPM4, TRPM4B, transient receptor potential cation channel, subfamily M, member 4, Genbank accession no. NM_017636);
(13) CRIPTO (CR, CR1, CRGF, CRIPTO, TDGF1, teratocarcinoma-derived growth factor, Genbank accession no. NP_003203 or NM_003212);
(14) CD21 (CR2 (Complement receptor 2) or C3DR (C3d/Epstein Barr virus receptor) or Hs. 73792, Genbank accession no. M26004);
(15) CD79b (CD79B, CD79β, IGb (immunoglobulin associated beta), B29, Genbank accession no. NM_000626);
(16) FcRH2 (IFGP4, IRTA4, SPAP1A (SH2 domain containing phosphatase anchor protein 1a), SPAP1B, SPAP1C, Genbank accession no. NM_030764);
(17) HER2 (ErbB2, Genbank accession no. M11730);
(18) NCA (CEACAM6, Genbank accession no. M18728);
(19) MDP (DPEP1, Genbank accession no. BC017023);

(20) IL20Rα (IL20Ra, ZCYTOR7, Genbank accession no. AF184971);
(21) Brevican (BCAN, BEHAB, Genbank accession no. AF229053);
(22) EphB2R (DRT, ERK, Hek5, EPHT3, Tyro5, Genbank accession no. NM_004442);
(23) ASLG659 (B7h, Genbank accession no. AX092328);
(24) PSCA (Prostate stem cell antigen precursor, Genbank accession no. AJ297436);
(25) GEDA (Genbank accession no. AY260763);
(26) BAFF-R (B-cell activating factor receptor, BLys receptor 3, BR3, Genbank accession no. AF116456);
(27) CD22 (B-cell receptor CD22-β isotype, Genbank accession no. AK026467);
(28) CD79a (CD79A, CD79α, immunoglobulin-associated alpha, a B-cell specific protein that covalently interacts with Ig beta (CD79B) and forms a complex with Ig M molecules on the surface, transduces a signal involved in B-cell differentiation, Genbank accession No. NP-001774.1);
(29) CXCR5 (Burkitt's lymphoma receptor 1, a G protein-coupled receptor that is activated by the CXCL13 chemokine, plays a role in lymphocyte migration and humoral defense, plays a role in HIV-2 infection and perhaps in AIDS, lymphoma, myeloma, and leukemia, Genbank accession No. NP_001707.1);
(30) HLA-DOB (Beta subunit of MHC class II molecule (Ia antigen) that binds peptides and presents them to CD4+ T lymphocytes, Genbank accession No. NP_002111.1);
(31) P2X5 (Purinergic receptor P2X ligand-gated ion channel 5, an ion channel gated by extracellular ATP, may be involved in synaptic transmission and neurogenesis, its deficiency may contribute to the pathophysiology of idiopathic detrusor instability, Genbank accession No. NP_002552.2);
(32) CD72 (B-cell differentiation antigen CD72, Lyb-2, Genbank accession No. NP_001773.1);
(33) LY64 (lymphocyte antigen 64 (RP105), type I membrane protein family which is rich in leucine repeat (LRR), regulates B-cell activation and apoptosis, loss of function is associated with increased disease activity in patients with systemic lupus erythematosis, Genbank accession No. NP_005573.1);
(34) FcRH1 (Fc receptor-like protein 1, a putative receptor for the immunoglobulin Fc domain that contains C2 type Ig-like and ITAM domains, may play a role in B-lymphocyte differentiation, Genbank accession No. NP_443170.1);
(35) IRTA2 (Translocation-related immunoglobulin superfamily receptor 2, a putative immunoreceptor with possible roles in B cell development and lymphoma genesis; gene disorder caused by translocation occurs in certain B-cell malignancies, Genbank accession No. NP_112571.1);
(36) TENB2 (putative transmembrane proteoglycan, related to the EGF/heregulin family of growth factors and follistatin, Genbank accession No. AF179274).

As used herein, the term "drug" or "D" refers to any compound possessing a desired biological activity and having a reactive functional group that may be used to prepare the conjugate of the invention. The desired biological activity includes diagnosis, cure, alleviation, treatment, or prevention of disease in human or other animals. Thus, so long as it has the necessary reactive functional group, the term "drug" refers to the drugs recognized by pharmacopeia, and the drugs such as that identified by official Homeopathic Pharmacopeia of the United States, official National Formulary, or any supplement thereof. Typical drugs are set forth in the Physician's Desk Reference (PDR) and in the Orange Book maintained by the U.S. Food and Drug Administration (FDA). New drugs are continually being discovered and developed, and the present invention provides that these new drugs may also be incorporated into the prodrug of the conjugate of the present invention.

Preferably, the drug is a cytotoxic drug useful in cancer therapy; a protein or polypeptide possessing a desired biological activity, such as a toxin, e.g., abrin, ricin A, pseudomonas exotoxin, and diphtheria toxin; other suitable proteins including tumor necrosis factor, α-interferon, β-interferon, nerve growth factor, platelet derived growth factor, tissue plasminogen activator, and biological response modifiers, such as lymphokines, interleukin-1 (IL-1), interleukin-2 (IL-2), interleukin-6 (IL-6), granulocyte macrophage colony stimulating factor (GM-CSF), granulocyte colony stimulating factor, or other growth factors.

In one aspect, the drug is maytansine or maytansinoids. Maytansine inhibits cell proliferation by inhibiting the formation of microtubules of the microtubulin protein (Science 1975, 189, 1002-1005; US 5208020). Maytansinoids are derivatives of maytansine. Both maytansine and maytansinoids are highly cytotoxic, but their clinical use in cancer therapy has been greatly limited due to poor selectivity for tumors. However, the high cytotoxic potency enables them to be attractive drug in ADCs. The structures shown below are maytansine, maytansinoids, and three representative maytansinoids commonly used in ADC:

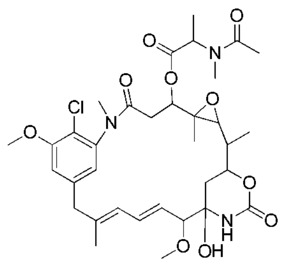

Maytansine

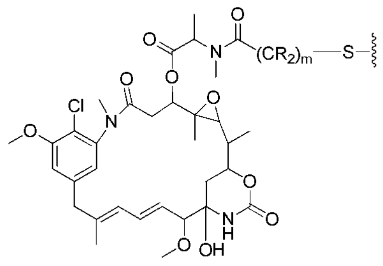

Maytansinoids

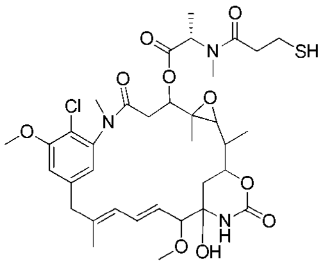

DM1

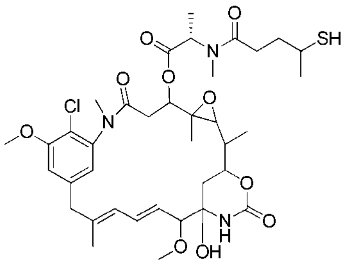

DM3

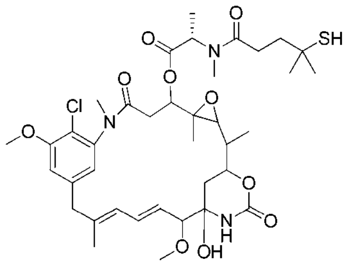

DM4

The main raw material for preparing maytansinoids is maytansinol, which is mainly obtained from ansamitocins hydrolysis. Ansamitocins could be accessibly produced by fermentation. Ansamitocin derivatives (WO 2012/061590) and alaninyl maytansinol (US 2012/0121615) are also reported to be good candidates as ADC "warheads" (the structures of the two compounds are shown below).

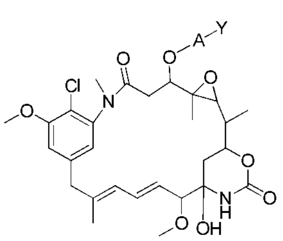

A is C=O, (C=O)NR', and (C=O)O
Y is a substituent group

Ansamitocin derivatives

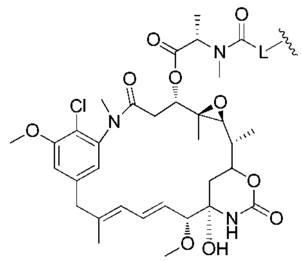

L is 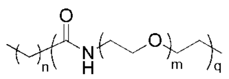

Alaninyl maytansinol

In another aspect, the drug is auristatins. Auristatins are analogues of Dolastatin 10, which was biologically active polypeptide isolated from the marine mollusk *Dolabella auricularia* (US 7498298). Dolastatin 10 is an agent that inhibits tubulin polymerization by binding to the same domain on tubulin as the anticancer drug vincristine. Dolastitin 10, auristatin PE, and auristatin E are all linear peptides having four amino acids, three of which are unique to the dolastatins, and a C-terminal amide. Two representative auristatins, monomethyl auristatin E (MMAE) and monomethyl auristatin F (MMAF), are preferred drug candidates for ADCs.

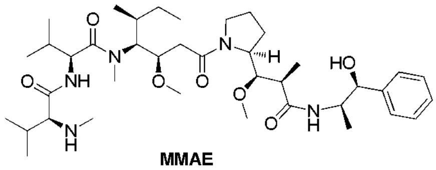

MMAE

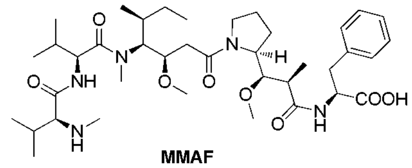

MMAF

In another aspect, the drug is tubulysins. Tubulysins are natural products first isolated from myxobacterial culture, which have anti-mitotic activity that act by inhibiting tubulin polymerization, and among which Tubulysin D is the most potent. Tubulysin D is a complex tetrapeptide, and unstable in both acidic or basic conditions due to the O-acyl/N,O-acetal functional groups. US 2011/0021568 and US 2013/0224228 disclosed a series of tubulysin analogs respectively, which remove the unstable groups from the structure and have high cytotoxic potency.

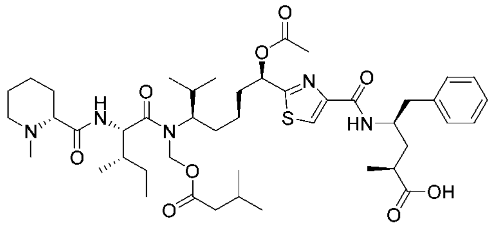

Tubulysin D

In another aspect, the drug is calicheamicins. Calicheamicins are antitumor antibiotics that bind to the minor groove of DNA to promote cleavage of double-stranded DNA at a specific site, thus causing cell death. Calicheamicins are potent at sub-picomolar concentrations *in vitro,* but their low therapeutic index precluded further clinical development. The high potency, however, makes them good candidates for ADCs (such as Gemtuzumab Ozogamicin and Inotuzumab Ozogamicin).

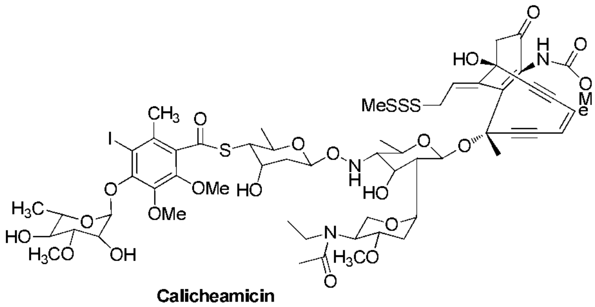

Calicheamicin

In another aspect, the drug are doxorubicin. Doxorubicin can embed DNA double helix structure to block DNA replication and is used as chemotherapeutic agent. Due to the relative low potency of doxorubicin ($IC_{50}$ of 0.1-0.2 $\mu$M for human carcinoma lines, whereas subnanomolar activities are now typically seen for ADC payloads), application of doxorubicin as ADC drug is not popular.

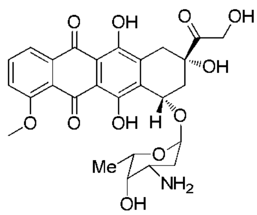

doxorubicin

In another aspect, the drug are benzodipyrrole antibiotic (duocarmycins, CC-1065 and the like) and other cyclopropapyrroloind-4-one (CPI) derivatives, which are potent minor-groove binding-DNA alkylating agents. Cyclopropabenzindol-4-one analogues (CBI) are chemically more stable, biologically more potent, and synthetically more accessible than their parent compounds comprising the nature CPI alkylating subunit. One representative CBI derivative is the phenolic hydroxyl group-protected CBI, which has decreased prodrug toxicity and improved water solubility.

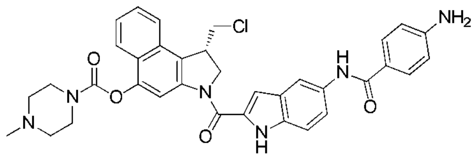

CBI

In another aspect, the drug is pyrrolo[2,1-c][1,4]benzodiazepines (PBDs) or PBD dimers. PBDs are natural products produced by Streptomyces species with the unique characteristic of forming nondistortive covalent adducts in the minor groove of DNA, specifically at the purine-guanine-purine sequences. There is a growing interest in using PBDs as part of a small-molecule strategy for targeting DNA sequences and also as novel anticancer and antibacterial agents (Biochemistry 2008, 47, 11818-11829). The biological activity of these molecules can be potentiated by joining two PBD

units together through their C8/C8'-hydroxy groups via a flexible carbon chain (WO 2011/130616). The PBD dimers are thought to form sequence-selective DNA lesions such as the palindromic 5'-Pu-GATC-Py-3' interstrand cross-link, which mainly accounts for their biological activity. These compounds have been shown to be highly useful cytotoxic agents and good candidates as ADC warheads.

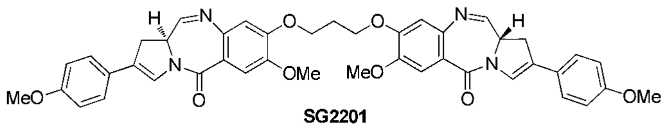

SG2201

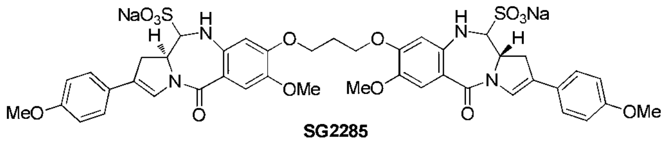

SG2285

In another aspect, the drug is Camptothecins (CPT). Camptothecin and its derivatives, as topoisomerase (TOP1) inhibitors, selectively capture the TOP1 cleavage complex, making them highly promising anticancer drugs. Camptothecin derivatives such as topotecan, irinotecan (CPT-11), and beloteccan have been approved for the treatment of non-small cell lung cancer, ovarian cancer, and colorectal cancer. These compounds have also been successfully used as warheads in ADC drug design, such as SN-38, Exatecan (DX-8951f), DXd and the like.

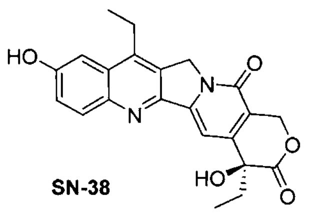

SN-38

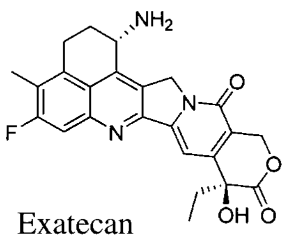

Exatecan

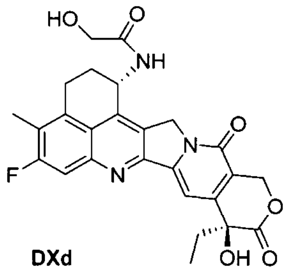

DXd

In another aspect, the drug is amanitins. Amanitin is an octapeptide toxin with a bicyclic structure produced by the poisonous fungus Amanita phalloides. This toxin specifically binds to RNA polymerase II (or B) for non-competitive inhibition, thereby inhibiting the formation of phosphodiester bonds (the initiation and elongation of RNA chain synthesis), thus inhibiting the catalytic synthesis of mRNA and leading to apoptosis. Since transcription catalyzed by RNA polymerase II is crucial for cellular function, α-amanitin can uniquely kill both dividing and quiescent cells simultaneously. This characteristic makes it an attractive cytotoxic candidate for ADC drugs.

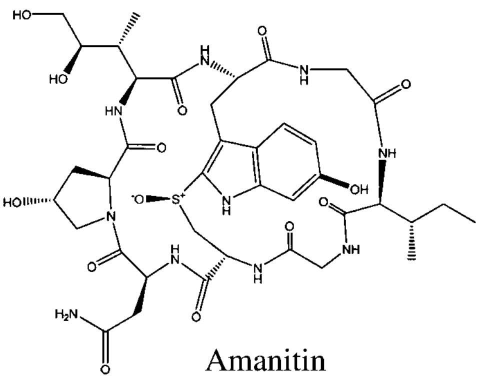

Amanitin

In another aspect, drugs are not limited to the categories mentioned above, but also include all drugs that can be used in antibody-drug conjugates, including but not limited to immune agonists (such as TLR7/8, Sting agonists), antibiotics, hormones, etc.

As used herein, the term "linker" or "ADC linker" refers to a bifunctional or multifunctional molecule that can react with a protein/antibody and a drug respectively, and thus link the protein/antibody to the drug as a "bridge". According to drug release mechanism in cells, "linker" or "ADC linker" could be classified into two categories of non-cleavable linker and

cleavable linker.

Non-cleavable linker is a kind of relatively stable linker, which is difficult to be cleaved under *in vivo* conditions. For ADCs with non-cleavable linkers, the drug release mechanism is as follows. After the conjugate binds to the antigen and is endocytosed by the cell, the antibody is enzymatically digested in the lysosome, releasing an active molecule composed of a small molecule drug, a linker, and antibody amino acid residues. The chemical modification of the drug didn't diminish its cytotoxic potential. This form of the drug is, however, charged (amino acid residue) and hard to diffuse into neighboring cells. Hence, it can't kill adjacent tumor cells that don't express the target antigen (antigen-negative cells) (bystander effects) (Bioconjugate Chem. 2010, 21, 5-13). Some common linkers, such as MC linker, MCC linker, etc., are shown as below:

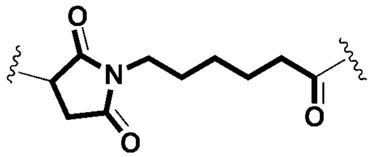

**MC linker**

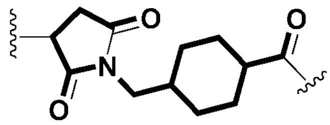

**MCC linker**

Cleavable linkers, as the name implies, could be cleaved within the target cells to release the active drugs (small molecule drugs themselves). Cleavable linkers can be categorized into two main groups of chemically labile and enzyme-labile linkers.

Chemically labile linkers could be selectively cleaved according to the properties of the plasma and cytoplasm. Such properties include pH value, glutathione concentration, etc.

pH sensitive linkers are generally called acid-cleavable linkers. The linkers are relatively stable in the neutral environment (pH 7.3-7.5) of blood, but will undergo hydrolysis in the mildly acidic endosomes (pH 5.0-6.5) and lysosomes (pH 4.5-5.0). Most of the linkers, such as hydrozones, carbonates, acetals, ketals, were used for the first generation of ADCs. However, due to the limited plasma stability of the acid-cleavable linkers, the ADCs based on this kind of linkers have relatively short half-life (2-3 days). The shortened half-lives preclude the application of pH-sensitive linkers in the new generations of ADCs to a certain degree.

Glutathione-sensitive linkers are generally called disulfide linkers. The release is attributed to the high intracellular concentration (millimolar range) *versus* the relatively low concentration in the blood (micromolar range) of glutathione. This is especially true for tumor cells, where the hypoxic state results in enhanced activity of reductive enzymes and thus even higher glutathione concentrations. Disulfide bonds are thermodynamically stable and thus provide good stability in plasma.

Enzyme-labile linkers, such as peptide linkers, can achieve better control of the drug release. The peptide linker will be effectively cleaved by lysosomal proteases, such as cathepsin B or plasmin (elevated levels in certain tumor tissues). Such peptidic linkages are deemed stable in plasma circulation, as proteases are usually not extracellularly active due to the extracellular unfavorable pH and the serum protease inhibitors. In view of the high plasma stability and good intracellular cleaving selectivity and efficiency, enzyme-labile linkers are broadly selected as cleavable linker candidates in ADCs. Typical enzyme-labile linkers include vc linker, etc.

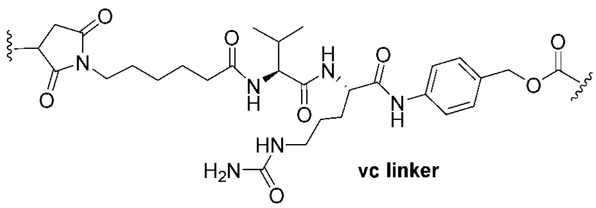

**vc linker**

Self-immolative linker is generally sited between cleavable linker and active drug, or is part of a cleavable linker itself. The working mechanism of self-immolative linker is that it can undergo self-structural rearrangement to release the linked active drug when the cleavable linker was cleaved under suitable conditions. Typical self-immolative linkers include p-aminobenzyl alcohol (PAB), etc.

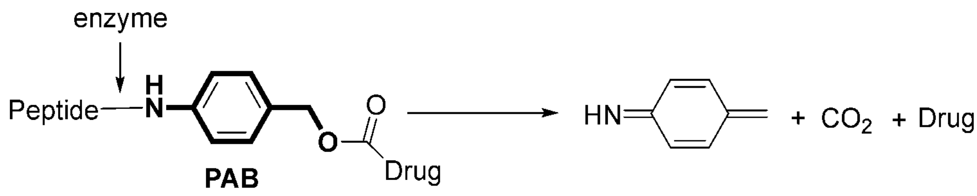

The antibody-drug conjugate according to the present invention consists of an antibody, a linker containing a glycosylpyridine fragment, and a drug, wherein the glycosylpyridine linker is an enzyme-labile linker. In one aspect, the antibody-drug conjugate according to the present invention targets a special cell population and binds to the specific cell surface proteins (antigens) to form a complex, followed by the internalization of the complex into the cell and releasing of the drug within the cell in active form.

In another aspect, the antibody-drug conjugate according to the present invention targets a special cell population and binds to the specific cell surface proteins (antigens) to take effects; or releases drugs outside the cell, followed by the permeation of the drugs into the cell to take effects.

The pharmaceutical composition of the present invention can be in the form of a sterile injectable aqueous solution. The acceptable vehicles and solvents that can be employed include water, Ringer's solution and isotonic sodium chloride solution.

It is well known to those skilled in the art that the dosage of a drug depends on a variety of factors including, but not limited to the following factors of activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound or the type of pharmaceutically acceptable salt can be verified according to the traditional therapeutic regimens.

The main advantages of the present invention are as follows:

1. The present invention provides an antibody-drug conjugate containing a glycosylpyridine linker fragment. The glycosylpyridine linker fragment has good hydrophilicity, thereby greatly improving the hydrophilicity of the antibody-drug conjugate, reducing the aggregation of ligand-drug conjugates, and improving the PK property of the antibody-drug conjugate *in vivo.*
2. The glycosylpyridine linker fragment according to the present invention is applicable to the construction of most antibody-drug conjugates, possessing universality and therefore broad application prospects.

## DESCRIPTION OF THE DRAWINGS

Figures 1A to 1K are hydrophobic interaction chromatograms (HIC) of the antibody-drug conjugates of the present invention. Figures 1A to 1E correspond to antibody-drug conjugates Ab1-LD-02, Ab1-LD-10, Ab1-LD-11, Ab1-LD-12, and Ab1-LD-14, respectively; Figures 1F to 1K correspond to antibody-drug conjugates Ab2-LD-02, Ab2-LD-04, Ab2-LD-14, Ab2-LD-15, Ab2-LD-16, and Ab2-LD-17, respectively.

## EXAMPLES

The present invention will be further described in details with the following examples. However, it should be understood that these examples are used to illustrate the present invention, but should not be considered as limiting the scope of the invention. The unstated experiment conditions are generally according to routine conditions or conditions suggested by manufacturers. All reactions were conducted under nitrogen atmosphere, except for hydrogenation reaction.

Unless otherwise defined, all of the professional and scientific terms used in the present invention have the same meaning as those familiar by the person skilled in the art. Furthermore, any method or material similar or equal to those used in the present invention can be applied herein. The methods and materials used in the present invention are only used for illustration.

Abbreviation

| | |
|---|---|
| Ab | Antibody |
| ACN | Acetonitrile |
| ADC | Antibody-Drug Conjugate |

| | |
|---|---|
| BOC (Boc) | *tert*-Butyloxycarbonyl |
| DIPEA | Diisopropylethylamine |
| ELISA | Enzyme-Linked Immunosorbent Assay |
| Eq (eq) | Equivalent |
| HIC | Hydrophobic Interaction Chromatography |
| HPLC | High Performance Liquid Chromatography |
| LCMS | Liquid Chromatography-Mass Spectrometry |
| mAb | Monoclonal Antibody |
| MS | Mass Spectrometry |
| Prep-RP-HPLC | Preparative High Performance Liquid Chromatography |
| Rt | Retention Time |
| SDS-PAGE | Polyacrylamide Gel Electrophoresis |
| SEC | Size Exclusion Chromatography |
| TEA | Triethylamine |
| TFA | Trifluoroacetic Acid |

Unless otherwise stated, all of the anhydrous reagents are purchased from the suppliers and kept under nitrogen atmosphere. All other reagents and solvents purchased are of high purity and need not to be purified before use. The commercially available reagents mentioned in the examples are used in accordance with the manufacturer's instructions.

NMR spectrum is collected on a Bruker Avance III 400 MHz instrument. Chemical shift ($\delta$) unit is ppm, and the reference reagent is tetramethylsilane (chemical shift = 0).

For liquid chromatography-mass spectrometry, low resolution mass spectrum is collected on Agilent 6110 (acid method) or 6120B (alkali method) mass spectrometers coupled with Hewlett-Packard Agilent 1200 high performance liquid chromatography.

Method 1: Waters Sunfire C18 reverse phase column (4.60 $\times$ 50 mm, 3.5 $\mu$m) is used in the acid high performance liquid chromatography method for separation, and the eluting gradient is 5%-95% B (acetonitrile, containing 0.01% TFA) in A (water, containing 0.01% TFA) over 1.3 min. The flow rate is 2.0 mL/min, and the column temperature is 50°C.

Method 2: Waters Sunfire C18 reverse phase column (4.6 $\times$ 30 mm, 2.7 $\mu$m) is used in the acid high performance liquid chromatography method for separation, and the eluting gradient is 5%-95% B (acetonitrile, containing 0.01% TFA) in A (water, containing 0.01% TFA) over 1.4 min. The flow rate is 2 mL/min, and the column temperature is 50°C.

Method 3: Waters Xbridge C18 reverse phase column (4.60 $\times$ 50 mm, 3.5 $\mu$m) is used in the base high performance liquid chromatography method for separation, and the eluting gradient is 5%-95% B (acetonitrile) in A (water, containing 10 mM ammonium bicarbonate) over 1.5 min. The flow rate is 1.8 mL/min, and the column temperature is 50°C.

Preparative reverse phase high performance liquid chromatography (prep-RP-HPLC) is conducted on a Gilson instrument. Waters Sunfire C18 reverse phase column (250 $\times$ 19 mm, 10 $\mu$m) is used for separation.

Method 4: Acid preparative chromatography, mobile phases: A is water phase containing 0.1% TFA, B is ACN; the flow rate is 15 mL/min.

Method 5: Base preparative chromatography, mobile phases: A is water phase containing 10 mM ammonium bicarbonate, B is ACN; the flow rate is 15 mL/min.

The cell lines Ramos (CRL-1596, ATCC) and BJAB (ACC757, DSMZ) used are human non-Hodgkin lymphoma cells. The cell lines HH (CRL-2105, ATCC), L540 (CSC-C0233, Creative Bioarray), and SU-DHL-1 (CRL-2955, ATCC) used are human Hodgkin lymphoma cells. Ab1 (humanized anti-CD79b monoclonal antibody, IgG1) and Ab2 (humanized anti-CD30 monoclonal antibody, IgG1) are antibodies developed in-house. The enzyme labeled anti-antibody is purchased from Sigma (Shanghai). Substrate solution is purchased from Decent Biotech (Shanghai). Cell Counting Kit (CCK-8) cell proliferation-cytotoxicity assay kit is purchased from Dojindo (Shanghai).

The following is the sequence of Ab1 (humanized anti-CD79b monoclonal antibody, IgG1):

Ab1 heavy chain

QVQLVQSGAEVKKPGSSVKVSCKTSGNTFTSYGINWVKQAPGQGLEWIGEIFPR
SGNIYYNEKFKGRVTITADKSTSTAYMELSSLRSEDTAVYYCAKGGTGDFDYWG
QGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGK

SEQ ID NO：1

Ab1 light chain

DVVMTQSPLSLPVTLGQPASISCRSSQNIVHSDGNTYLEWYQQRPGQSPRLLIYK
VSFRLSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO：2

The following is the sequence of Ab2 (humanized anti-CD30 monoclonal antibody, IgG1):

Ab2 heavy chain

QVQLVQSGAEVKKPGASVKVSCKASGYTFTSYWIHWIKQAPGQGLEWIGYINP
SSSYTDYNQNFKDKATLTADKSSSTAYMELSSLRSEDTAVYYCARREDYVPFAY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK

SEQ ID NO：3

Ab2 light chain

DIVMTQSPSSLSASVGDRVTITCKASQDVSTAVAWYQQKPGQSPKLLIYWASTR
HTGVPDRFTGSGSGTDYTLTISSLQPEDFATYYCQQHYRIPHTFGGGTKVEIKRT
VAAPSVFIFPPSDEQLKSGTASVVCLLNNFYPREAKVQWKVDNALQSGNSQESV

TEQDSKDSTYSLSSTLTLSKADYEKHKVYACEVTHQGLSSPVTKSFNRGEC

SEQ ID NO：4

Example 1: Synthesis of linker intermediate KL-1

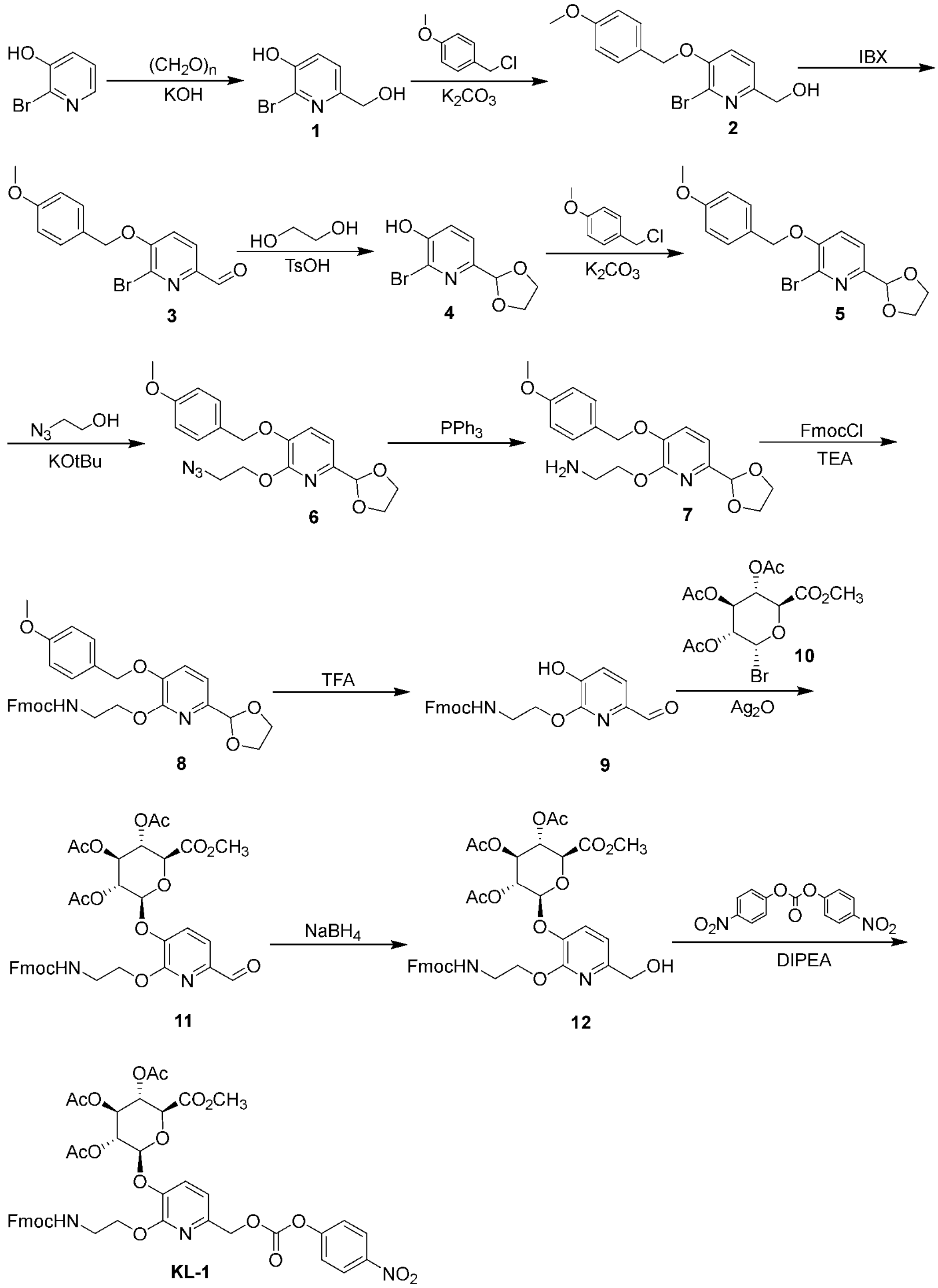


Step 1: Preparation of compound 1

2-Bromo-3-hydroxypyridine (15 g, 86.2 mmol), potassium hydroxide (KOH, 4.83 g, 86.2 mmol), and paraformaldehyde ($(CH_2O)_n$, 7.76 g, 258.6 mmol) were added to 135 mL of water, and the reaction solution was stirred at 80°C for 17 hours. The reaction solution was cooled to room temperature, acetic acid (5.17 g, 86.2 mmol) was added thereto, and the solvent was removed by concentration under reduced pressure. The residues were purified by silica gel column chromatography (eluent: ethyl acetate/petroleum ether (2/1)) to obtain compound **1** as a yellow solid (6.8 g). LCMS (method 1): Rt = 1.18 min, m/z ($ES^+$) 204.1 $[M+H]^+$.

Step 2: Preparation of compound **2**

Compound **1** (6.8 g, 33.33 mmol), 4-methoxybenzyl chloride (5.7 g, 36.66 mmol), and potassium carbonate ($K_2CO_3$, 6.9 g, 50 mmol) were added to N,N-dimethylformamide (30 mL), and the reaction solution was stirred at room temperature for 17 hours. The mixture was poured into ice water (150 mL) and extracted with ethyl acetate (30 mL × 4). The combined organic phases were dried over anhydrous sodium sulfate, filtered, and concentrated under reduced pressure to remove the solvent. The residues were washed with dichloromethane (15 mL × 2) and dried to obtain compound **2** as a white solid (6.2 g).
LCMS (method 3): Rt = 1.88 min, m/z ($ES^+$) 324.0 $[M+H]^+$.

Step 3: Preparation of compound **3**

Compound **2** (6.2 g, 19.14 mmol) was dissolved in dimethyl sulfoxide (40 mL), and 2-iodoxybenzoic acid (IBX, 6.4 g, 22.97 mmol) was added thereto under stirring. The reaction solution was stirred at room temperature for 17 hours and then filtered. The filtrate was poured into an ice-water mixture (200 mL), and the mixture was extracted with ethyl acetate (30 mL × 2). The combined organic phases were washed with saturated brine, dried over anhydrous sodium sulfate, filtered, and concentrated under reduced pressure to remove the solvent, yielding crude compound **3** as a white solid (6.0 g).
LCMS (method 3): Rt = 2.02 min, m/z ($ES^+$) 322.0 $[M+H]^+$.

Step 4: Preparation of compound **4**

Compound **3** (6.0 g, 18.63 mmol), ethylene glycol (2.3 g, 37.26 mmol), and *p*-toluenesulfonic acid monohydrate ($TsOH \cdot H_2O$, 177 mg, 0.93 mmol) were added to toluene (50 mL) successively, and the reaction solution was stirred at 100°C for 17 hours. After the reaction solution was cooled to room temperature, water (100 mL) was added thereto for extraction, and the separated aqueous phase was extracted with ethyl acetate (30 mL × 2). The combined organic phases were dried over anhydrous sodium sulfate, filtered, and concentrated under reduced pressure to remove the solvent. The residues were purified by silica gel column chromatography (eluent: ethyl acetate/petroleum ether (1/1)) to obtain compound **4** as a yellow solid (1.8 g).
LCMS (method 1): Rt = 1.59 min, *m*/*z* ($ES^+$) 246.1 $[M+H]^+$.

Step 5: Preparation of compound **5**

Compound **4** (1.8 g, 7.32 mmol), 4-methoxybenzyl chloride (1.26 g, 8.05 mmol), and potassium carbonate (1.52 g, 10.98 mmol) were added to N,N-dimethylformamide (25 mL) successively, and the reaction solution was stirred at room temperature for 17 hours. The reaction solution was poured into ice water (120 mL) and then extracted with ethyl acetate (20 mL × 2). The combined organic phases were washed with saturated brine, dried over anhydrous sodium sulfate, filtered, and concentrated under reduced pressure to remove the solvent. The residues were purified by silica gel column chromatography (eluent: ethyl acetate/petroleum ether (1/4)) to obtain compound **5** as a white solid (2.0 g).
LCMS (method 3): Rt = 2.01 min, m/z ($ES^+$) 366.0 $[M+H]^+$.

Step 6: Preparation of compound **6**

Azidoethanol (4.75 g, 54.6 mmol) was added to a solution of potassium tert-butoxide in tetrahydrofuran (KOtBu, 1 N, 54.6 mL), and the reaction solution was stirred at room temperature for half an hour. Compound **5** (10.0 g, 27.3 mmol) was added to the above solution, and the reaction solution was stirred at 80°C for 2 hours. After the reaction solution was cooled to room temperature, water (300 mL) was added thereto, followed by extraction with ethyl acetate (200 mL × 3). The combined organic phases were washed with saturated brine (300 mL × 2), dried over anhydrous sodium sulfate, filtered, and concentrated under reduced pressure to remove the solvent, yielding compound **6** as a yellow oil (8.5 g).
LCMS (method 3): Rt = 2.06 min, m/z ($ES^+$) 373.1 $[M+H]^+$.

Step 7: Preparation of compound **7**

Compound **6** (54 g, 145 mmol) was dissolved in tetrahydrofuran (1000 mL), and triphenylphosphine ($PPh_3$, 57.1 g, 218 mmol) and water (10 mL) were added under stirring successively. The reaction solution was stirred at room temperature overnight, and then the solvent was removed by concentration under reduced pressure. The residues were purified by silica gel column chromatography (eluent: ethyl acetate/petroleum ether (1/1) → methanol/dichloromethane (1/10)) to obtain compound **7** as a white solid (28.0 g).

LCMS (method 3): Rt = 1.79 min, m/z ($ES^+$) 347.1 $[M+H]^+$.

Step 8: Preparation of compound **8**

Compound **7** (28 g, 145 mmol) and triethylamine (16.36 g, 162 mmol) were added to tetrahydrofuran (300 mL) successively. The solution was cooled to 0°C, and fluorenylmethyl chloroformate (FmocCl, 25.1 g, 97.2 mmol) was added. The reaction solution was stirred at room temperature for 1 hour and then filtered. The obtained filtrate was concentrated under reduced pressure to obtain a yellow oil. The residues were purified by silica gel column chromatography (eluent: ethyl acetate/dichloromethane/petroleum ether (1/1/4)) to obtain compound **8** as a yellow solid (25.0 g).

LCMS (method 3): Rt = 2.21 min, m/z ($ES^+$) 569.1 $[M+H]^+$.

Step 9: Preparation of compound **9**

Compound **8** (1.1 g, 1.93 mmol) was dissolved in a mixed solution of dichloromethane (8 mL) and water (0.8 mL), and trifluoroacetic acid (4 mL) was added thereto under ice-bath. The reaction solution was stirred under ice-water bath for 1 hour. Then, saturated sodium bicarbonate solution (100 mL) was added thereto to quench the reaction, and the mixture was extracted with dichloromethane (15 mL × 3). The combined organic phases were dried over anhydrous sodium sulfate, filtered, and concentrated under reduced pressure to remove the solvent. The residues were purified by silica gel column chromatography (eluent: ethyl acetate/dichloromethane/petroleum ether (1/1/3)) to obtain compound **9** as a yellow solid (690 mg).

LCMS (method 3): Rt = 1.67 min, m/z ($ES^+$) 405.1 $[M+H]^+$.

Step 10: Preparation of compound **11**

Compound **9** (690 mg, 1.7 mmol), Compound **10** (1.0 g, 2.55 mmol, prepared with reference to the method disclosed in WO2017/51249), and silver oxide ($Ag_2O$, 1.97 g, 8.5 mmol) were added to acetonitrile (150 mL) successively, and the reaction solution was stirred at room temperature protected from light for 17 hours. The reaction solution was filtered, and the filter cake was washed with tetrahydrofuran (15 mL × 3). The filtrates were combined and concentrated under reduced pressure to remove the solvent. The residues were purified by silica gel column chromatography (eluent: ethyl acetate/dichloromethane/petroleum ether (1/1/3)) to obtain compound **11** as a white solid (800 mg).

LCMS (method 3): Rt = 1.81 min, m/z ($ES^+$) 721.1 $[M+H]^+$.

Step 11: Preparation of compound **12**

Compound **11** (800 mg, 1.11 mmol) was dissolved in a mixed solution of isopropanol (1 mL) and dichloromethane (5 mL), and sodium borohydride ($NaBH_4$, 63 mg, 1.67 mmol) was added under ice-water bath. After the reaction solution was stirred in an ice-water bath for 1.5 hours, 25 mL of water was added thereto, and the mixed solution was extracted with dichloromethane (15 mL × 3). The combined organic phases were dried over anhydrous sodium sulfate, filtered, and concentrated under reduced pressure to remove the solvent. The residues were purified by silica gel column chromatography (eluent: ethyl acetate/dichloromethane/petroleum ether (1/1/1)) to obtain compound **12** as a white solid (310 mg).

LCMS (method 1): Rt = 1.59 min, m/z ($ES^+$) 723.2 $[M+H]^+$.

Step 13: Preparation of compound **KL-1**

Compound **12** (270 mg, 0.373 mmol), bis(4-nitrophenyl) carbonate (227 mg, 0.746 mmol), and diisopropylethylamine (96 mg, 0.746 mmol) were added to dry N,N'-dimethylformamide (5 mL) successively, and the reaction solution was stirred at room temperature for 17 hours. Water (30 mL) was added to the reaction solution, followed by extraction with ethyl acetate (30 mL × 3). The combined organic phases were washed with saturated brine (30 mL × 2), dried over anhydrous sodium sulfate, filtered, and concentrated under reduced pressure to remove the solvent. The residues were purified by silica gel column chromatography (eluent: ethyl acetate/petroleum ether (1/10 → 1/1)) to obtain compound **KL-1** as a white solid (310 mg).

LCMS (method 1): Rt = 2.27 min, *m/z* ($ES^+$) 888.2 $[M+H]^+$.

Example 2: Synthesis of linker intermediate **KL-2**

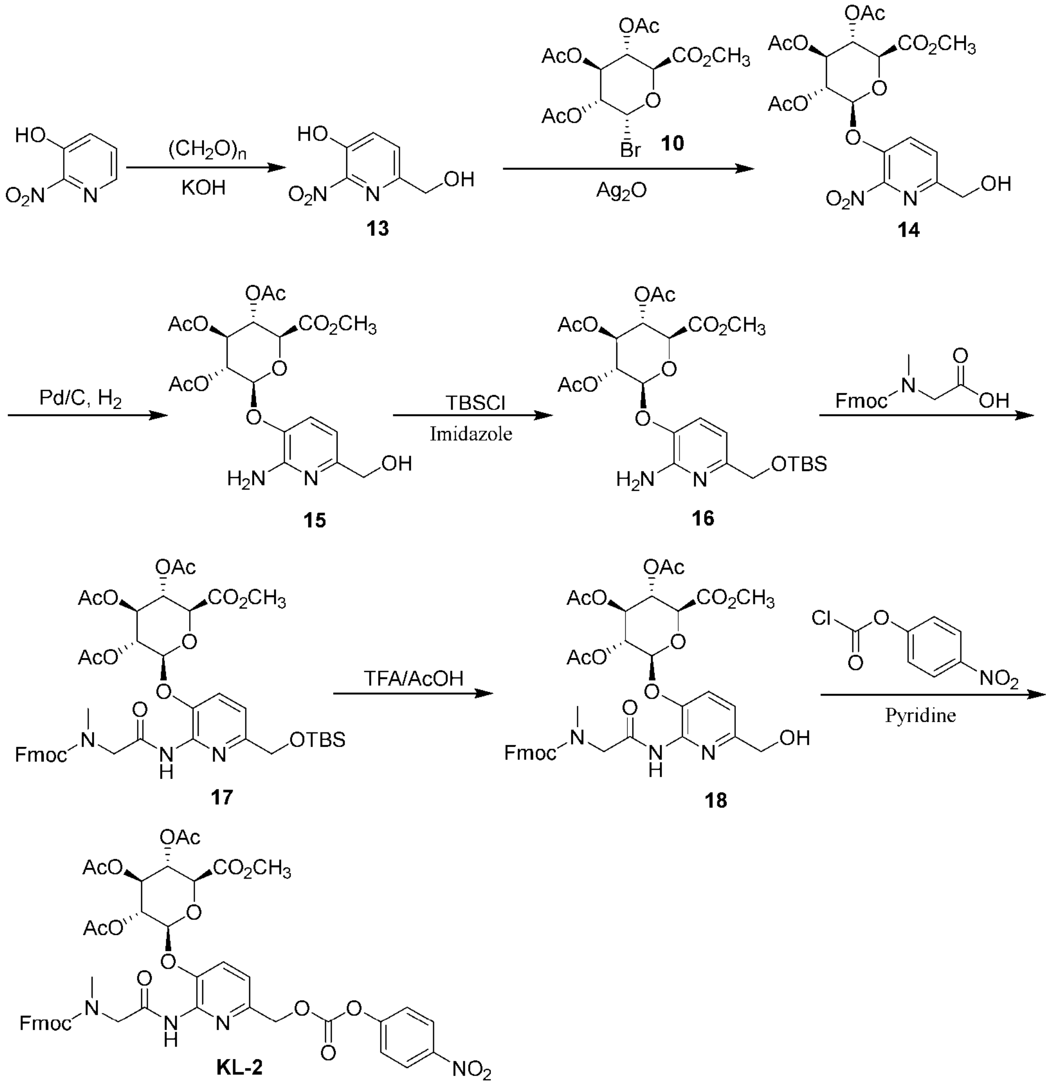


Step 1: Preparation of compound **13**

2-Nitro-3-hydroxypyridine (7 g, 50 mmol) was dissolved in a 37% formaldehyde aqueous solution (100 mL), and an aqueous solution of potassium hydroxide (2.8 g, 50 mmol) was added dropwise thereto. The reaction solution was stirred under reflux for 4 days. The reaction solution was cooled to room temperature, and a 1 N hydrochloric acid solution (50 mL) was added dropwise thereto. The resulting solution was extracted with ethyl acetate (100 mL × 3). The combined organic phases were dried over anhydrous sodium sulfate, filtered, and concentrated under reduced pressure to remove the solvent, yielding a brownish oil residue. The residues were purified by silica gel column chromatography (eluent: petroleum ether/ethyl acetate (1/10 → 1/1)) to obtain compound **13** as a yellow solid (2.0 g).

LCMS (method 1): Rt = 1.18 min, m/z ($ES^+$) 171.1 $[M+H]^+$

$^1H$ NMR (500 MHz, $CDCl_3$) 6 10.20 (s, 1 H), 7.66-7.71 (m, 2 H), 4.80 (s, 2 H), 3.10 (s, 1 H).

Step 2: Preparation of compound **14**

Compound **13** (800 mg, 4.7 mmol) was dissolved in acetonitrile (50 mL), and compound **10** (1.5 g, 3.76 mmol) and silver oxide (2.17 g, 9.4 mmol) were added under ice-water bath successively. The reaction solution was stirred at room temperature for 1 hour. The mixed solution was filtered, and the filter cake was washed with acetonitrile (50 mL × 2). The filtrate was combined and concentrated under reduced pressure to remove the solvent. The residues were purified by silica gel column chromatography (eluent: ethyl acetate/petroleum ether (1/2 → 3/1)) to obtain compound **14** as a yellow solid (400 mg).

LCMS (method 3): Rt = 1.72 min, m/z (ES+) 509.0 $[M+Na]^+$.

$^{1}H$ NMR (500 MHz, $CDCl_3$) $\delta$ 7.87 (d, 1H), 7.64 (d, 1H), 5.30-5.38 (m, 2H), 5.21-5.26 (m, 2H), 4.79 (s, 2H), 4.23 (d, 1H), 3.74 (s, 3H), 2.14 (s, 3H), 2.07 (d, 6H).

Step 3: Preparation of compound **15**

Compound **14** (520 mg, 1.02 mmol) was dissolved in ethyl acetate (100 mL), and 10% palladium on carbon (Pd/C, 52 mg) was added thereto. The reaction solution was stirred and hydrogenated at room temperature overnight. The reaction solution was filtered to remove palladium on carbon, and the filtrate was concentrated under reduced pressure to remove the solvent, yielding compound **15** as a yellow oil (450 mg).
LCMS (method 3): Rt = 1.64 min, *m/z* ($ES^+$) 457.1 $[M+H]^+$.

Step 4: Preparation of compound **16**

Compound **15** (450 mg, 1 mmol) was dissolved in dichloromethane (20 mL), and imidazole (102 mg, 1.5 mmol) and *tert*-butyldimethylsilyl chloride (TBSCl, 225 mg, 1.5 mmol) were added successively. The reaction solution was stirred at room temperature for 2 hours. The mixture was concentrated under reduced pressure to remove the solvent, and the residues were purified by silica gel column chromatography (eluent: ethyl acetate/petroleum ether (1/10 → 1/2)) to obtain compound **16** as a yellow solid (350 mg).
LCMS (method 3): Rt = 2.16 min, m/z ($ES^+$) 571.2 $[M+H]^+$.

Step 5: Preparation of compound **17**

Fmoc-sarcosine (286 mg, 0.921 mmol) was dissolved in dichloromethane (10 mL), and sulfoxide chloride (367 mg, 1.84 mmol) and a trace amount of N,N-dimethylformamide (4.5 mg, 0.0614 mmol) were added successively. The reaction solution was stirred at room temperature for 2 hours. The reaction solution was concentrated under reduced pressure to remove the solvent. Dichloromethane (10 mL) was added to the residues, and then a solution of compound **16** (350 mg, 0.614 mmol) and DIPEA (158 mg, 1.23 mmol) in dichloromethane (10 mL) was added thereto. The resulting reaction solution was stirred at room temperature overnight. The reaction solution was concentrated under reduced pressure to remove the solvent, and the residues were purified by silica gel column chromatography (eluent: ethyl acetate/petroleum ether (1/10 → 1/1)) to obtain compound **17** as a yellow solid (200 mg).
LCMS (method 1): R*t* = 2.23 min, m/z ($ES^+$) 864.3 $[M+H]^+$.

Step 6: Preparation of compound **18**

Compound **17** (190 mg, 0.22 mmol) was dissolved in tetrahydrofuran (6 mL), and glacial acetic acid (AcOH, 2 mL) and water (2 mL) were added thereto. The reaction solution was stirred at room temperature for 2 days. Saturated sodium bicarbonate solution (30 mL) was added to the reaction solution, followed by extraction with ethyl acetate (30 mL × 3). The combined organic phases were dried over anhydrous sodium sulfate, filtered, and concentrated under reduced pressure to remove the solvent, yielding a yellow oil. The residues were purified by silica gel column chromatography (eluent: methanol/dichloromethane (0/1 → 1/20)) to obtain compound **18** as a yellow solid (100 mg). LCMS (method 1): R*t* = 1.86 min, m/z ($ES^+$) 750.2 $[M+H]^+$.

Step 7: Preparation of compound **KL-2**

Compound **18** (87 mg, 0.116 mmol), 4-nitrophenyl chloroformate (35 mg, 0.174 mmol), and pyridine (13.7 mg, 0.174 mmol) were added to dichloromethane (4 mL) successively, and the reaction solution was stirred at room temperature overnight. The reaction solution was concentrated under reduced pressure to remove the solvent, and the residues were purified by silica gel column chromatography (eluent: ethyl acetate/petroleum ether (1/10 → 1/2)) to obtain compound **KL-2** as a white solid (45 mg).
LCMS (method 1): Rt = 2.08 min, m/z ($ES^+$) 915.2 $[M+H]^+$.

Example 3: Synthesis of linker intermediate **KL-3**

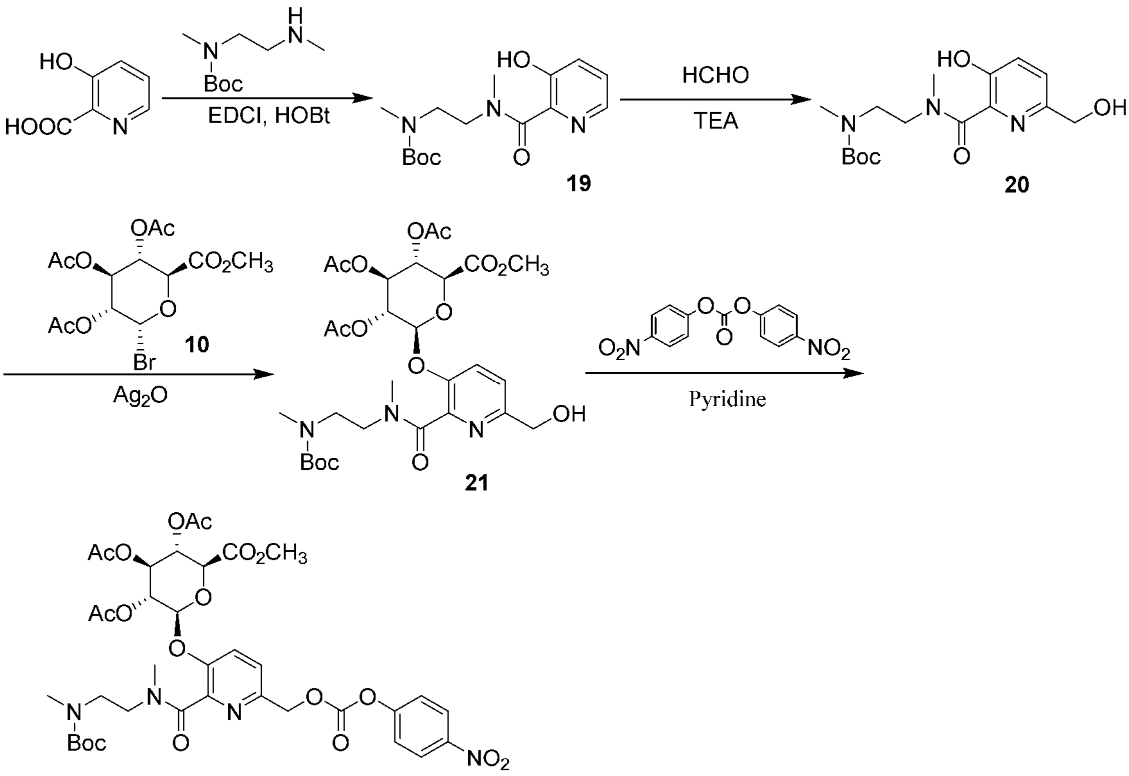


19

20

10

21

KL-3

Step 1: Preparation of compound **19**

*Tert*-butyl methyl(2-(methylamino)ethyl)carbamate (6.00 g, 31.6 mmol), 2-carboxy-3-hydroxypyridine (4.00 g, 28.8 mmol), 1-hydroxybenzotriazole (HOBt, 4.30 g, 31.6 mmol), 1-ethyl-(3-dimethylaminopropyl)carbodiimide hydrochloride (EDCI, 6.10 g, 31.6 mmol), and diisopropylethylamine (5.60 g, 43.2 mmol) were added to N,N'-dimethylformamide (60 mL) successively, and the reaction solution was stirred at room temperature for 16 hours. Water (300 mL) was added to the reaction solution, followed by extraction with ethyl acetate (150 mL × 3). The combined organic phases were washed with saturated brine (200 mL × 2), dried over anhydrous sodium sulfate, filtered, and concentrated under reduced pressure to remove the solvent. The residues were purified by silica gel column chromatography (eluent: ethyl acetate/petroleum ether (1/5)) to obtain compound **19** as an off-white solid (6.96 g).

LCMS (method 3): R*t* = 1.77 min, m/z ($ES^+$) 310.2 $(M+H)^+$.

Step 2: Preparation of compound **20**

Compound **19** (4.80 g, 15.5 mmol), triethylamine (3.13 g, 31 mmol), and 37% formaldehyde (HCHO, 3.77 g, 46.5 mmol) were added to water (50 mL) successively, and the reaction solution was stirred at 90°C for 16 hours. The reaction solution was cooled to room temperature, neutralized by adding glacial acetic acid, and then extracted with ethyl acetate (100 mL × 3). The combined organic phases were dried over anhydrous sodium sulfate, filtered, and concentrated under reduced pressure to remove the solvent. The residues were purified by silica gel column chromatography (eluent: ethyl acetate/petroleum ether (1/2)) to obtain compound **20** as an off-white solid (1.4 g).

LCMS (method 3): Rt = 1.63 min, *m/z* ($ES^+$) 340.2 $(M+H)^+$.

Step 3: Preparation of compound **21**

Compound **20** (339 mg, 1.0 mmol), compound **10** (397 mg, 1.0 mmol), and silver oxide (462 mg, 2.0 mmol) were added to acetonitrile (50 mL) successively, and the reaction solution was stirred at 40°C protected from light for 16 hours. After cooling to room temperature, the reaction solution was filtered, and the filter cake was washed with acetonitrile. The combined filtrates were concentrated under reduced pressure to remove the solvent. The residues were purified by prep-RP-HPLC (method 5: 40%-95% B in 8 min → 95% B in 4 min) to obtain compound **21** as a white solid (120 mg).

LCMS (method 3): Rt = 1.84 min, m/z ($ES^+$) 656.3 $(M+H)^+$.

Step 4: Preparation of compound **KL-3**

Compound **21** (100 mg, 0.15 mmol), bis(4-nitrophenyl) carbonate (48 mg, 0.24 mmol), and pyridine (20 mg, 0.25 mmol) were added to dichloromethane (3 mL) successively under ice-bath, and the reaction solution was stirred at room temperature for 16 hours. Water (20 mL) was added to the reaction solution, followed by extraction with dichloromethane (30 mL × 3). The combined organic phases were dried over anhydrous sodium sulfate, filtered, and concentrated under reduced pressure to remove the solvent. The residues were purified by silica gel column chromatography (eluent: ethyl acetate/petroleum ether (1/10)) to obtain compound **KL-3** as an off-white solid (100 mg).

LCMS (method 3): Rt = 2.06 min, m/z ($ES^+$) 821.3 $(M+H)^+$.

Example 4: Synthesis of linker intermediate **KL-4**

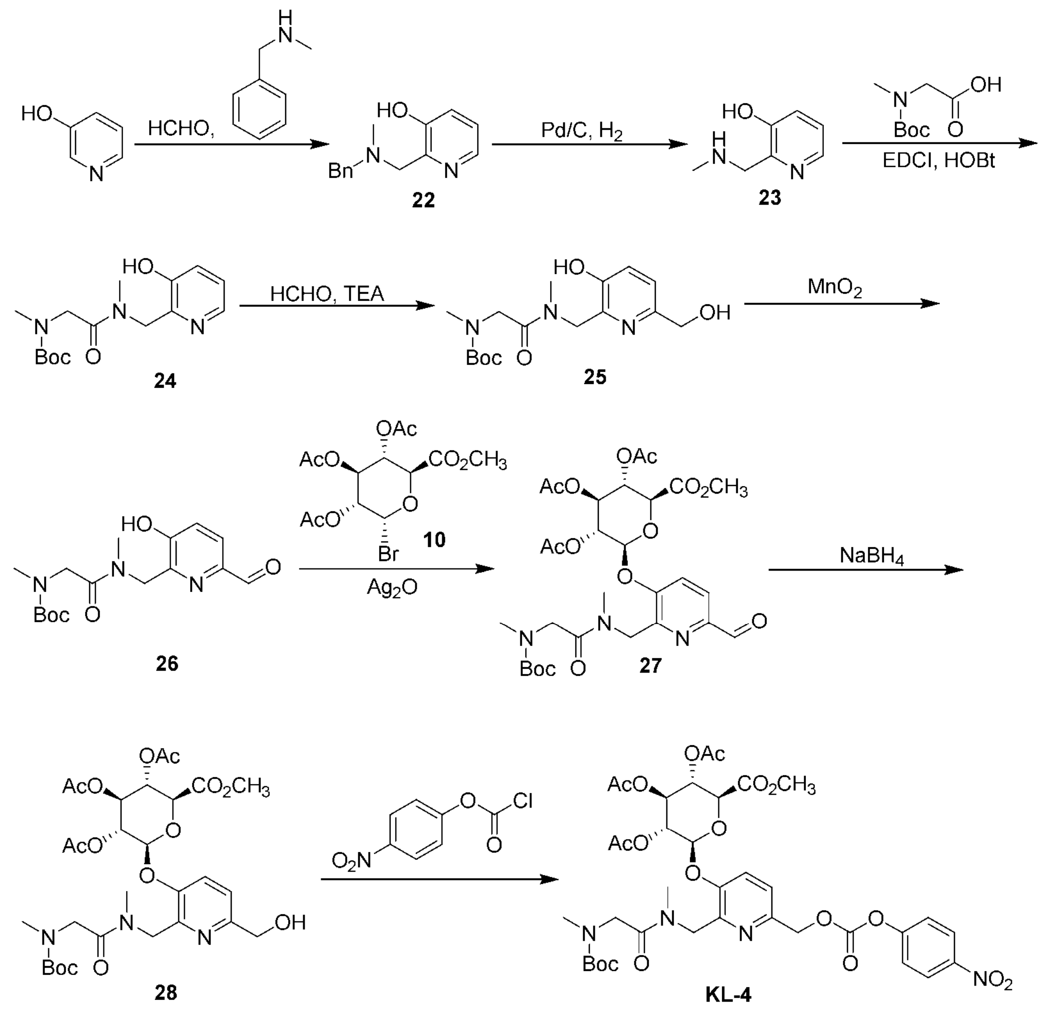


Step 1: Preparation of compound **22**

3-Hydroxypyridine (9.50 g, 100 mmol), 37% formaldehyde solution (12.20 g, 150 mmol), N-methylbenzylamine (11.5 g, 95 mmol), and concentrated hydrochloric acid (8.5 mL, 100 mmol) were added to ethanol (120 mL) successively, and the reaction solution was stirred at 85°C overnight. 10% Sodium hydroxide solution was added to adjust the pH to neutral. The ethanol was removed by concentration under reduced pressure, and the mixture was extracted with ethyl acetate (80 mL). The organic phase was dried over anhydrous sodium sulfate, filtered, and concentrated under reduced pressure to remove the solvent. The residues were purified by silica gel column chromatography (eluent: dichloromethane/methanol (150/1)) to obtain compound **22** as a light yellow solid (11.50 g).

LCMS (method 3): R*t* = 1.87 min, m/z ($ES^+$) 229.1 $(M+H)^+$.

Step 2: Preparation of compound **23**

Compound **22** (12.5 g, 55 mmol) was dissolved in methanol (100 mL), and 10% palladium on carbon (1.0 g) was added thereto. The reaction solution was stirred under hydrogenation conditions at room temperature for 16 hours. The reaction solution was filtered, and the filtrate was concentrated under reduced pressure to remove the solvent. The residues were purified by silica gel column chromatography (eluent: dichloromethane/methanol (10/1)) to obtain compound **23** as a pink solid (6.6 g).
LCMS (method 3): Rt = 0.38 min, m/z ($ES^+$) 139.2 $(M+H)^+$.

Step 3: Preparation of compound **24**

Boc-sarcosine (6.24 g, 33 mmol), compound **23** (5.10 g, 37 mmol), 1-hydroxybenzotriazole (5.00 g, 37 mmol), 1-ethyl-(3-dimethylaminopropyl)carbodiimide hydrochloride (7.10 g, 37 mmol), and diisopropylethylamine (7.22 g, 56 mmol) were added to DMF (80 mL) successively, and the reaction solution was stirred at room temperature for 16 hours. The reaction solution was poured into 300 mL of water and extracted with ethyl acetate (200 mL × 3). The combined organic phases were washed with saturated brine (300 mL × 2), dried over anhydrous sodium sulfate, filtered, and concentrated under reduced pressure to remove the solvent. The residues were purified by silica gel column chromatography (eluent: dichloromethane/methanol (80/1 → 30/1)) to obtain compound **24** as a pale yellow solid (7.8 g).
LCMS (method 3): Rt = 1.75 min, m/z ($ES^+$) 310.1 $(M+H)^+$.
$^1$HNMR (400 MHz, DMSO-$d_6$) $\delta$ 7.98-7.97 (m, 1H), 7.19-7.13 (m, 2H), 4.56-4.51 (m, 2H), 4.15-4.04 (m, 2H), 2.99-2.98 (m, 1H), 2.84 (s, 1H), 2.79-2.76 (m, 3H), 2.72 (s, 1H), 1.40-1.38 (m, 4H), 1.28 (s, 5H).

Step 4: Preparation of compound **25**

Compound **24** (7.72 g, 25 mmol), triethylamine (5.05 g, 50 mmol), and 37% formaldehyde solution (6.08 g, 75 mmol) were added to water (60 mL) successively, and the reaction solution was stirred at 90°C for 16 hours. The reaction solution was cooled to room temperature, acetic acid was added thereto to adjust the pH to neutral, and the mixture was extracted with ethyl acetate (60 mL × 3). The combined organic phases were dried over anhydrous sodium sulfate, filtered, and concentrated under reduced pressure to remove the solvent. The residues were purified by silica gel column chromatography (eluent: dichloromethane/methanol (50/1 → 20/1)) to obtain compound **25** as an off-white solid (1.32 g).
LCMS (method 3): Rt = 1.67 min, m/z ($ES^+$) 340.1 $(M+H)^+$.

Step 5: Preparation of compound **26**

Compound **25** (1.32 g, 3.9 mmol) was dissolved in dichloromethane (25 mL), and active manganese dioxide ($MnO_2$, 1.35 g, 15.6 mmol) was added thereto. The reaction solution was stirred under reflux for 16 hours. After cooling to room temperature, the reaction solution was filtered, and the filtrate was concentrated under reduced pressure to remove the solvent. The residues were purified by silica gel column chromatography (eluent: dichloromethane/methanol (100/1 → 80/1)) to obtain compound **26** as an off-white solid (0.86 g).
LCMS (method 3): Rt = 1.55 min, m/z ($ES^+$) 338.1 $(M+H)^+$.

Step 6: Preparation of compound **27**

Compound **26** (438 mg, 1.3 mmol), compound **10** (560 mg, 1.4 mmol), and silver oxide (534 mg, 2.3 mmol) were added to acetonitrile (8 mL) successively, and the reaction solution was stirred at room temperature protected from light for 16 hours. The reaction solution was filtered, and the filtrate was concentrated under reduced pressure to remove the solvent. The residues were purified by prep-RP-HPLC (method 5: 40%-95% B in 8 min → 95% B in 4 min) to obtain compound **27** as a white solid (112 mg).
LCMS (method 3): Rt = 1.96 min, m/z ($ES^+$) 654.2 $(M+H)^+$.

Step 7: Preparation of compound **28**

Compound **27** (112 mg, 0.17 mmol) was dissolved in a mixed solution of isopropanol (1 mL) and dichloromethane (2 mL). Sodium borohydride (10 mg, 0.25 mmol) was added thereto under ice-bath, and the reaction solution was stirred at room temperature for 2 hours. Water (20 mL) was added to the reaction solution, followed by extraction with dichloromethane (20 mL × 3). The combined organic phases were dried over anhydrous sodium sulfate, filtered, and concentrated under reduced pressure to remove the solvent. The residues were purified by silica gel column chromatography (eluent: dichloromethane/methanol (60/1)) to obtain compound **28** as an off-white solid (100 mg).

LCMS (method 3): Rt = 1.81 min, m/z ($ES^+$) 656.0 $(M+H)^+$.

Step 8: Preparation of compound **KL-4**

Compound **28** (92 mg, 0.14 mmol) and 4-nitrophenyl chloroformate (56 mg, 0.28 mmol) were added to dichloromethane (4 mL). Triethylamine (43 mg, 0.42 mmol) was added thereto under ice-bath, and the reaction solution was stirred at room temperature for 16 hours. The reaction solution was concentrated under reduced pressure to remove the solvent, and the residues were purified by silica gel column chromatography (eluent: petroleum ether/ethyl acetate (10/1 $\rightarrow$ 3/1)) to obtain compound **KL-4** as an off-white solid (41 mg).

LCMS (method 3): Rt = 2.03 min, *m*/*z* ($ES^+$) 820.9 $(M+H)^+$.

Example 5: Synthesis of linker intermediate **KL-5**

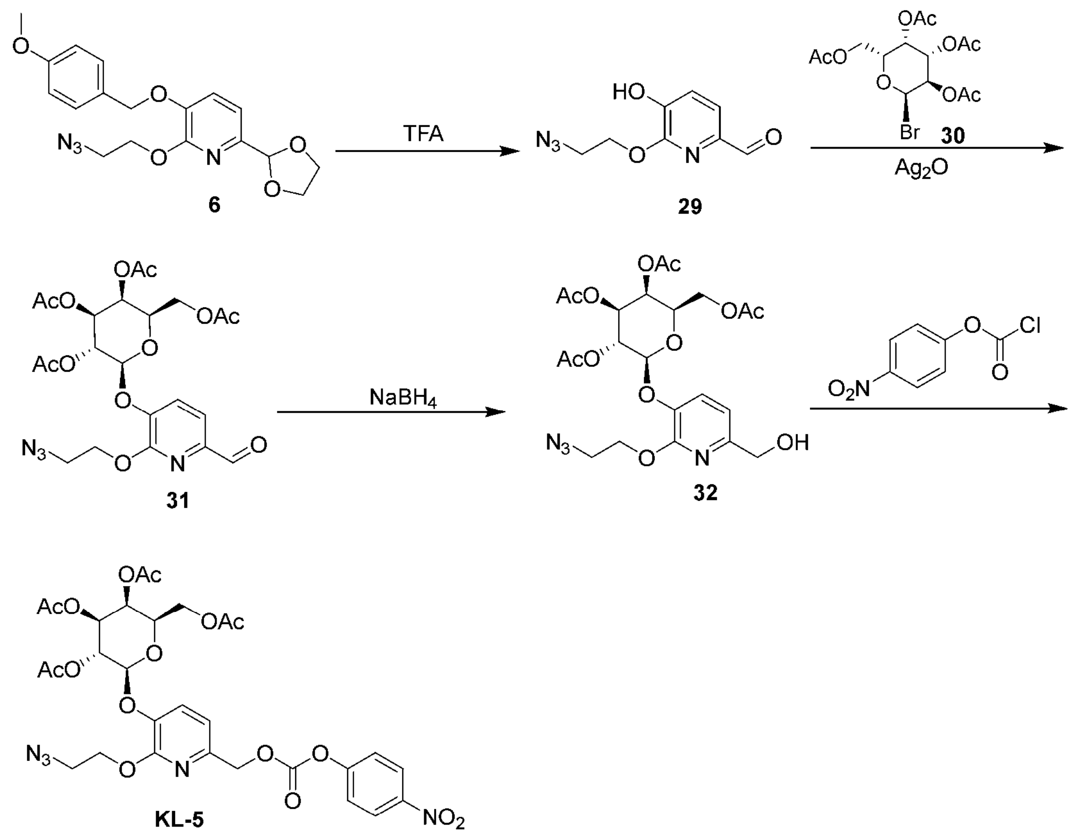

6 29 31 32 KL-5

Step 1: Preparation of compound **29**

Compound **6** (4.5 g, 12 mmol) was dissolved in a mixed solution of dichloromethane (16 mL) and water (0.4 mL). Trifluoroacetic acid (8 mL) was added thereto under ice-bath, and the reaction solution was stirred at room temperature for 2 hours. Saturated sodium bicarbonate solution (40 mL) was added to the reaction solution to quench the reaction, followed by extraction with dichloromethane (50 mL $\times$ 3). The combined organic phases were dried over anhydrous sodium sulfate, filtered, and concentrated under reduced pressure to remove the solvent. The residues were purified by silica gel column chromatography (eluent: ethyl acetate/dichloromethane/petroleum ether (1/1/3)) to obtain compound **29** as a colorless oil (1.64 g).

LCMS (method 3): R*t* = 0.67 min, m/z ($ES^+$) 209.1 $(M+H)^+$.

Step 2: Preparation of compound **31**

Compound **29** (422 mg, 2.03 mmol), compound **30** (1.0 g, 2.43 mmol, synthesized with reference to the method disclosed in US2019/367488), and silver oxide (938 mg, 4.06 mmol) were added to acetonitrile (50 mL) successively, and

the reaction solution was stirred at room temperature protected from light for 17 hours. The reaction solution was filtered, and the filter cake was washed with tetrahydrofuran (15 mL × 3). Then, the combined filtrates were concentrated under reduced pressure to remove the solvent. The residues were purified by silica gel column chromatography (eluent: ethyl acetate/dichloromethane/petroleum ether (1/1/4 → 1/1/2)) to obtain compound 31 as a colorless oil (874 mg).

LCMS (method 3): R*t* = 1.89 min, *m*/*z* ($ES^+$) 539.1 $(M+H)^+$.

Step 3: Preparation of compound **32**

Compound **31** (874 mg, 1.62 mmol) was dissolved in a mixed solution of isopropanol (10 mL) and dichloromethane (20 mL). Then, sodium borohydride (92 mg, 2.43 mmol) was added thereto under ice-water bath, and the reaction solution was stirred in the ice-water bath for 1 hour. Water (50 mL) was added to the reaction solution, followed by extraction with ethyl acetate (50 mL × 3). The combined organic phases were dried over anhydrous sodium sulfate, filtered, and concentrated under reduced pressure to remove the solvent to obtain compound **32** as a colorless oil (850 mg).

LCMS (method 1): Rt = 1.63 min, m/z ($ES^+$) 541.2 $(M+H)^+$.

Step 4: Preparation of compound **KL-5**

Compound **32** (850 mg, 1.57 mmol) was dissolved in dry dichloromethane (10 mL), and then 4-nitrophenyl chloroformate (476 mg, 2.36 mmol) and pyridine (373 mg, 4.72 mmol) were added thereto successively. The reaction solution was stirred at room temperature for 17 hours. The reaction solution was concentrated under reduced pressure to remove the solvent, and the residues were purified by silica gel column chromatography (eluent: ethyl acetate/petroleum ether (1/3 → 1/1)) to obtain compound **KL-5** as a colorless oil (950 mg).

LCMS (method 1): Rt = 2.10 min, m/z ($ES^+$) 706.1 $(M+H)^+$.

Example 6: Synthesis of linker-drug **LD-01**

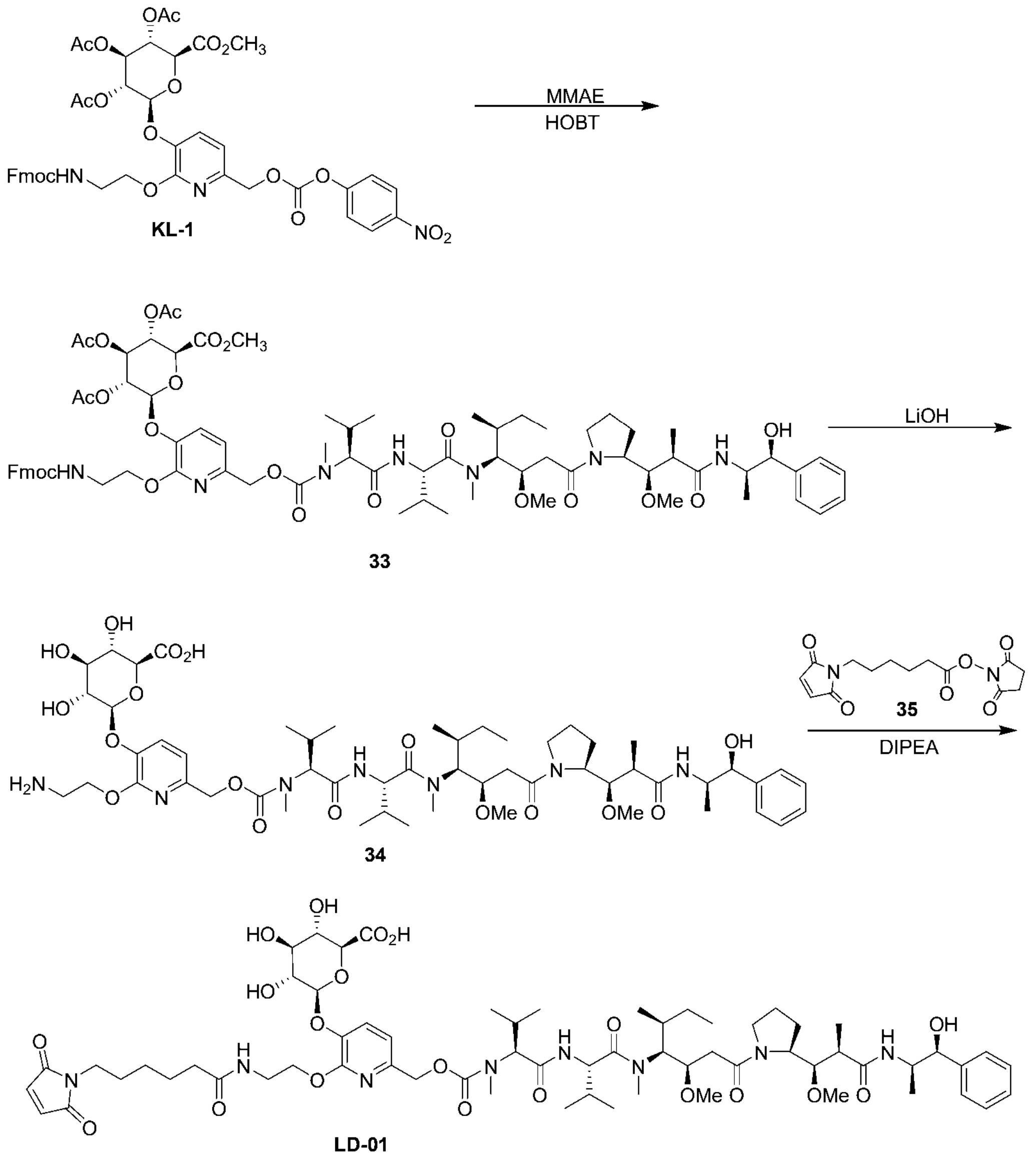


KL-1

33

34

LD-01

Step 1: Preparation of compound **33**

Compound **KL-1** (240 mg, 0.271 mmol), 1-hydroxybenzotriazole (18.4 mg, 0.136 mmol), pyridine (0.5 mL), and MMAE (195 mg, 0.271 mmol, Haoyuan Pharmaceutical) were added to dry N,N-dimethylformamide (2 mL) successively, and the reaction solution was stirred at room temperature for 17 hours. Acetic acid was added to the reaction solution to quench the reaction, which was then purified by prep-RP-HPLC (method 4: 60%-95% B in 8 min → 95% B in 4 min) to obtain compound **33** as a white solid (200 mg).

LCMS (method 1): Rt = 1.98 min, *m/z* ($ES^+$) 1467.5 $(M+H)^+$.

Step 2: Preparation of compound **34**

Compound **33** (200 mg, 0.136 mmol) was dissolved in methanol (10 mL), and then an aqueous solution of lithium hydroxide (1 M, 1.36 mL, 1.36 mmol) was added thereto. The reaction solution was stirred at room temperature for 3 hours, then quenched by adding glacial acetic acid (150 mg), and purified by prep-RP-HPLC (method 4: 45%-95% B in 8 min → 95% B in 4 min) to obtain compound **34** as a white solid (100 mg).

LCMS (method 2): Rt = 1.21 min, *m/z* ($ES^+$) 1104.4 $(M+H)^+$.

Step 3: Preparation of compound **LD-01**

Compound **34** (20 mg, 0.018 mmol) was dissolved in N,N-dimethylformamide (1 mL), and then compound **35** (6.7

mg, 0.022 mmol, Haoyuan Pharmaceutical) and diisopropylethylamine (9.3 mg, 0.072 mmol) were added thereto successively. The reaction solution was stirred at room temperature for 2 hours, then quenched by adding glacial acetic acid (24 mg), and the resulting solution was purified by prep-RP-HPLC (method 4: 50%-95% B in 8 min → 95% B in 4 min) to obtain compound **LD-01** as a white solid (13.4 mg).

LCMS (method 1): Rt = 1.70 min, m/z (ES$^+$) 649.5 [1/2(M+2H)]$^+$.

Example 6: Synthesis of linker-drug **LD-02**

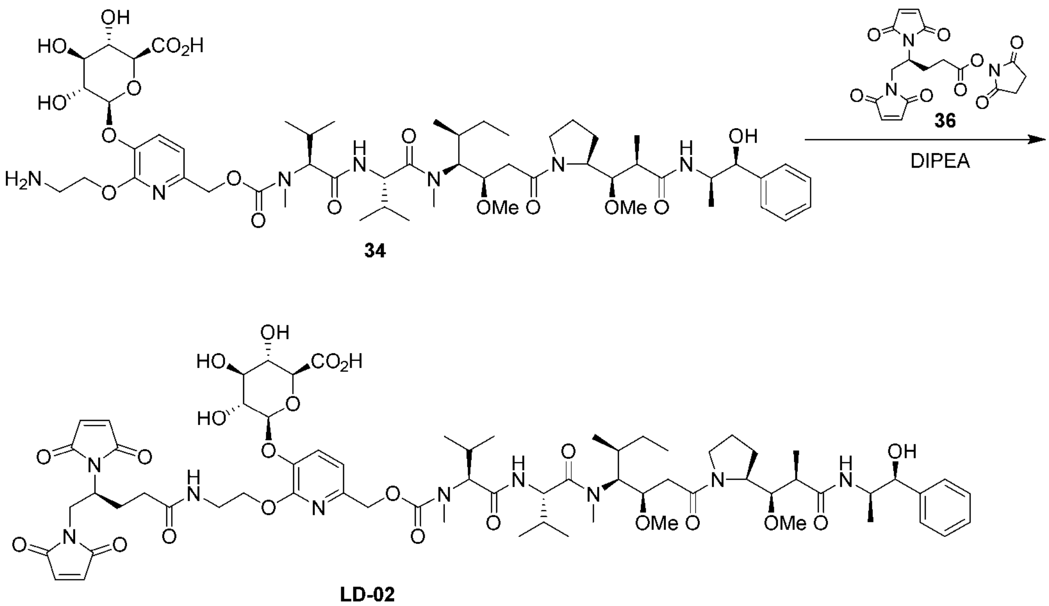


**36**

**34**

**LD-02**

Compound **34** (55 mg, 0.05 mmol) was dissolved in N,N-dimethylformamide (2 mL), and then compound **36** (23 mg, 0.06 mmol, synthesized with reference to the method disclosed in EP2949343, 2015, A1) and diisopropylethylamine (25.8 mg, 0.2 mmol) were successively added thereto. The reaction solution was stirred at room temperature for 2 hours, then quenched by adding glacial acetic acid (24 mg), and the resulting solution was purified by prep-RP-HPLC (method 4: 50%-95% B in 8 min → 95% B in 4 min) to obtain compound **LD-02** as a white solid (50 mg).

LCMS (method 2): Rt = 1.36 min, m/z (ES$^+$) 1378.4 (M+H)$^+$.

Example 7: Synthesis of linker-drug **LD-03**

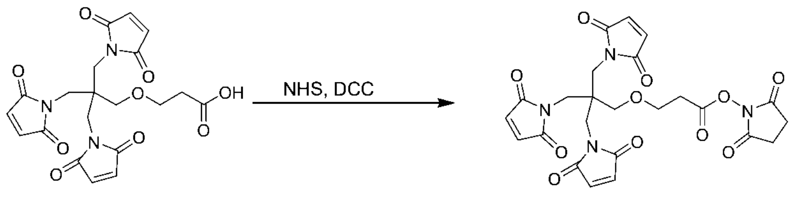


37 38

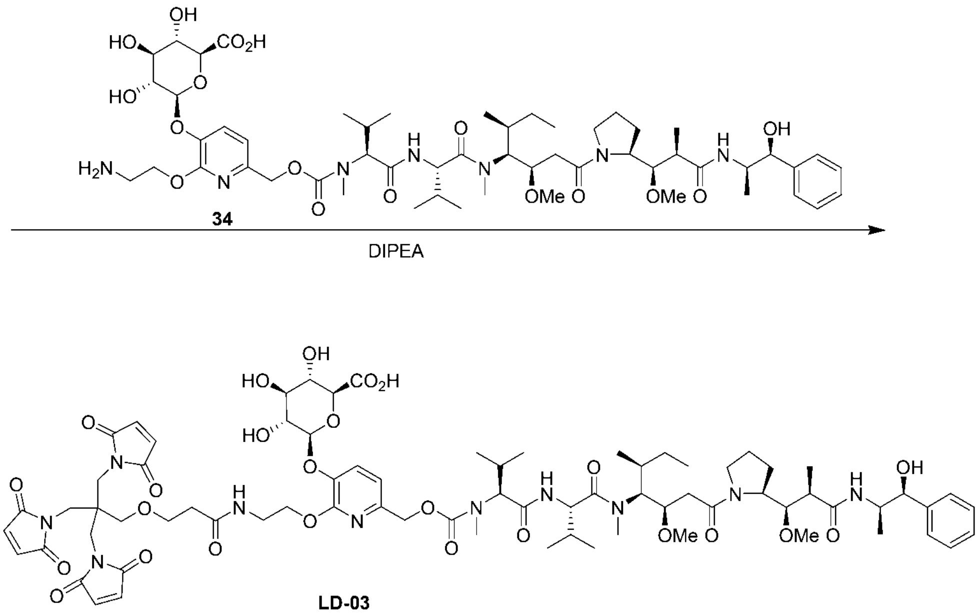


34

LD-03

Step 1: Preparation of compound **33**

Compound **37** (100 mg, 0.225 mmol, synthesized with reference to the method disclosed in WO2016/192528) was dissolved in tetrahydrofuran, and then N-hydroxysuccinimide (NHS, 39 mg, 0.34 mmol) and dicyclohexylcarbodiimide (DCC, 70 mg, 0.34 mmol) were added thereto successively. The reaction solution was stirred at room temperature for 17 hours. The reaction solution was concentrated under reduced pressure to remove the solvent, and the residues were purified by silica gel column chromatography (eluent: methanol/dichloromethane (0/1 → 1/30)) to obtain compound **37** as a yellow solid (60 mg).

Step 2: Preparation of compound **LD-03**

Compound **34** (20 mg, 0.018 mmol) was dissolved in N,N-dimethylformamide (2 mL), and then compound **38** (15 mg, 0.027 mmol) and diisopropylethylamine (10 mg, 0.076 mmol) were added thereto successively. The reaction solution was stirred at room temperature for 2 hours and then quenched by adding glacial acetic acid (24 mg). The resulting solution was purified by prep-RP-HPLC (method 4: 30%-95% B in 8 min → 95% B in 4 min) to obtain compound **LD-03** as a white solid (9.9 mg).

LCMS (method 1): Rt =1.75 min, *m/z* ($ES^+$) 766.5 $[1/2(M+2H)]^+$.

Example 8: Synthesis of linker-drug **LD-04**

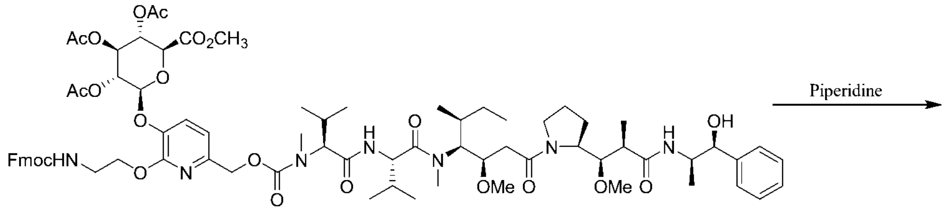


33

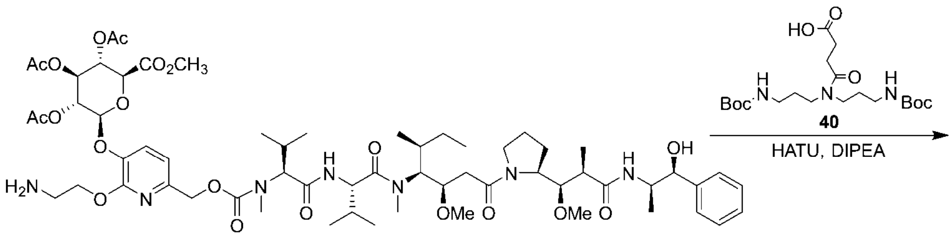


39

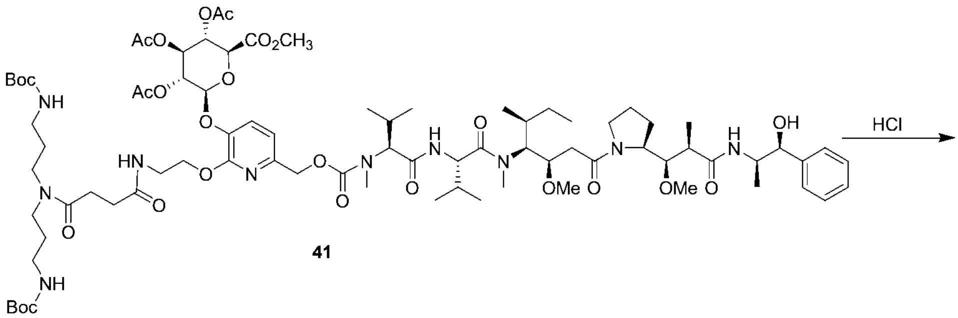


41

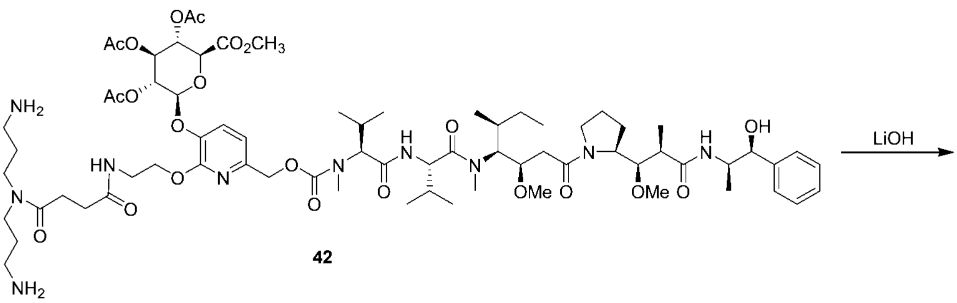


42

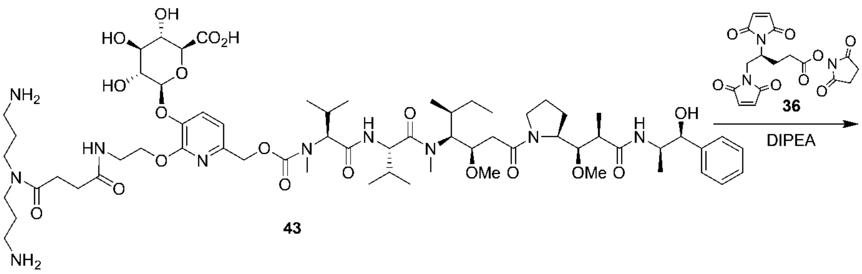


43

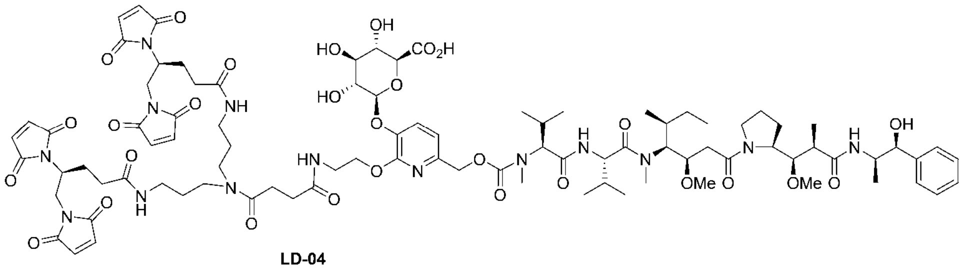

LD-04

Step 1: Preparation of compound **39**

Piperidine (0.35 g, 4.1 mmol) was added to a solution of compound **33** (300 mg, 0.2 mmol) in tetrahydrofuran (30 mL), and the reaction solution was stirred at room temperature for 17 hours. The reaction solution was purified by prep-RP-HPLC (method 4: 15%-85% B in 8 min → 95% B in 4 min) to obtain compound **39** as a white solid (200 mg).
LCMS (method 1): R*t* =1.63 min, *m*/*z* ($ES^+$) 623.0 $[1/2(M+2H)]^+$.

Step 2: Preparation of compound **40**

Compound **39** (98 mg, 0.079 mmol), compound **40** (41 mg, 0.095 mmol, synthesized with reference to the method disclosed in CN107652219B), diisopropylethylamine (49 mg, 0.38 mmol), and HATU (60 mg, 0.098 mmol) were added to N,N-dimethylformamide (5 mL) successively, and the resulting reaction solution was stirred at room temperature for 17 hours. Water (30 mL) was added to the reaction solution to quench the reaction, and the resulting solution was extracted with ethyl acetate (25 mL × 3). The combined organic phases were washed with saturated brine (30 mL × 2), dried over anhydrous sodium sulfate, filtered, and concentrated under reduced pressure to obtain crude compound **41** as a yellow solid (120 mg).
LCMS (method 1): Rt = 2.01 min, m/z ($ES^+$) 829.6 $[1/2(M+2H)]^+$.

Step 3: Preparation of compound **42**

Compound **41** (120 mg, 0.079 mmol) was added to a mixed solution of hydrochloric acid in dioxane (4 M, 5 mL) and ethyl acetate (5 mL), and the resulting reaction solution was stirred at room temperature for 2 hours. The reaction solution was concentrated under reduced pressure to obtain crude compound **42** as a yellow solid (100 mg).
LCMS (method 1): R*t* = 1.48 min, m/z ($ES^+$) 729.5 $[1/2(M+2H)]^+$.

Step 4: Preparation of compound **43**

Compound **42** (100 mg, 0.069 mmol) was added to a mixed solution of methanol (1 mL) and water (2 mL), and then lithium hydroxide monohydrate ($LiOH\text{-}H_2O$, 84 mg, 2 mmol) was added thereto. The reaction solution was stirred at room temperature for 2 hours, then quenched by adding glacial acetic acid (120 mg), and the resulting solution was purified by prep-RP-HPLC (method 4: 15%-85% B in 8 min → 95% B in 4 min) to obtain compound **43** as a white solid (14 mg).
LCMS (method 1): Rt = 1.38 min, m/z ($ES^+$) 659.5 $[1/2(M+2H)]^+$.

Step 4: Preparation of compound **LD-04**

Compound **43** (14 mg, 0.010 mmol), compound **36** (8.2 mg, 21.2 μmol), and diisopropylethylamine (6 mg, 70.41 μmol) were added to dry N,N-dimethylformamide (1 mL) successively. The resulting reaction solution was stirred at room temperature for 2 hours, and then glacial acetic acid (10 mg) was added to quench the reaction. The reaction solution was purified by prep-RP-HPLC (method 4: 15%-85% B in 8 min → 95% B in 4 min) to obtain compound **LD-04** as a white solid (14 mg).
LCMS (method 1): Rt = 1.64 min, *m*/*z* ($ES^+$) 933.6 $[1/2(M+2H)]^+$.

Example 9: Synthesis of linker-drug **LD-05**

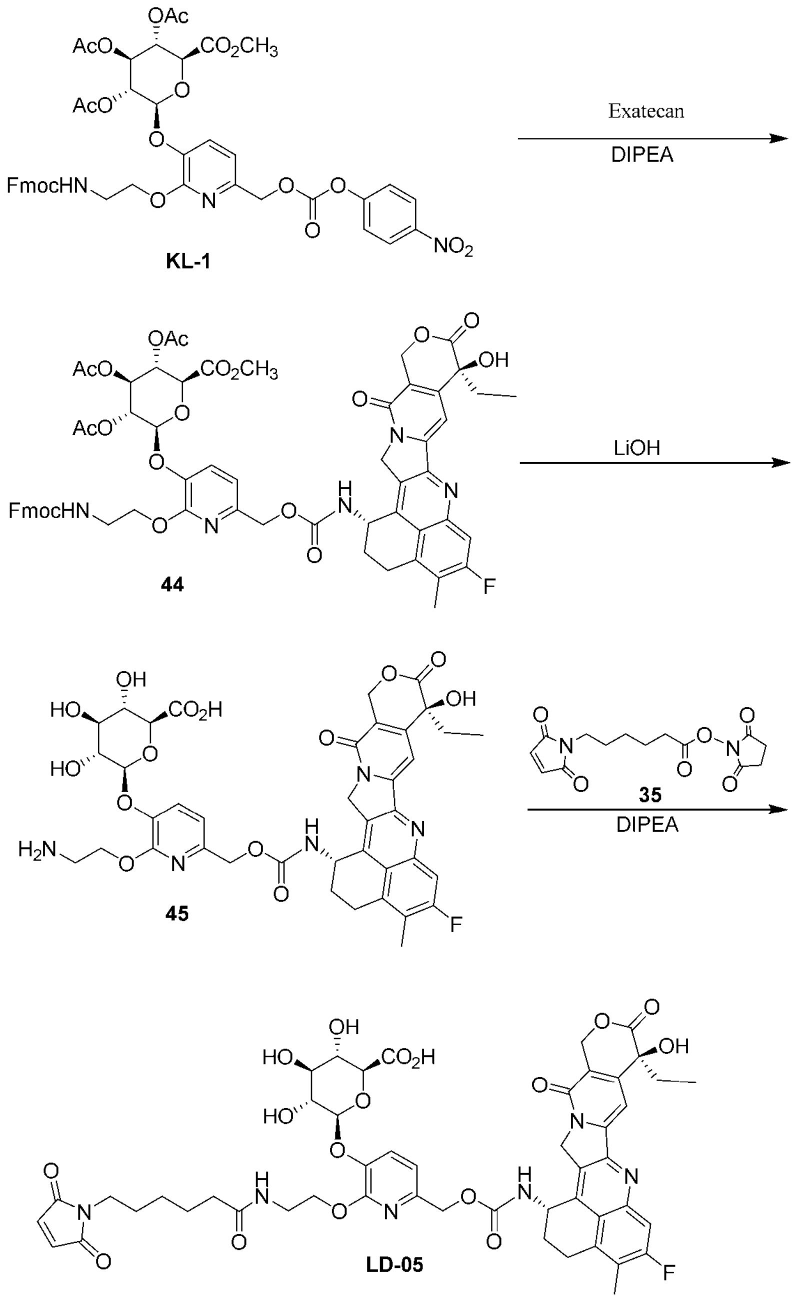


Step 1: Preparation of compound **44**

Compound **KL-1** (500 mg, 0.564 mmol), HOBt (38 mg, 0.282 mmol), pyridine (2 mL), diisopropylethylamine (291 mg, 2.256 mmol), and Exatecan (300 mg, 0.564 mmol, Haoyuan Pharmaceutical) were added to dry N,N-dimethylformamide (20 mL) successively, and the resulting reaction solution was stirred at room temperature for 17 hours. Water (150 mL) was added to the reaction solution, followed by extraction with ethyl acetate (500 mL × 4). The combined organic phases were washed with saturated brine (600 mL × 2), dried over anhydrous sodium sulfate, filtered, and concentrated under reduced pressure to remove the solvent. The obtained brown oil was purified by silica gel column chromatography (eluent: methanol/dichloromethane (1/60 → 1/20)) to obtain compound **44** as a brown solid (1.74 g).

LCMS (method 1): R*t* = 2.04 min, m/z ($ES^+$) 593.1 $[1/2(M+2H)]^+$.

Step 2: Preparation of compound **45**

Compound **44** (400 mg, 0.338 mmol) was dissolved in methanol (80 mL). The resulting solution was cooled in an ice-water bath, and then an aqueous solution of lithium hydroxide (1 N, 13.5 mL, 13.5 mmol) was added thereto. The

reaction solution was reacted at 0°C for 2 hours, and then glacial acetic acid (1 g) was added to quench the reaction. The mixed solution was concentrated under reduced pressure to one-third of its volume and then purified by prep-RP-HPLC (method 4: 20%-90% B in 8 min → 95% B in 4 min) to obtain compound **45** as a yellow solid (80 mg).

LCMS (method 1): Rt = 1.55 min, m/z ($ES^+$) 411.8 $[1/2(M+2H)]^+$.

Step 3: Preparation of compound **LD-05**

Compound **45** (40 mg, 0.0487 mmol) was dissolved in anhydrous N,N-dimethylformamide solution (2 mL), and then compound **35** (18 mg, 0.0585 mmol) was added thereto. The reaction solution was cooled to 0°C, and then diisopropylethylamine (25 mg, 0.195 mmol) was added. The reaction solution was stirred at room temperature for 4 hours. Glacial acetic acid (25 mg) was added to to quench the reaction, and the resulting mixture was purified by prep-RP-HPLC (method 4: 30%-90% B in 8 min → 95% B in 4 min) to obtain compound **LD-05** as a yellow solid (13.7 mg).

LCMS (method 1): Rt = 1.81 min, m/z ($ES^+$) 1015.4 $(M+H)^+$.

Example 10: Synthesis of linker-drug **LD-06**

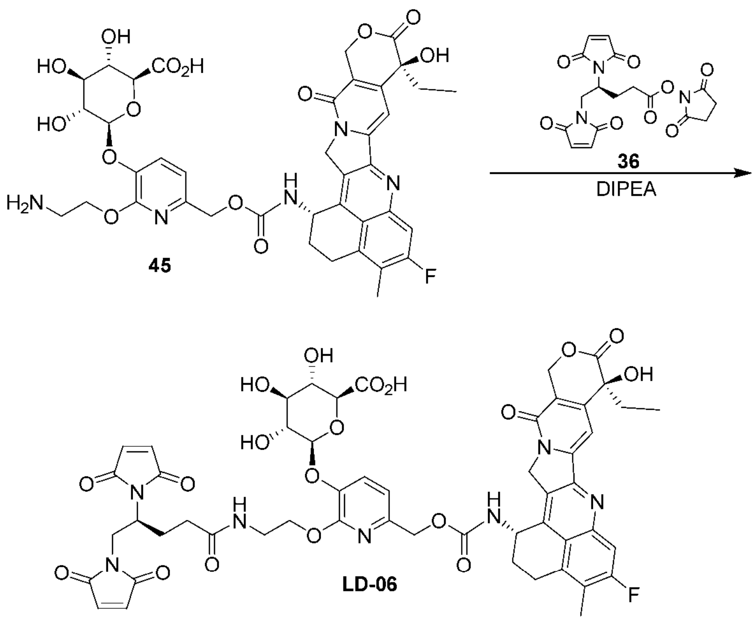


45

LD-06

Compound 45 (40 mg, 0.0487 mmol) was dissolved in anhydrous N,N-dimethylformamide (2 mL), and then compound **36** (18 mg, 0.0585 mmol) was added. The solution was cooled to 0°C, and diisopropylethylamine (25 mg, 0.195 mmol) was added thereto. The reaction solution was stirred at room temperature for 4 hours. Glacial acetic acid (25 mg) was added to quench the reaction, and the resulting solution was purified by prep-RP-HPLC (method 4: 30%-90% B in 8 min → 95% B in 4 min) to obtain compound **LD-06** as a yellow solid (9.4 mg).

LCMS (method 1): Rt = 1.81 min, m/z ($ES^+$) 1096.4 $(M+H)^+$.

Example 11: Synthesis of linker-drug **LD-07**

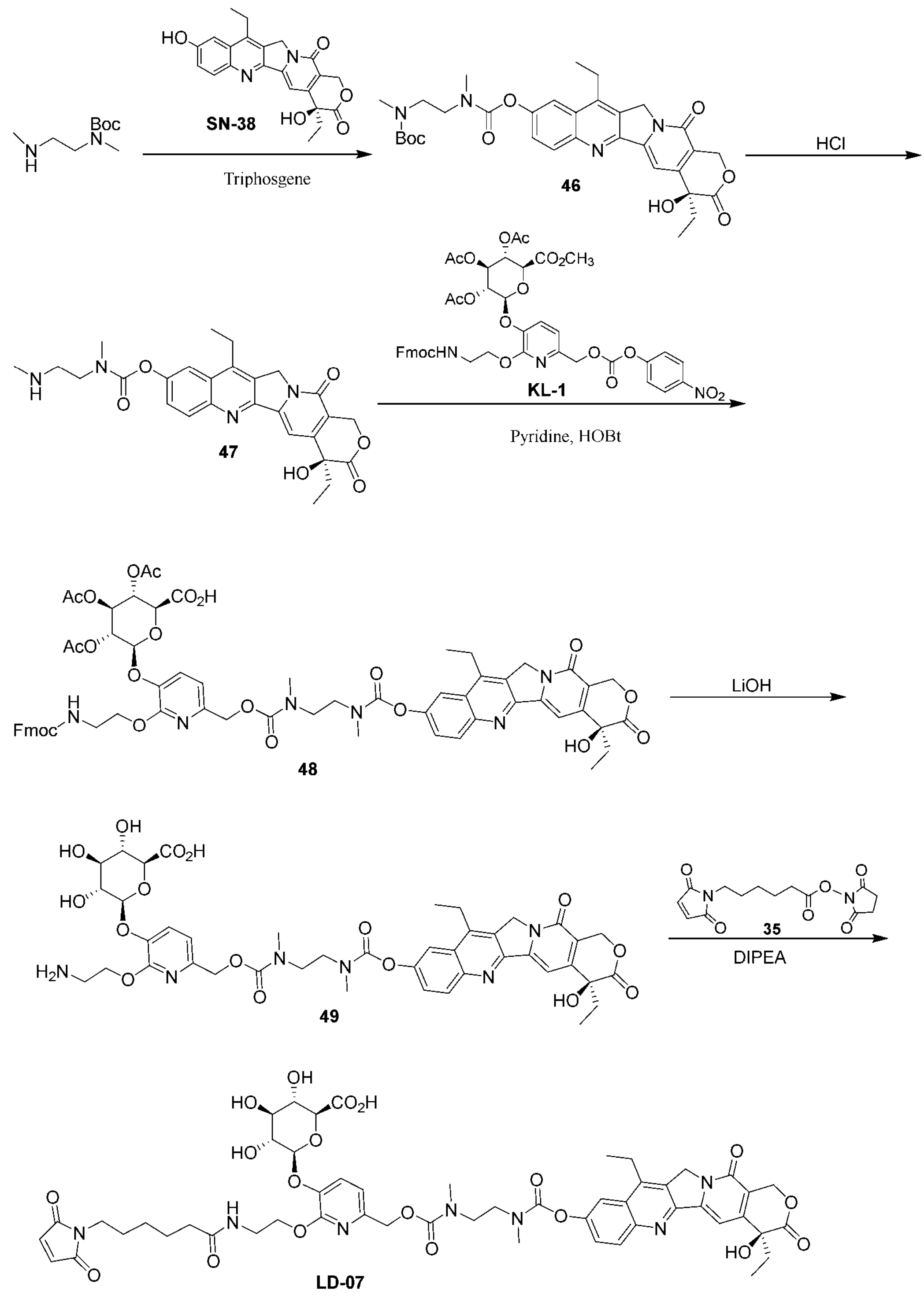


Step 1: Preparation of compound **46**

*Tert*-butyl methyl(2-(methylamino)ethyl)carbamate (550 mg, 2.93 mmol) and triethylamine (325 mg, 3.22 mmol) were added to toluene (15 mL) successively, and then triphosgene (348 mg, 1.17 mmol) was added under ice-bath. The reaction solution was reacted under ice-bath for 2 hours, then warmed to room temperature and continued to react for 16 hours. The reaction solution was filtered, and the obtained filtrate was concentrated to remove the solvent. Then, pyridine (15 mL) and **SN-38** (918 mg, 2.34 mmol, Titan Scientific) were added thereto successively, and the reaction solution was continued to stir at room temperature for 16 hours. The reaction solution was concentrated under reduced pressure to remove the solvent, and the residues were purified by silica gel column chromatography (eluent: methanol/dichloromethane (1/40 → 1/20)) to obtain compound **46** as a yellow solid (670 mg).

LCMS (method 3): Rt = 1.89 min, m/z ($ES^+$) 606.1 $(M+H)^+$.

Step 2: Preparation of compound **47**

Compound **46** (310 mg, 0.51 mmol) was dissolved in dichloromethane (4 mL), and then a solution of hydrochloric acid in dioxane (4 M, 4 mL, 16 mmol) was added. The reaction solution was stirred at room temperature for 4 hours. The reaction solution was concentrated to remove the solvent to obtain compound **47** as a yellow solid (277 mg).
LCMS (method 1): Rt = 1.41 min, m/z ($ES^+$) 506.2 $(M+H)^+$.

Step 3: Preparation of compound **48**

Compound **47** (174 mg, 0.32 mmol), compound **KL-1** (284 mg, 0.32 mmol), HOBt (43 mg, 0.32 mmol), and pyridine (0.5 mL) were added to anhydrous N,N-dimethylformamide (5 mL) successively, and the reaction solution was stirred at room temperature for 16 hours. After completion of the reaction, water (30 mL) was added, and the mixed solution was extracted with ethyl acetate (50 mL × 4). The combined organic phases were washed with saturated brine (60 mL × 2), dried over anhydrous sodium sulfate, filtered, and concentrated under reduced pressure to remove the solvent. The residues were purified by silica gel column chromatography (eluent: methanol/dichloromethane (1/40 - 1/20)) to obtain compound **48** as a yellow solid (324 mg).
LCMS (method 3): Rt = 1.98 min, *m/z* ($ES^+$) 628.3 $[1/2(M+2H)]^+$.

Step 4: Preparation of compound **49**

Compound **48** (324 mg, 0.26 mmol) was dissolved in a mixed solution of methanol (2 mL) and water (2 mL), and then lithium hydroxide monohydrate (109 mg, 2.6 mmol) was added thereto. The reaction solution was stirred at room temperature for 2 hours. Glacial acetic acid (160 mg) was added to quench the reaction, and the resulting solution was purified by prep-RP-HPLC (method 4: 20%-80% B in 8 min → 95% B in 4 min) to obtain compound **49** as a yellow solid (52 mg).
LCMS (method 1): Rt = 1.36 min, m/z ($ES^+$) 893.3 $(M+H)^+$.

Step 5: Preparation of compound **LD-07**

Compound **49** (30 mg, 0.034 mmol), compound **35** (9 mg, 0.034 mmol), and diisopropylethylamine (17 mg, 0.136 mmol) were added to anhydrous N,N-dimethylformamide (1 mL) successively, and the reaction solution was stirred at room temperature for 3 hours. Glacial acetic acid (20 mg) was added to quench the reaction, and the resulting solution was purified by prep-RP-HPLC (method 4: 30%-90% in 8 min → 95% B in 4 min) to obtain compound **LD-07** as a yellow solid (16 mg).
LCMS (method 1): Rt = 1.56 min, m/z ($ES^+$) 1087.4 $(M+H)^+$.

Example 12: Synthesis of linker-drug **LD-08**

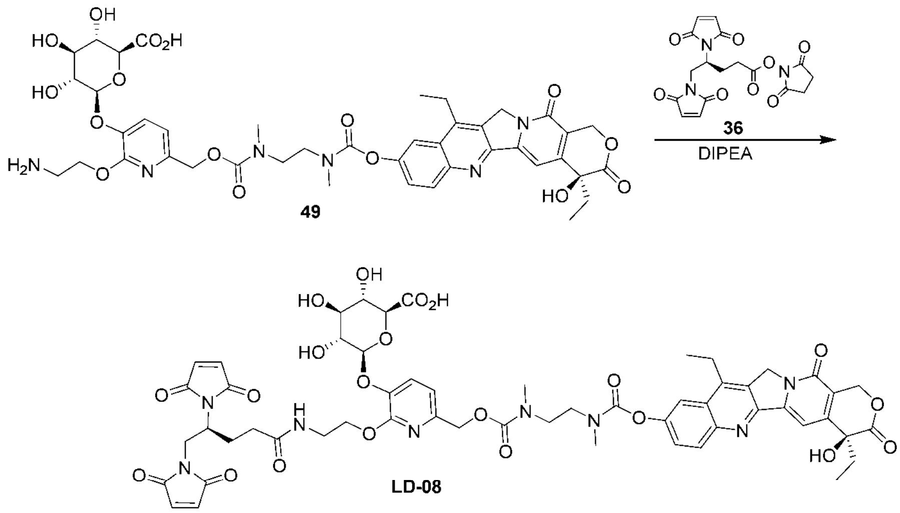


49

LD-08

Compound **49** (21.8 mg, 0.024 mmol), compound **36** (8.5 mg, 0.024 mmol), and diisopropylethylamine (12.6 mg, 0.098 mmol) were added to anhydrous N,N-dimethylformamide (1 mL) successively, and the reaction solution was stirred at room temperature for 3 hours. Glacial acetic acid (20 mg) was added to quench the reaction, and the resulting solution was purified by prep-RP-HPLC (method 4: 35%-90% B in 8 min → 95% B in 4 min) to obtain compound **LD-08** as a yellow solid (6.7 mg).

LCMS (method 1): Rt = 1.74 min, *m/z* ($ES^+$) 584.3 $[1/2(M+2H)]^+$.

Example 13: Synthesis of linker-drug **LD-09**

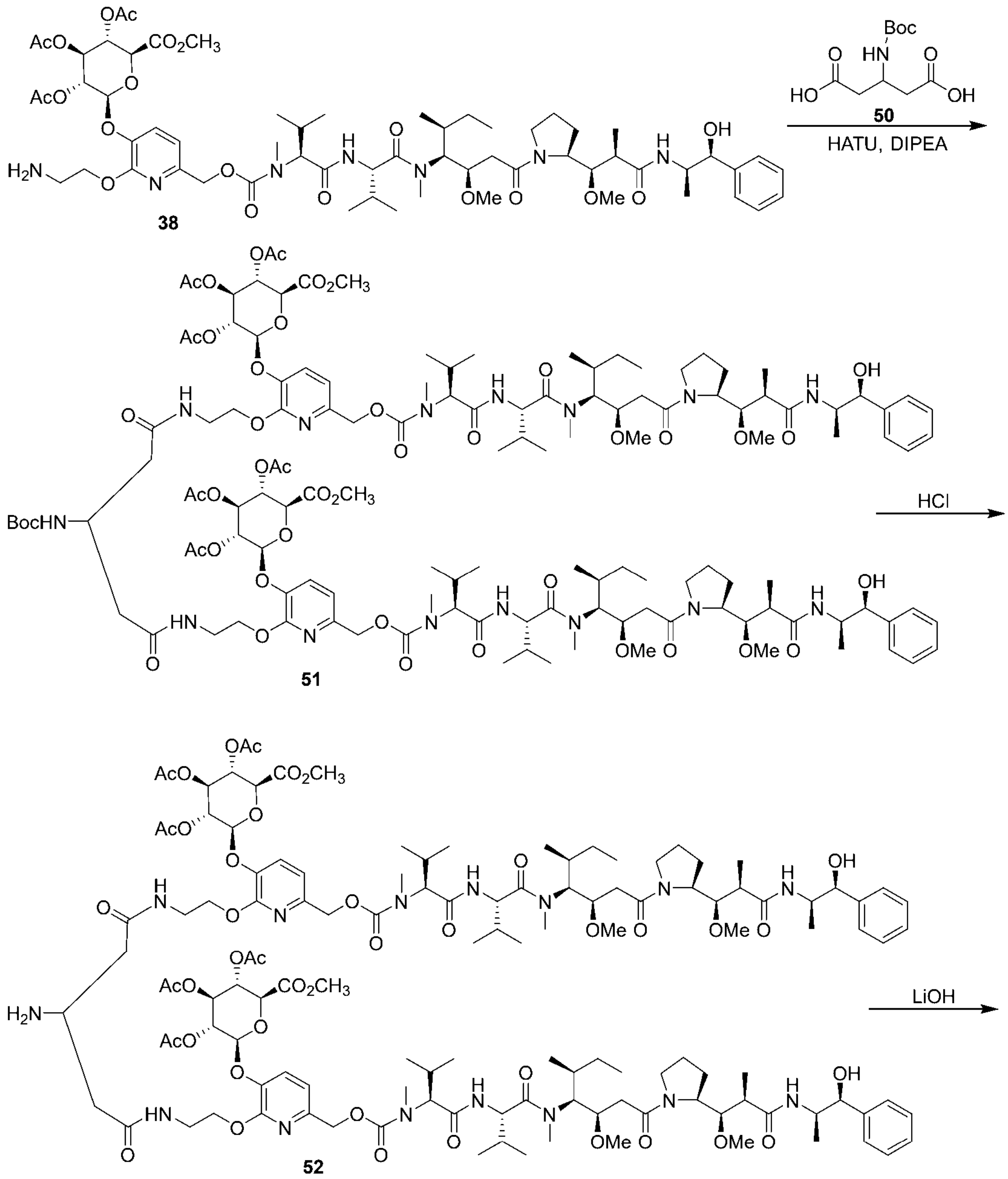


38

51

52

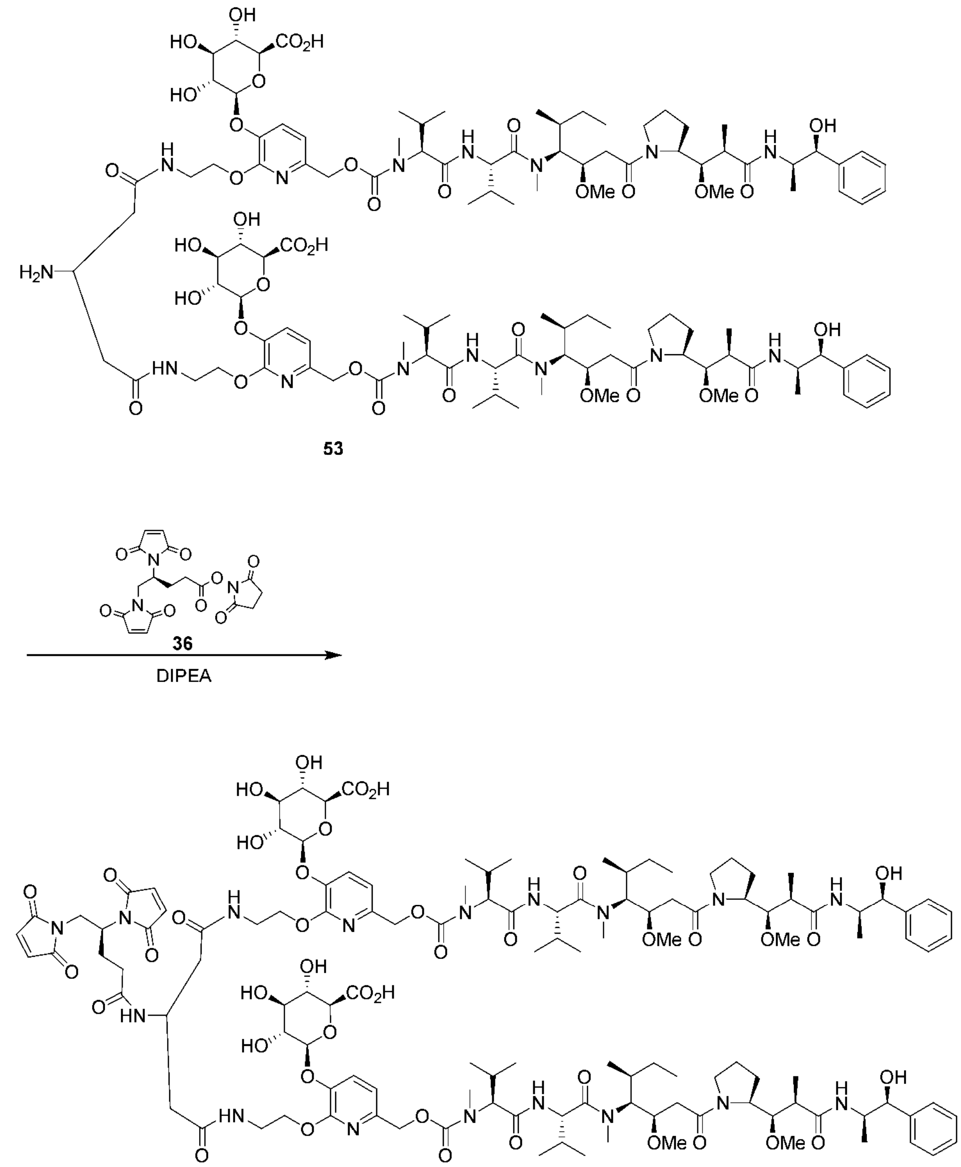


LD-09

Step 1: Preparation of compound **51**

Compound **50** (6.4 mg, 0.026 mmol, Titan Scientific), HATU (48.6 mg, 0.128 mmol), compound **38** (80 mg, 0.064 mmol), and diisopropylethylamine (33 mg, 0.256 mmol) were added to DMF (5 mL) successively, and the reaction solution was stirred at room temperature for 5 hours. Glacial acetic acid (20 mg) was added to quench the reaction, which was then purified by prep-RP-HPLC (method 4: 30%-95% B in 8 min → 95% B in 4 min) to obtain compound **51** as a white solid (30 mg).

LCMS (method 2): Rt = 1.92 min, m/z ($ES^+$) 900.9 $[1/3(M+3H)]^+$.

Step 2: Preparation of compound **52**

Compound **51** (30 mg, 0.011 mmol) was dissolved in ethyl acetate (0.8 mL), and then a 4 N solution of hydrochloric acid in dioxane (0.2 mL) was added thereto. The reaction solution was stirred at room temperature for 5 hours. The solvent was removed by concentration under reduced pressure to obtain compound **52** as a white solid (20 mg).

LCMS (method 2): R*t* = 2.26 min, m/z ($ES^+$) 867.5 $[1/3 (M+3H)]^+$.

Step 3: Preparation of compound **53**

Compound **52** (20 mg, 0.0077 mmol) was dissolved in methanol (1 mL). 1 M lithium hydroxide aqueous solution

(77 μL, 0.077 mmol) was added thereto under ice-bath, and the reaction solution was stirred at room temperature for 3 hours. Glacial acetic acid (2 mg) was added to quench the reaction, which was then purified by prep-RP-HPLC (method 4: 20%-95% B in 8 min → 95% B in 4 min) to obtain compound **53** as a white solid (4 mg).

LCMS (method 2): R*t* = 2.04 min, *m*/*z* ($ES^+$) 744.0 $[1/3(M+3H)]^+$.

Step 4: Preparation of compound **LD-09**

Compound **53** (4 mg, 0.0017 mmol) was dissolved in DMF (0.5 mL). Compound **36** (0.8 mg, 0.002 mmol) was added thereto, and then diisopropylethylamine (0.88 mg, 0.0068 mmol) was added under ice-bath. The reaction solution was stirred at room temperature for 3 hours. Glacial acetic acid (2 mg) was added to quench the reaction, which was then purified by prep-RP-HPLC (method 4: 35%-95% B in 8 min → 95% B in 4 min) to obtain compound **LD-09** as a white solid (0.8 mg).

LCMS (method 2): Rt = 2.17 min, *m*/*z* ($ES^+$) 865.2 $[1/3(M+3H)]^+$.

Example 14: Synthesis of linker-drug **LD-10**

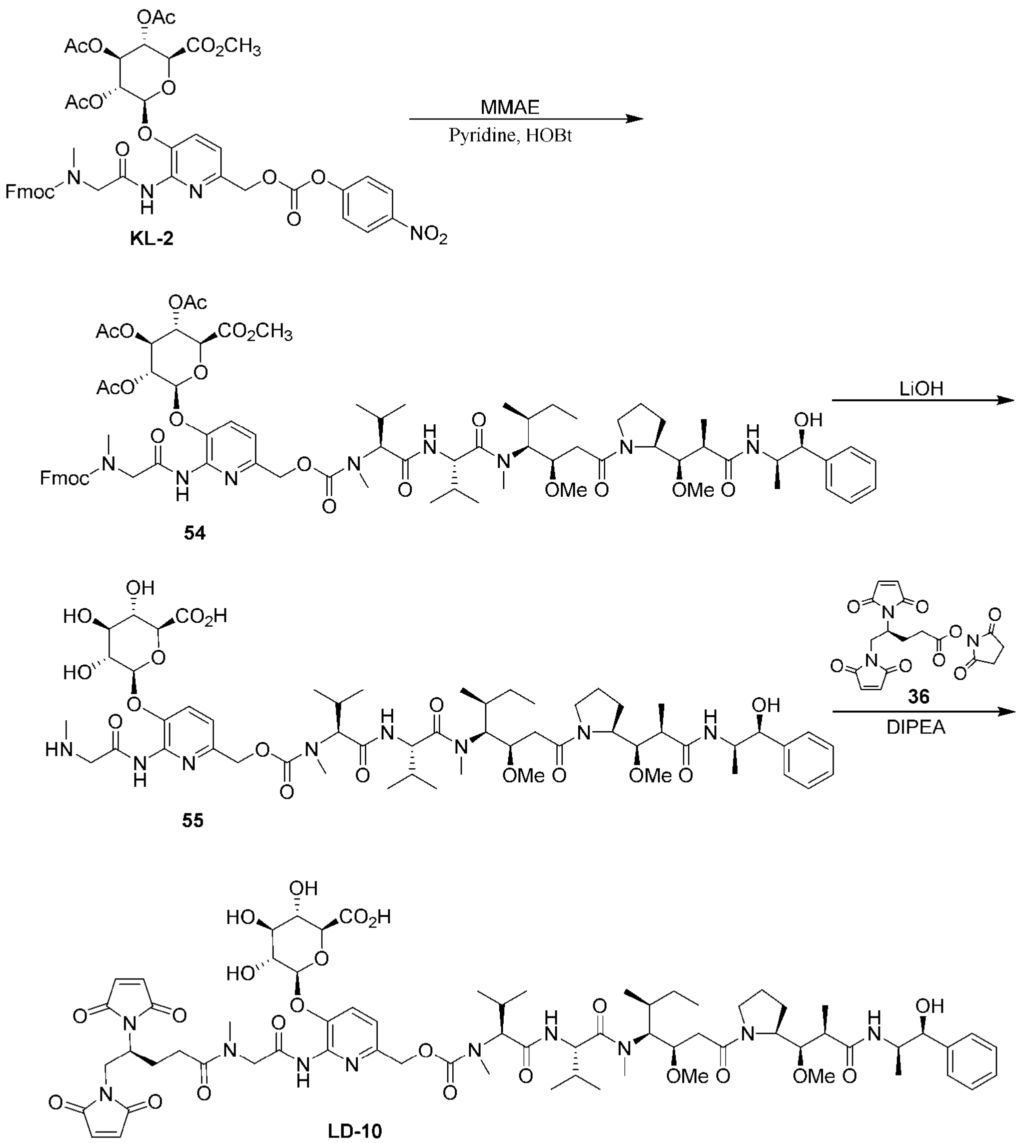


**KL-2**

**54**

**55**

**LD-10**

Step 1: Preparation of compound **54**

Compound **KL-2** (30 mg, 0.033 mmol) was dissolved in dry N,N-dimethylformamide (0.8 mL), and then 1-hydroxybenzotriazole (2.3 mg, 0.017 mmol), pyridine (0.2 mL), and **MMAE** (23.7 mg, 0.033 mmol) were added thereto successively. The reaction solution was stirred at room temperature overnight and then purified by prep-RP-HPLC (method 4: 65%-95% B in 8 min → 95% B in 4 min) to obtain compound **54** as a white solid (20 mg).

LCMS (method 1): R*t* = 2.14 min, *m*/*z* ($ES^+$) 747.3 $[1/2(M+2H)]^+$.

Step 2: Preparation of compound **55**

Compound **54** (20 mg, 0.0134 mmol) was dissolved in methanol (1 mL), and then a 1 N lithium hydroxide aqueous solution (0.135 mL, 0.134 mmol) was added thereto. The reaction solution was stirred at room temperature for 5 hours. Glacial acetic acid (0.1 mL) was added to quench the reaction, which was then purified by prep-RP-HPLC (method 4: 50%-95% B in 8 min → 95% B in 4 min) to obtain compound **55** as a white solid (10 mg).

LCMS (method 1): R*t* = 1.47 min, *m*/*z* ($ES^+$) 566.4 $[1/2 (M+2H)]^+$.

Step 3: Preparation of compound **LD-10**

Compound **55** (13 mg, 0.0115 mmol) was dissolved in anhydrous N,N-dimethylformamide (1 mL), and then compound **32** (5.4 mg, 0.0138 mmol) and diisopropylethylamine (5.9 mg, 0.046 mmol) were added thereto successively. The reaction solution was stirred at room temperature for 5 hours. Glacial acetic acid (6 mg) was added to quench the reaction, which was then purified by prep-RP-HPLC (method 4: 55%-95% B in 8 min → 95% B in 4 min) to obtain compound **LD-10** as a white solid (4 mg).

LCMS (method 1): R*t* = 1.73 min, *m*/*z* ($ES^+$) 703.3 $[1/2(M+2H)]^+$.

Example 15: Synthesis of linker-drug **LD-11**

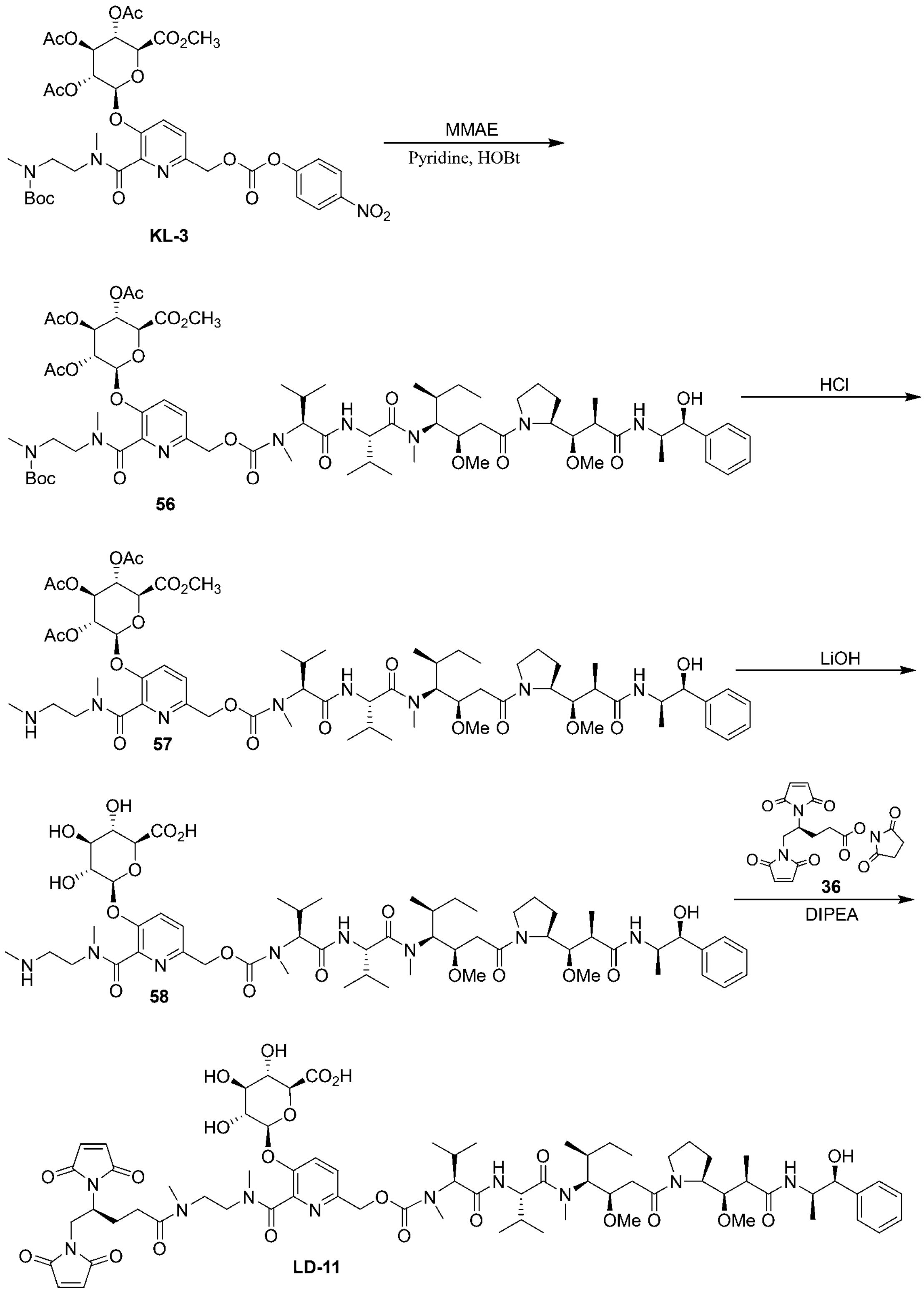


KL-3

56

57

58

LD-11

Step 1: Preparation of compound **56**

Compound **KL-3** (100 mg, 0.12 mmol), **MMAE** (70 mg, 0.10 mmol), 1-hydroxybenzotriazole (7 mg, 0.05 mmol), and pyridine (0.05 mL) were added to anhydrous N,N'-dimethylformamide (1 mL) successively, and the reaction solution was stirred at room temperature for 16 hours. After completion of the reaction, the reaction solution was purified by prep-RP-HPLC (method 5: 30%-95% B in 8 min → 95% B in 4 min) to obtain compound **52** as a white solid (25 mg).

LCMS (method 1): R*t* = 2.04 min, *m*/*z* ($ES^+$) 700.4 $[1/2(M+2H)]^+$.

Step 2: Preparation of compound **57**

Compound **56** (25 mg, 0.0179 mmol) was dissolved in ethyl acetate (2 mL), and then a 4 M solution of hydrochloric acid in dioxane (2 mL, 8 mmol) was added thereto. The reaction solution was stirred at room temperature for 2 hours. After the reaction, the reaction solution was concentrated under reduced pressure to remove the solvent, yielding compound **57** as a yellow solid (20 mg).
LCMS (method 1): R*t* = 1.53 min, m/z ($ES^+$) 650.5 $[1/2(M+2H)]^+$.

Step 3: Preparation of compound **58**

Compound **57** (20 mg, 0.015 mmol) was dissolved in methanol (1 mL), and then a 1 M lithium hydroxide aqueous solution (0.15 mL, 0.15 mmol) was added thereto. The reaction solution was stirred at room temperature for 3 hours. Glacial acetic acid (0.1 mL) was added to quench the reaction, and the resulting solution was purified by prep-RP-HPLC (method 4: 50%-95% B in 8 min → 95% B in 4 min) to obtain compound **58** as a white solid (14 mg).
LCMS (method 1): R*t* = 1.42 min, m/z ($ES^+$) 1160.8 $(M+H)^+$.

Step 4: Preparation of compound **LD-11**

Compound **58** (14 mg, 0.0121 mmol) was dissolved in N,N'-dimethylformamide (1 mL), and then compound **36** (5.6 mg, 0.0145 mmol) and diisopropylethylamine (9.4 mg, 0.0726 mmol) were added thereto successively. The reaction solution was stirred at room temperature for 16 hours. Glacial acetic acid (10 mg) was added to quench the reaction, and the resulting solution was purified by prep-RP-HPLC (method 4: 30%-95% B in 8 min → 95% B in 4 min) to obtain compound **LD-11** as a white solid (2.7 mg).
LCMS (method 2): R*t* = 1.78 min, *m*/*z* ($ES^+$) 717.4 $[1/2(M+2H)]^+$.

Example 16: Synthesis of linker-drug **LD-12**

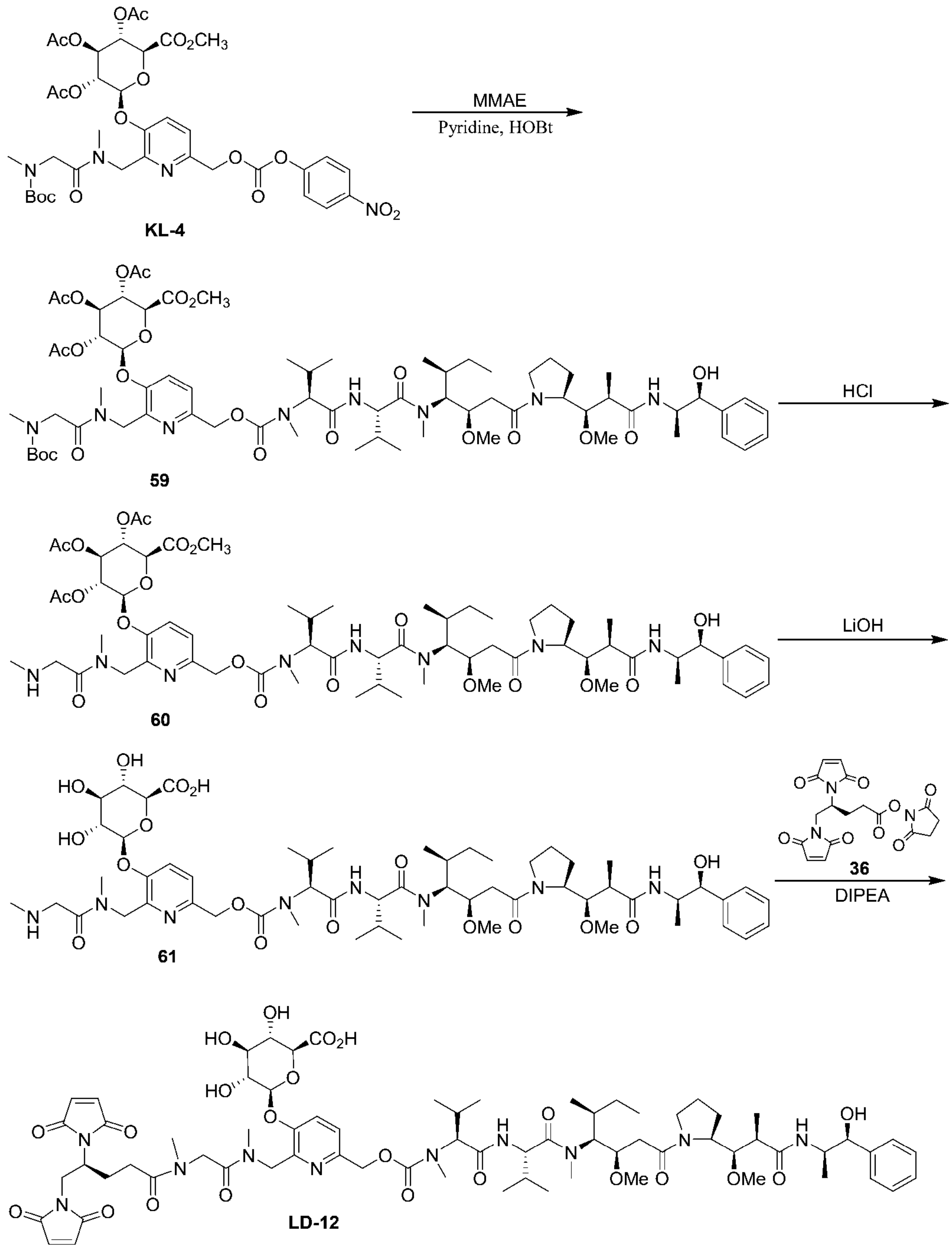


KL-4

59

60

61

LD-12

Step 1: Preparation of compound **59**

Compound **KL-4** (41 mg, 0.05 mmol), **MMAE** (43 mg, 0.06 mmol), 1-hydroxybenzotriazole (3 mg, 0.02 mmol), and pyridine (0.03 mL) were added to DMF (1 mL) successively, and the reaction solution was stirred at room temperature for 16 hours. After the reaction, the reaction solution was purified by prep-RP-HPLC (method 5: 30%-95% B in 8 min → 95% B in 4 min) to obtain compound **59** as a white solid (50 mg).

LCMS (method 3): R*t* = 2.14 min, *m*/*z* ($ES^+$) 700.5 $[1/2 (M+2H)]^+$.

Step 2: Preparation of compound **60**

Compound **59** (50 mg, 0.04 mmol) was dissolved in ethyl acetate (1 mL). A 4 M solution of hydrochloric acid in dioxane (0.3 mL) was added thereto under ice-bath, and the reaction solution was stirred at room temperature for 2 hours. After completion of the reaction, the solvent was removed by concentration under reduced pressure, and the residues

were purified by prep-RP-HPLC (method 4: 30%-95% B in 8 min → 95% B in 4 min) to obtain compound **60** as a white solid (30 mg).

LCMS (method 3): R*t* = 1.53 min, *m/z* (ES+) 650.6 [1/2 (M+2H)]+.

Step 3: Preparation of compound **61**

Compound **60** (30 mg, 0.02 mmol) was dissolved in methanol (0.5 mL). A 1 M lithium hydroxide aqueous solution (0.5 mL) was added thereto under ice-bath, and the reaction solution was stirred at room temperature for 2 hours. Glacial acetic acid (30 mg) was added to quench the reaction, which was then purified by prep-RP-HPLC (method 4: 30%-95% B in 8 min → 95% B in 4 min) to obtain compound **61** as a white solid (15 mg).

LCMS (method 2): R*t* = 1.43 min, *m/z* (ES+) 1160.7 (M+H)+.

Step 4: Preparation of compound **LD-12**

Compound **61** (15 mg, 0.01 mmol) was dissolved in N,N-dimethylformamide (0.9 mL). Compound **36** (6.2 mg, 0.02 mmol) and diisopropylethylamine (6.7 mg, 0.05 mmol) were added to the reaction solution successively under ice-bath, which was stirred at room temperature for 16 hours. Acetic acid was added to quench the reaction, which was then purified by prep-RP-HPLC (method 4: 30%-95% B in 8 min → 95% B in 4 min) to obtain compound **LD-12** as a white solid (2 mg).

LCMS (method 2): R*t* = 1.68 min, *m/z* (ES+) 717.5 [1/2(M+2H)]+.

Example 17: Linker-drugs **LD-13, LD-14,** and **LD-15**

Linker-drugs **LD-13, LD-14,** and **LD-15** were synthesized according to methods reported in the literature **(LD-13:** US7498298; **LD-14:** WO2014114207; **LD-15:** WO2019033773).

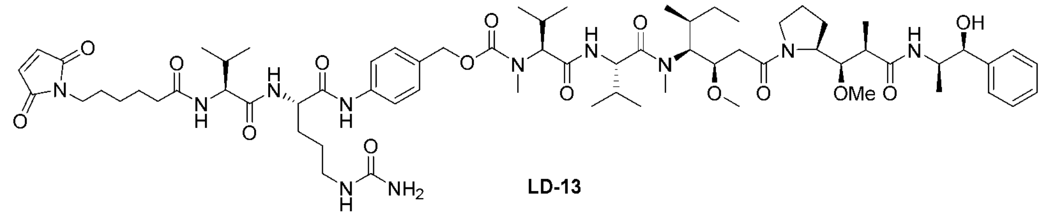

**LD-13**

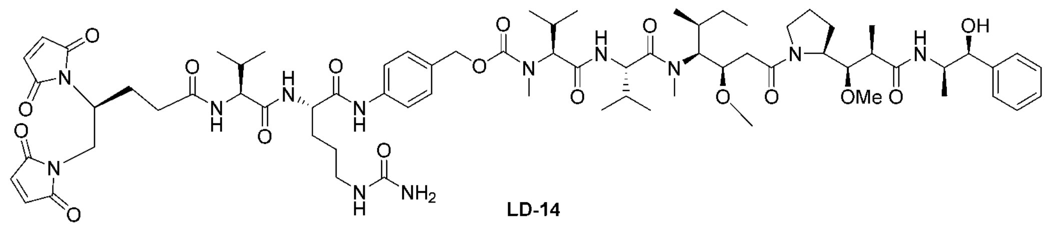

**LD-14**

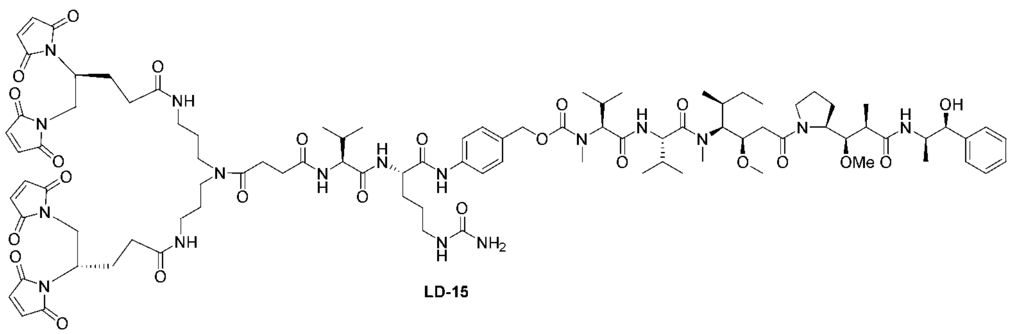

**LD-15**

Example 18: Synthesis of linker-drug **LD-16**

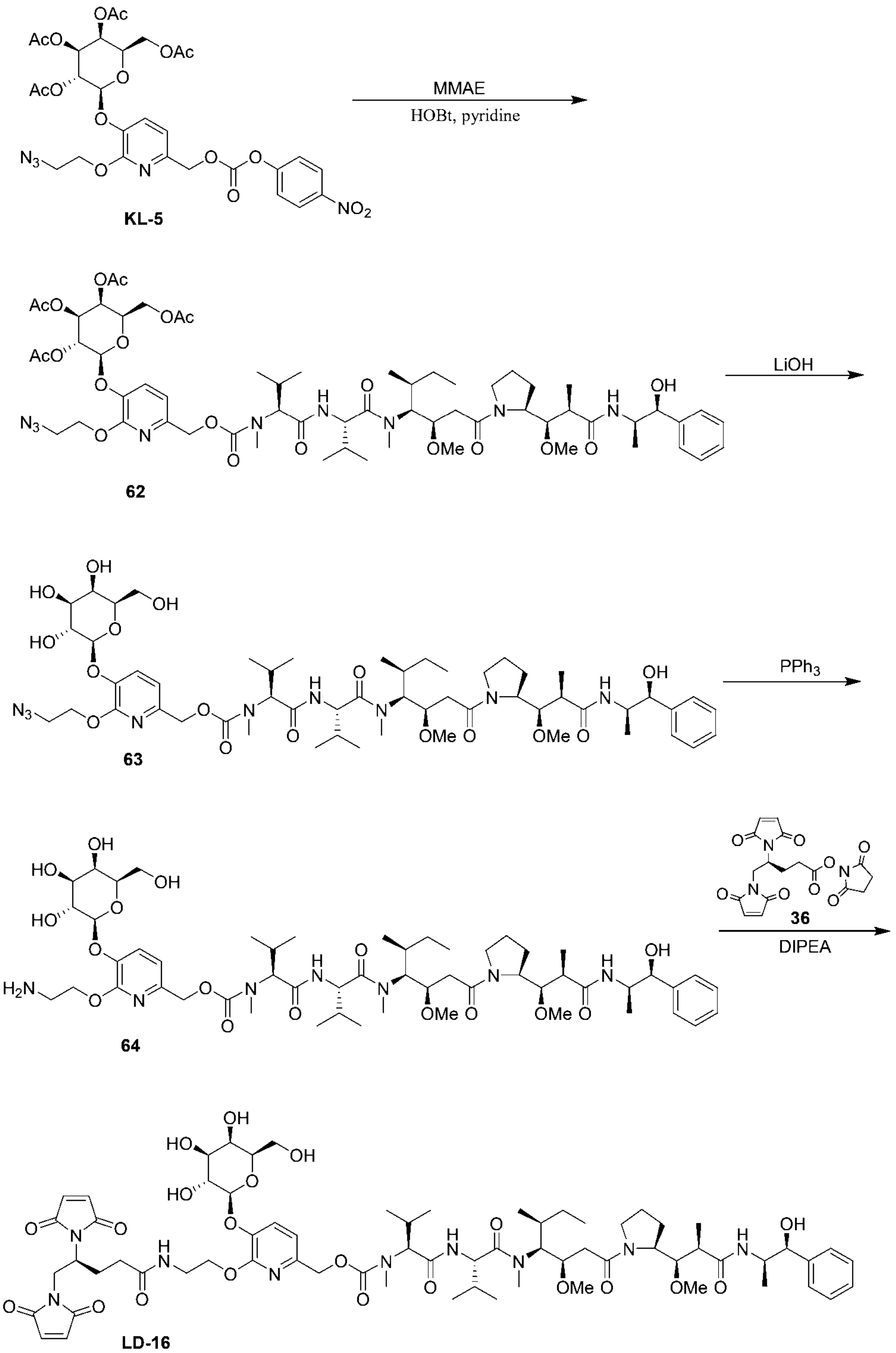


KL-5

62

63

64

LD-16

Step 1: Synthesis of compound **62**

Compound **KL-5** (197 mg, 0.28 mmol) was dissolved in anhydrous N,N-dimethylformamide (3 mL), and then pyridine (0.5 mL), 1-hydroxybenzotriazole (19 mg, 0.14 mmol), and **MMAE** (201 mg, 0.28 mmol) were added thereto successively. The reaction solution was stirred at room temperature for 17 hours. Acetic acid was added to the reaction solution to quench the reaction, followed by the addition of water and extraction with ethyl acetate (30 mL × 2). The combined organic phases were washed with saturated brine, dried over anhydrous sodium sulfate, and concentrated under reduced pressure to remove the solvent. The residues were purified by silica gel column chromatography (eluent: petroleum ether/ethyl acetate (1/0 → 5/1 → 3/1), then dichloromethane/methanol (30/1)) to obtain compound **62** as a colorless oil (308 mg).

LCMS (method 3): rt = 2.15 min; *m/z* ($ES^+$) 643.0 [1/2 (M+2H)]$^+$.

Step 2: Synthesis of compound **63**

Compound **62** (308 mg, 0.24 mmol) was dissolved in methanol (4.8 mL), and then a 1 M lithium hydroxide aqueous solution (2.4 mL, 2.4 mmol) was added thereto. The reaction solution was stirred at room temperature for 3 hours. Glacial acetic acid (144 mg) was added to the reaction solution to quench the reaction, followed by the addition of water and extraction with dichloromethane (30 mL × 2). The combined organic phases were washed with saturated brine, dried over anhydrous sodium sulfate, and concentrated under reduced pressure to remove the solvent. The residues were purified by silica gel column chromatography (eluent: dichloromethane/methanol (40/1 → 15/1)) to obtain compound **63** as a colorless gum (240 mg).

LCMS (method 3): rt = 1.91 min, *m/z* ($ES^+$) 1116.5 (M+H)$^+$.

Step 3: Synthesis of compound **64**

Compound **63** (240 mg, 215 μmol) was dissolved in tetrahydrofuran (4 mL), and triphenylphosphine (67 mg, 258 μmol) and water (23 mg) were added thereto under stirring. The reaction solution was stirred at room temperature overnight. The reaction solution was concentrated under reduced pressure to remove the solvent, and the residues were purified by prep-RP-HPLC (method 4: 15%-95% B in 8 min → 95% B in 4 min) to obtain compound **64** as a white solid (150 mg).

LCMS (method 1): rt = 1.45 min, *m/z* ($ES^+$) 1091.6 (M+H)$^+$.

Step 4: Synthesis of compound **LD-16**

Compound **64** (21 mg, 19 μmol) was dissolved in N,N-dimethylformamide (1 mL), and then compound **36** (8.5 mg, 22 μmol) and diisopropylethylamine (10 mg, 76 μmol) were added thereto successively. The reaction solution was stirred at room temperature for 4 hours. Glacial acetic acid (20 mg) was added to the reaction solution to quench the reaction, which was then purified by prep-RP-HPLC (method 4: 15%-95% B in 8 min → 95% B in 4 min) to obtain compound **LD-16** as a white solid (13.6 mg).

LCMS (method 3): rt = 1.84 min, *m/z* ($ES^+$) 683.0 [1/2(M+2H)]$^{+2}$.

Example 19: Synthesis of linker-drug **LD-17**

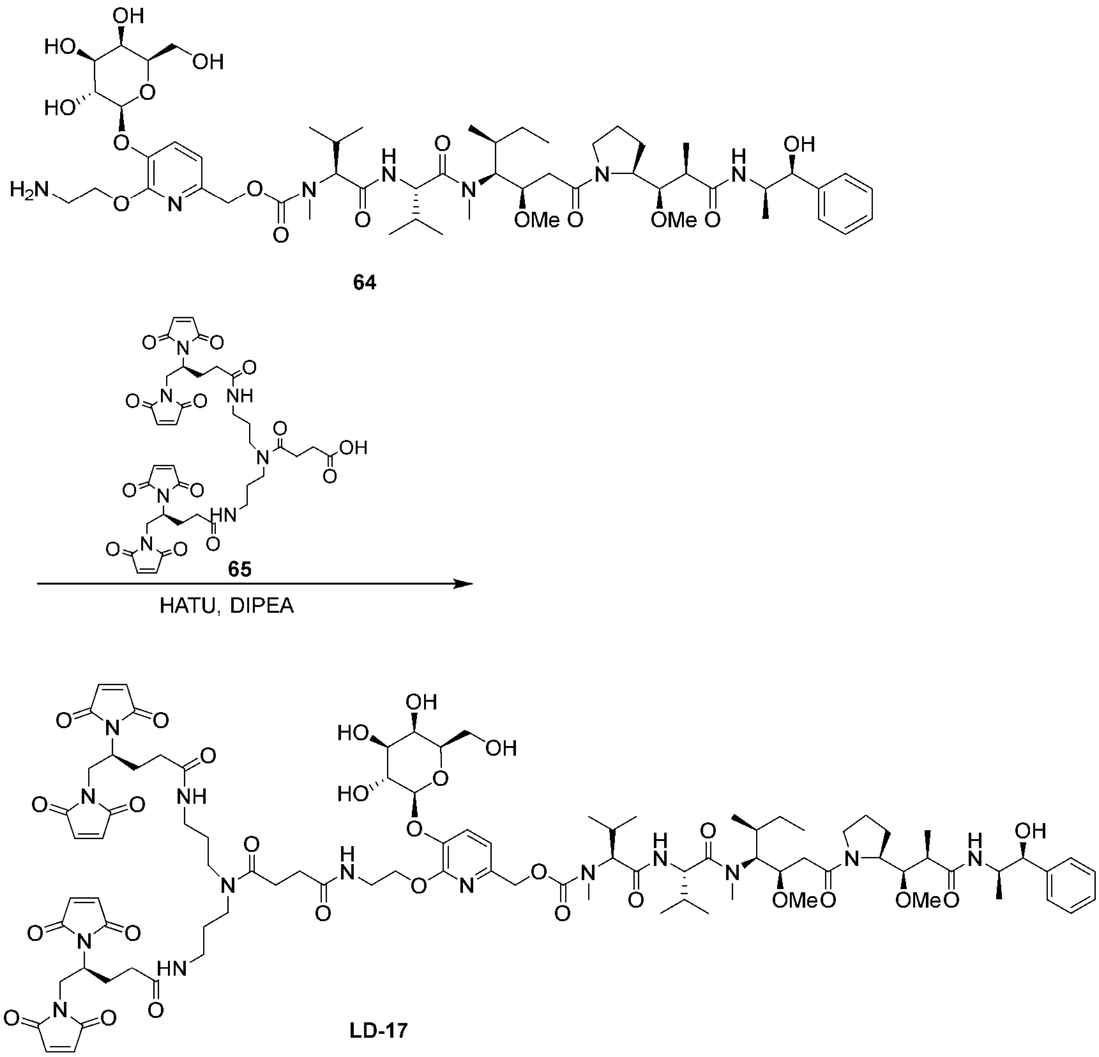


64

65

LD-17

Compound **64** (27.2 mg, 25 μmol) was dissolved in N,N-dimethylformamide (1 mL), and then compound **65** (25.3 mg, 32.5 μmol), HATU (19 mg, 50 μmol), and diisopropylethylamine (12.9 mg, 100 μmol) were added thereto successively. The reaction solution was stirred at room temperature for 2 hours. Glacial acetic acid (26 mg) was added to the reaction solution to quench the reaction, and the mixture was purified by prep-RP-HPLC (method 4: 20%-95% B in 8 min → 95% B in 4 min) to obtain compound **LD-17** as a white solid (27 mg).

LCMS (method 2): rt = 1.09 min, m/z ($ES^+$) 926.8 [1/2 $(M+2H)]^+$.

Example 20: Preparation of antibody-drug conjugates

Tris(2-carboxyethyl)phosphine hydrochloride (2-10 eq, concentration of the stock solution is 10 mM) was added to an antibody solution (2-10 mg/mL, 67 mM PBS buffer, pH 7.0-7.2). The reaction solution was incubated in a constant temperature shaker at 37°C for 2 hours. The reaction solution was cooled to about 10°C, and subjected to ultrafiltration (*Merck* Millipore Amicon® Ultra, 50000 MWCO) or gel filtration to exchange the buffer (20 mM citric acid-trisodium citrate, 50 mM sodium chloride, 1 mM diethylenetriaminepentaacetic acid, pH 5.8-6.2). Dimethyl sulfoxide and the linker-drug (dimethyl sulfoxide stock solution, 3-15 equivalents) were added, ensuring the volume percentage of dimethyl sulfoxide in the reaction solution reached about 10%. The conjugation reaction was carried out at 10°C for 1.5 hours.

An excess of cysteine solution was added to the reaction solution to quench the unreacted linker-drug, and the quenching reaction was carried out at 10°C for 30 minutes. The reaction solution was first subjected to ultrafiltration (*Merck* Millipore Amicon® Ultra, 50000 MWCO) or gel filtration to remove cysteine-linker-drug adducts and excess cysteine, and then sterilized through a 0.22 μm pore size filter unit (*Merck* Millex-GV Filter) to obtain the antibody-drug conjugate, which was stored at 4°C.

Antibody-drug conjugates were prepared with Ab1 and Ab2 as antibodies and **LD-01 to LD-17** as linker-drugs according to the above preparation method (see Table 1).

Table 1 Prepared antibody-drug conjugates

| Linker-drug | Reaction parameter | | Antibody-drug conjugate (based on antibody Ab1) | Antibody-drug conjugate (based on antibody Ab2) |
|---|---|---|---|---|
| | TCEP equivalent | LD equivalent | | |
| **LD-01** | 10 | 15 | Ab1-LD-01 | NA |
| **LD-02** | 10 | 10 | Ab1-LD-02 | Ab2-LD-02 |
| **LD-04** | 10 | 3.5 | NA | Ab2-LD-04 |
| **LD-05** | 10 | 12 | NA | Ab2-LD-05 |
| **LD-06** | 10 | 8 | NA | Ab2-LD-06 |
| **LD-07** | 10 | 12 | Ab1-LD-07 | NA |
| **LD-08** | 10 | 6 | Ab1-LD-08 | NA |
| **LD-09** | 10 | 6 | Ab1-LD-09 | NA |
| **LD-10** | 10 | 10 | Ab1-LD-10 | NA |
| **LD-11** | 10 | 10 | Ab1-LD-11 | NA |
| **LD-12** | 10 | 10 | Ab1-LD-12 | NA |
| **LD-13** | 10 | 12 | NA | Ab2-LD-13 |
| **LD-14** | 10 | 8 | Ab1-LD-14 | Ab2-LD-14 |
| **LD-15** | 10 | 3.5 | Ab1-LD-15 | Ab2-LD-15 |
| **LD-16** | 10 | 8 | NA | Ab2-LD-16 |
| **LD-17** | 10 | 3.5 | NA | Ab2-LD-17 |
| NA: Not prepared. | | | | |

1) Average DAR value determination

The average DAR value was determined using one of the following two methods based on the actual situation.

1a) Hydrophobic interaction chromatography (HIC)

Calculated from the percentage of relative peak area of each peak in the HIC chromatogram and the number of drugs carried by the corresponding component (referring to Anal. Chem. 2013, 85, 1699-1704). The weighted peak ratio measures the contribution of a single DAR value of a component to the average DAR value, which is the relative peak area (%) multiplied by the number of drugs carried by the component. The average DAR value is obtained by summing the weighted peak ratios of all components and then dividing by 100, as shown in the following formula:

$$\text{DAR} = \Sigma(\text{relative peak area} \times \text{number of drugs carried by the component}) / 100$$

1b) Mass spectrometry (MS)

An appropriate amount of sample was taken and diluted to 1 mg/mL with 150 mM ammonium acetate solution. An ultra-performance liquid chromatograph Acquity UPLC (Waters) and a high-resolution Xevo G2-XS QTOF (Waters) mass spectrometer were used in the test, with 10 μL injection for detection. Mass spectral data in positive ion mode were collected. The collected non-denaturing mass spectral data were analyzed and processed using UNIFI software.

The measurement results of the average DAR values of the antibody-drug conjugates of the present invention are shown in Table 2 below.

Table 2 Average DAR values of antibody-drug conjugates

| Antibody-drug conjugate | Average DAR value | Antibody-drug conjugate | Average DAR value |
|---|---|---|---|
| Ab1-LD-01 | 8.0[a] | Ab2-LD-01 | NA |

(continued)

| Antibody-drug conjugate | Average DAR value | Antibody-drug conjugate | Average DAR value |
|---|---|---|---|
| Ab1-LD-02 | 4.1[a] | Ab2-LD-02 | 4.0[a] |
| Ab1-LD-04 | NA | Ab2-LD-04 | 2.0[a] |
| Ab1-LD-05 | NA | Ab2-LD-05 | 7.5[b] |
| Ab1-LD-06 | NA | Ab2-LD-06 | 4.0[b] |
| Ab1-LD-07 | 8.0[b] | Ab2-LD-07 | NA |
| Ab1-LD-08 | 4.0[b] | Ab2-LD-08 | NA |
| Ab1-LD-09 | 8.1[a] | Ab2-LD-09 | NA |
| Ab1-LD-10 | 4.1[a] | Ab2-LD-10 | NA |
| Ab1-LD-11 | 4.3[a] | Ab2-LD-11 | NA |
| Ab1-LD-12 | 4.4[a] | Ab2-LD-12 | NA |
| Ab1-LD-13 | NA | Ab2-LD-13 | 4.1[a] |
| Ab1-LD-14 | 4.2[a] | Ab2-LD-14 | 4.2[a] |
| Ab1-LD-15 | 2.2[a] | Ab2-LD-15 | 2.1[a] |
| Ab1-LD-16 | NA | Ab2-LD-16 | 4.0[a] |
| Ab1-LD-17 | NA | Ab2-LD-17 | 2.2[a] |
| NA: Not prepared.<br>a: HIC method<br>b: MS method | | | |

2) Hydrophobic interaction chromatography (HIC) analysis

Hydrophobic interaction chromatography was carried out on an Agilent 1100 chromatograph. The stationary phase was a TSKgel Butyl-NPR column (4.6 × 35 mm, 2.5 μm, Tosoh (Shanghai) Bioscience Co., Ltd.). The elution gradient was a linear gradient, exchanging from 100% Buffer A [50 mM potassium phosphate (pH 7.0) + 1.5 M ammonium sulfate] to 100% Buffer B [80% v/v 50 mM potassium phosphate (pH 7.0), 20% v/v isopropanol] within 25 minutes. The flow rate was 0.8 mL/min, the column temperature was set at 30°C, and the detection wavelength was set at 280 nm.

The HIC chromatograms of the ADCs of the present invention are shown in Figures 1A to 1K, and the retention times of their main peaks are shown in Table 3 and Table 4.

Table 3 Retention times of main peaks in HIC chromatograms of Ab1-based ADCs

| Antibody-drug conjugate | Retention time (min) | Average DAR value |
|---|---|---|
| Ab1-LD-02 | 14.3 | 4.1 |
| Ab1-LD-10 | 13.2 | 4.1 |
| Ab1-LD-11 | 14.4 | 4.3 |
| Ab1-LD-12 | 14.0 | 4.4 |
| Ab1-LD-14 | 16.8 | 4.2 |

From Table 3, it can be seen that compared with the ADC drug of Ab1-LD-14 which uses a vc-PABC cleavable linker, Ab1-LD-02, Ab1-LD-10, Ab1-LD-11, and Ab1-LD-12 all employ the glycosylpyridine-type linker of the present invention. The comparison of retention times of the main peaks in HIC chromatograms shows that the elution times of the latter are all significantly shorter than that of the former.

Table 4 Retention times of main peaks in HIC chromatograms of Ab2-based ADCs

| Antibody-drug conjugate | Retention time (min) | Average DAR value |
|---|---|---|
| Ab2-LD-02 | 12.0 | 4.0 |
| Ab2-LD-04 | 9.9 | 2.0 |
| Ab2-LD-14 | 13.5 | 4.2 |
| Ab2-LD-15 | 12.5 | 2.1 |
| Ab2-LD-16 | 11.2 | 4.0 |
| Ab2-LD-17 | 9.3 | 2.2 |

From Table 4, it can be seen that when the average DAR value is about 4, the retention times of the main peaks in HIC chromatograms for antibody-drug conjugates Ab2-LD-02 and Ab2-LD-16 having the glycosylpyridine-type linker are significantly shorter than that of the ADC drug Ab2-LD-14 with the vc-PABC linker. When the average DAR value is about 2, the retention times of the main peaks in HIC chromatograms for antibody-drug conjugates Ab2-LD-04 and Ab2-LD-17 with the glycosylpyridine-type linker are significantly shorter than that of the ADC drug Ab2-LD-15 with the vc-PABC linker.

The above results indicate that compared with the commonly used vc-PABC linker, the use of the glycosylpyridine-type linker can significantly increase the hydrophilicity of ADC products. An increase in hydrophilicity of ADC products can reduce the aggregate content of ADC products and improve the *in vivo* PK characteristics of ADC products.

3) Size exclusion chromatography (SEC) analysis

Size exclusion chromatography was carried out on an Agilent 1100 or Agilent 1260 chromatograph. The stationary phase was a TSKgel G3000SWXL column (7.8 × 300 mm, 5 μm, Tosoh (Shanghai) Bioscience Co., Ltd.). The mobile phase was 62 mM disodium hydrogen phosphate, 55 mM potassium dihydrogen phosphate, 100 mM sodium sulfate, pH 6.7 ± 0.1. The flow rate was 0.7 mL/min, the column temperature was set at 25°C, and the detection wavelength was set at 280 nm.

The monomer content of the ADC products in the Examples of the present invention tested by SEC was above 99%.

Test Example 1: Inhibition effect of the antibody-drug conjugates of the present invention on cell proliferation

Cell Proliferation Assay

The inhibition activity of the antibody or antibody-drug conjugate was determined by the following method. Mammalian cells expressing tumor-associated antigens or receptor proteins were seeded in 96-well plates at 8000 cells per well, and suspended in 200 μL of DMEM high glucose cell culture medium (GIBCO). The initial concentration of the ADC sample was 2 μg/mL, which was diluted in a 3-fold gradient with DMEM high glucose cell culture medium containing 2% FBS (GIBCO). The original culture medium was removed, and 200 μL of ADC sample was added to each well and incubation was continued for 72 hours. The original culture medium was removed, and 100 μL of CCK-8 chromogenic solution was added to each well and culture was continued for 30 minutes. The absorbance values at 450 nm/630 nm were read using a microplate reader. A curve was plotted, and the $IC_{50}$ was calculated.

The results of the inhibition effect of the antibody-drug conjugates of the present invention on cell proliferation are shown in Table 5 and Table 6.

Table 5 Results of inhibition effect of the antibody-drug conjugates of the present invention on cell proliferation

| Antibody-drug conjugate | $IC_{50}$ (ng/mL) | |
|---|---|---|
| | Ramos | BJAB |
| Ab1-LD-02 | 3.3 | 3.0 |
| Ab1-LD-04 | 4.2 | 5.2 |
| Ab1-LD-11 | 8.2 | 11.3 |
| Ab1-LD-15 | 7.4 | 12.1 |

The antibody-drug conjugates of the present invention all possess good cell proliferation inhibitory effects. As shown in Table 5, the cell proliferation inhibitory effect of Ab1-LD-04 is slightly better than that of Ab1-LD-15, while the structural difference between the two mainly lies in the cleavable linker part, the former employs the glycosylpyridine-type linker of the present invention, while the latter employs the vc-PABC type linker.

Table 6 Results of inhibition effect of the antibody-drug conjugates of the present invention on cell proliferation

| Antibody-drug conjugate | $IC_{50}$ (ng/mL) | | |
|---|---|---|---|
| | HH | L540 | SU-DHL-1 |
| Ab2-LD-02 | 28.3 | 166 | 23.9 |
| Ab2-LD-04 | 41.3 | 245 | 27.4 |
| Ab2-LD-05 | 101 | 68.7 | 39.6 |
| Ab2-LD-06 | 75.5 | 88.2 | 50.6 |
| Ab2-LD-14 | 28.8 | 119 | 37.6 |
| Ab2-LD-15 | 42.2 | 163 | 38.2 |
| Ab2-LD-16 | 22.6 | 136 | 19.6 |
| Ab2-LD-17 | 20.8 | 290 | 35.3 |

As shown in Table 6, when the average DAR values are similar, the antibody-drug conjugates with the glycosylpyridine-type linker have comparable cell proliferation inhibitory effects to the antibody-drug conjugates with the vc-PABC type linker.

Thus, it can be seen that the ADC drugs constructed based on the glycosylpyridine-type linker of the present invention can effectively release the cellularly active drug after entering the cell, playing a role of killing cells.

All literatures mentioned in the present invention are cited as references in the present application, as if each literature were individually cited as a reference. Furthermore, it should be understood that after reading the above content of the present invention, those skilled in the art can make various changes or modifications to the present invention, and these equivalent forms also fall within the scope defined by the appended claims of the present application.

## Claims

**1.** An antibody-drug conjugate of formula I,

$$L\!-\!\left(A\!-\!\left(G_g-W_w-D\right)_p\right)_q \qquad \text{I}$$

or a pharmaceutically acceptable salt or hydrate thereof,
wherein,
L is an antibody, an antibody fragment, or another type of ligand;
A is a linker fragment, connected to L and G simultaneously;
G is a glycosyl-substituted pyridine, as shown in formula II:

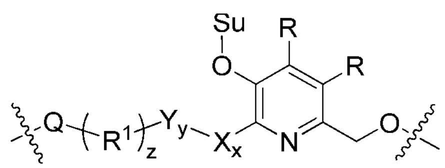

II

wherein,
Su is a sugar molecule;
each R is independently selected from the group consisting of hydrogen, CN, halogen, $NO_2$, and $C_1$-$C_4$ alkyl;
X and Y are each independently selected from the group consisting of $-CR^2R^3-$, $-C(=O)NR^4-$, $-NR^5C(=O)-$, and $-O-$;
Q is selected from the group consisting of $-NR^6-$ and $-O-$;

$R^1$ is selected from the group consisting of $C_1$-$C_6$ alkylene, and $C_1$-$C_6$ alkylene containing O in the skeleton;
$R^2$, $R^3$, $R^4$, $R^5$, and $R^6$ are each independently selected from the group consisting of hydrogen and $C_1$-$C_4$ alkyl;
x, y, and z are each independently selected from the group consisting of 0 and 1; g is selected from the group consisting of 1 and 2;
W is an optional self-immolative linker;
w is selected from the group consisting of 0 and 1;
D is a drug;
p is an integer selected from the group consisting of 1 to 4;
q is an integer selected from the group consisting of 1 to 8.

**2.** The antibody-drug conjugate of formula I according to claim 1, being an antibody-drug conjugate of formula III:

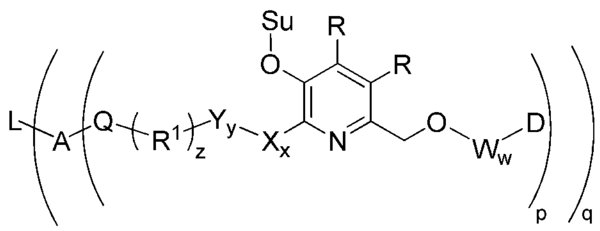

III

wherein, L, A, Q, $R^1$, R, X, Y, W, D, Su, x, y, z, and w are as defined in claim 1.

**3.** The antibody-drug conjugate of formula I according to claim 1 or 2, being an antibody-drug conjugate of formula IV or formula V:

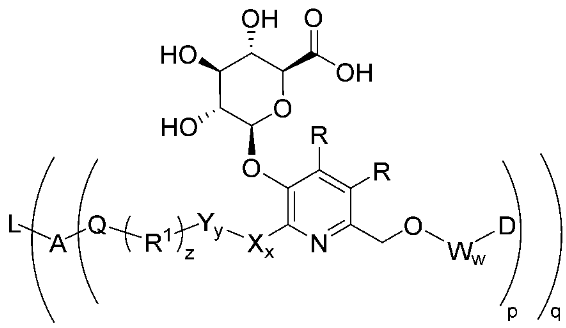

IV

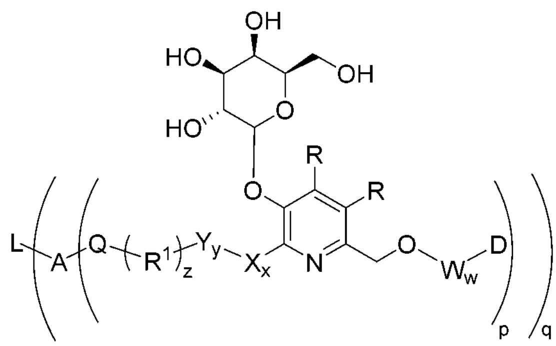

V

wherein, L, A, Q, $R^1$, R, X, Y, W, D, x, y, z, and w are as defined in claim 1.

**4.** The antibody-drug conjugate of formula I according to any one of claims 1 to 3, being an antibody-drug conjugate of formula VI or formula VII:

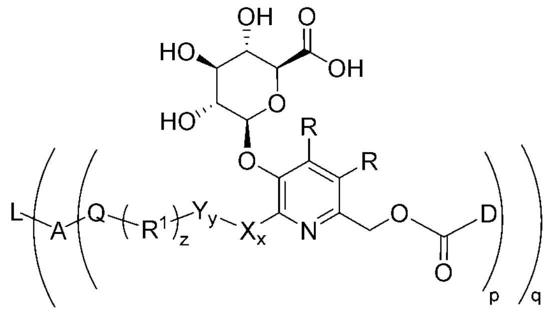

VI

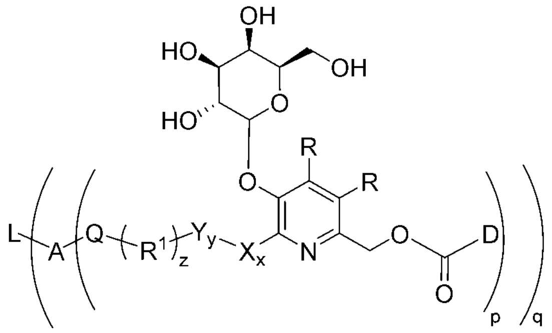

VII

wherein, L, A, Q, $R^1$, R, X, Y, D, x, y, and z are as defined in claim 1.

**5.** The antibody-drug conjugate of formula I according to any one of claims 1 to 4, wherein:

x is 0;
y is 1;
Y is selected from the group consisting of -C(=O)$NR^4$-, -$NR^5$C(=O)-, and -O-;
$R^4$ and $R^5$ are each independently selected from the group consisting of hydrogen and $C_1$-$C_4$ alkyl.

**6.** The antibody-drug conjugate of formula I according to any one of claims 1 to 4, wherein:

x is 1;
y is 1;
X is selected from -$CR^2R^3$-;
Y is selected from the group consisting of -C(=O)$NR^4$-, -$NR^5$C(=O)-, and -O-;
$R^2$ and $R^3$ are each independently selected from the group consisting of hydrogen and $C_1$-$C_4$ alkyl, preferably hydrogen;
$R^4$ and $R^5$ are each independently selected from the group consisting of hydrogen and $C_1$-$C_4$ alkyl.

**7.** The antibody-drug conjugate of formula I according to any one of claims 1 to 6, wherein, $R^1$ is $C_1$-$C_6$ alkylene, preferably $C_1$-$C_6$ linear alkylene, more preferably $C_2$-$C_4$ linear alkylene;
z is 1.

**8.** The antibody-drug conjugate of formula I according to any one of claims 1 to 7, wherein, Q is -$NR^6$-; $R^6$ is each independently selected from the group consisting of hydrogen and $C_1$-$C_4$ alkyl.

**9.** The antibody-drug conjugate of formula I according to any one of claims 1 to 8, wherein, each R is independently selected from the group consisting of hydrogen, halogen and $C_1$-$C_4$ alkyl, preferably hydrogen.

**10.** The antibody-drug conjugate of formula I according to any one of claims 1 to 9, wherein, G has the structure shown below:

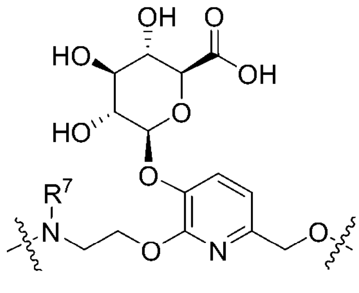

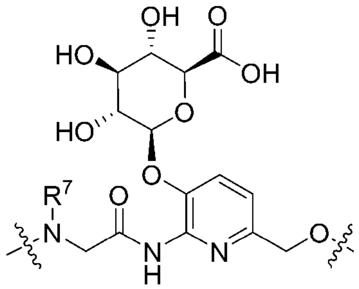

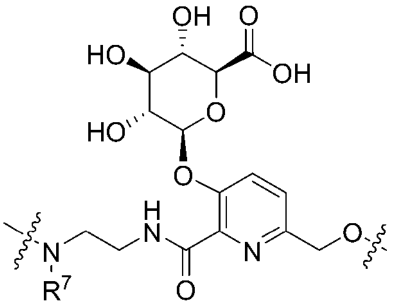

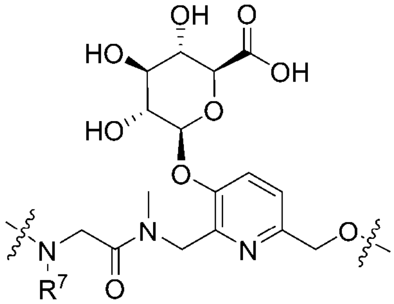

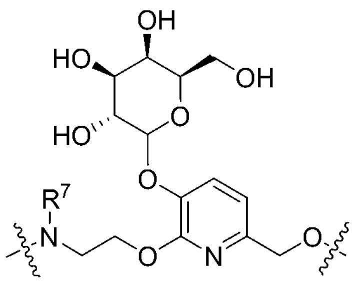

wherein, $R^7$ is selected from the group consisting of hydrogen, $C_{1-4}$ alkyl such as methyl, and a water-soluble group such as polyethylene glycol.

**11.** The antibody-drug conjugate of formula I according to any one of claims 1 to 10, wherein g is 1.

**12.** The antibody-drug conjugate of formula I according to any one of claims 1 to 11, wherein A is selected from the group consisting of the following structures:

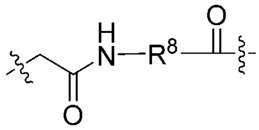 ,

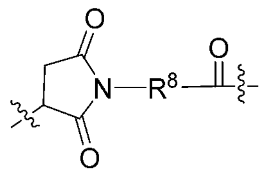 ,

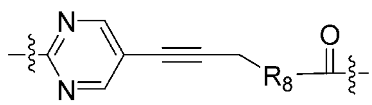 ,

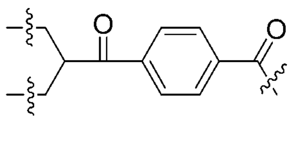 ,

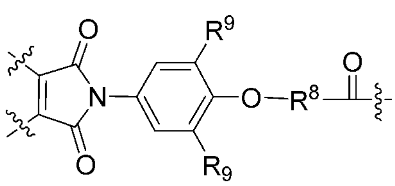 ,

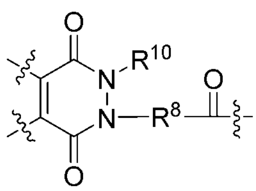 ,

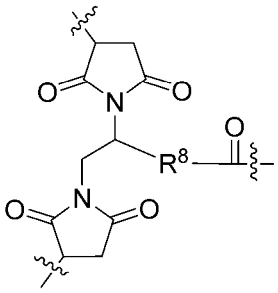
,
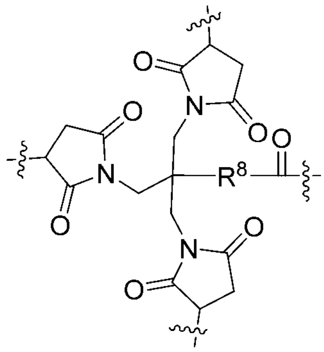
,
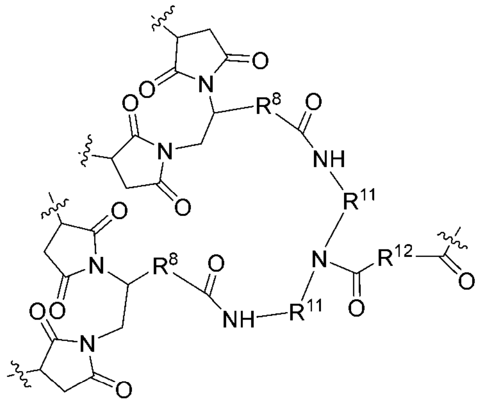

wherein,

$R^8$ is selected from the group consisting of $C_1$-$C_6$ alkylene, and $C_1$-$C_6$ alkylene containing O in the skeleton;
$R^9$ is selected from the group consisting of H and halogen;
$R^{10}$ is selected from the group consisting of $C_1$-$C_4$ alkyl;
$R^{11}$ and $R^{12}$ are each independently selected from the group consisting of $C_1$-$C_6$ alkylene.

**13.** The antibody-drug conjugate of formula I according to any one of claims 1 to 12, wherein L is a humanized antibody, chimeric antibody, or fully human antibody, or antibody fragment, or antibody Fc fusion protein.

**14.** The antibody-drug conjugate of formula I according to any one of claims 1 to 13, wherein L is an antibody or antibody fragment or antibody Fc fusion protein against a cell surface receptor or tumor-associated antigen.

**15.** The antibody-drug conjugate of formula I according to any one of claims 1 to 14, wherein L is a humanized anti-CD30 monoclonal antibody or a humanized anti-CD79b monoclonal antibody.

**16.** The antibody-drug conjugate of formula I according to any one of claims 1 to 15, wherein L is Ab1, which has a heavy chain shown in SEQ ID NO: 1 and a light chain shown in SEQ ID NO: 2.

**17.** The antibody-drug conjugate of formula I according to any one of claims 1 to 15, wherein L is Ab2, which has a heavy chain shown in SEQ ID NO: 3 and a light chain shown in SEQ ID NO: 4.

**18.** The antibody-drug conjugate of formula I according to any one of claims 1 to 17, wherein D is an antitumor drug, a drug for treating autoimmune diseases, or an anti-inflammatory drug; said antitumor drug is, for example, a tubulin inhibitor, a DNA alkylating agent, a DNA minor groove binder, or a DNA topoisomerase inhibitor.

**19.** The antibody-drug conjugate of formula I according to any one of claims 1 to 18, wherein D is Monomethyl Auristatin E (MMAE), SN-38, or Exatecan.

**20.** A compound of formula (A) or a pharmaceutically acceptable salt thereof,

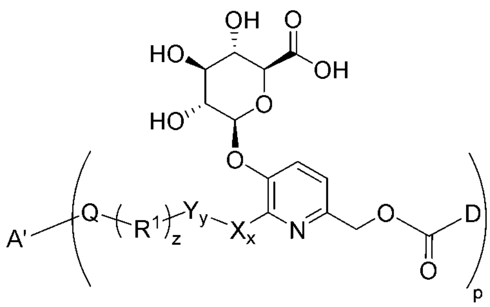

(A)

wherein,

A' is a linker fragment;
each R is independently selected from the group consisting of hydrogen, CN, halogen, $NO_2$, and $C_1$-$C_4$ alkyl, preferably hydrogen, halogen, $C_1$-$C_4$ alkyl, more preferably hydrogen;
X and Y are each independently selected from the group consisting of $-CR^2R^3-$, $-C(=O)NR^4-$, $-NR^5C(=O)-$, and -O-;
Q is selected from the group consisting of $-NR^6-$ and -O-, preferably $-NR^6-$;
$R^1$ is selected from the group consisting of $C_1$-$C_6$ alkylene, and $C_1$-$C_6$ alkylene containing O in the skeleton, preferably $C_1$-$C_6$ alkylene;
$R^2$ and $R^3$ are each independently selected from the group consisting of hydrogen and $C_1$-$C_4$ alkyl, preferably hydrogen;
$R^4$ and $R^5$ are each independently selected from the group consisting of hydrogen and $C_1$-$C_4$ alkyl;
$R^6$ is selected from the group consisting of hydrogen and $C_1$-$C_4$ alkyl;
x is 0 or 1;
y is 0 or 1, preferably 1;
z is 0 or 1, preferably 1;
D is a drug molecule;
p is an integer selected from the group consisting of 1 to 4, preferably 1 or 2.

**21.** The compound of formula (A) or the pharmaceutically acceptable salt thereof according to claim 20, wherein A' is selected from the group consisting of the following structures:

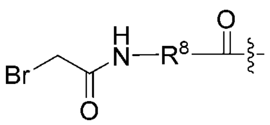 , 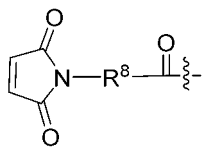 , 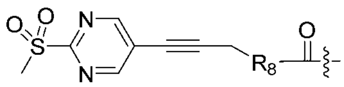 ,

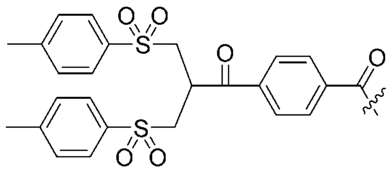 , 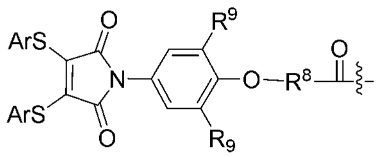 , 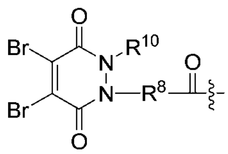 ,

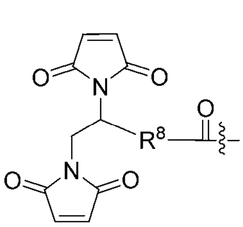 , 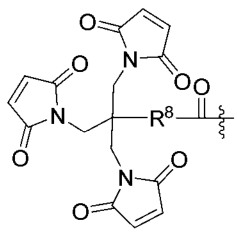 , 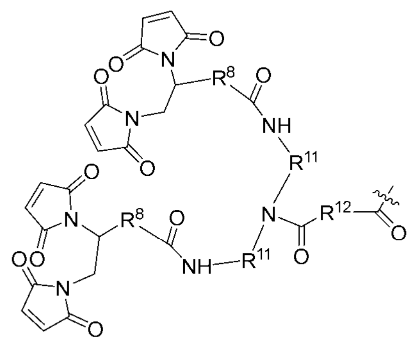

wherein, $R^8$ is selected from the group consisting of $C_1$-$C_6$ alkylene, and $C_1$-$C_6$ alkylene containing O in the skeleton;
$R^9$ is selected from the group consisting of H and halogen;
$R^{10}$ is selected from the group consisting of $C_1$-$C_4$ alkyl;
$R^{11}$ and $R^{12}$ are each independently selected from the group consisting of $C_1$-$C_6$ alkylene.

**22.** The compound of formula (A) or the pharmaceutically acceptable salt thereof according to claim 20 or 21, which is selected from the group consisting of the following structures:

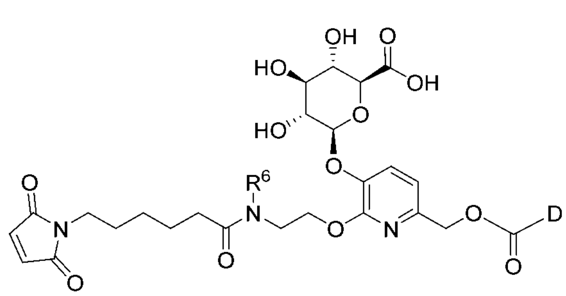

,

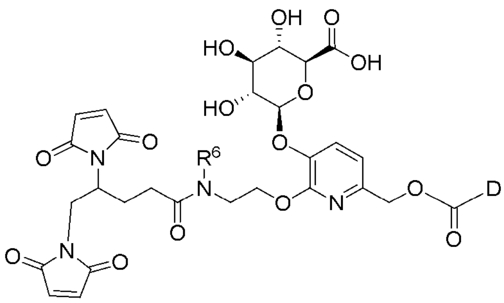

,

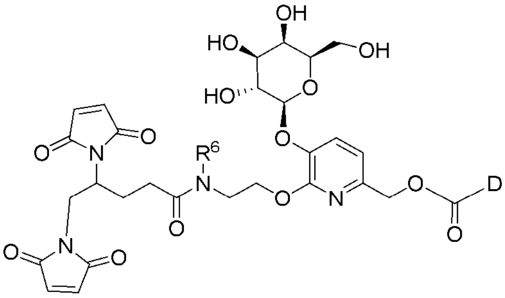

,

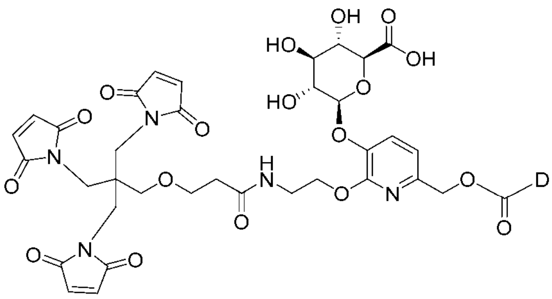

,

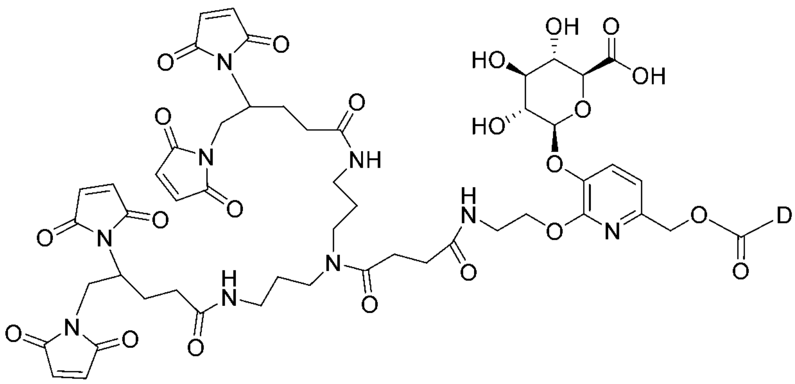

,

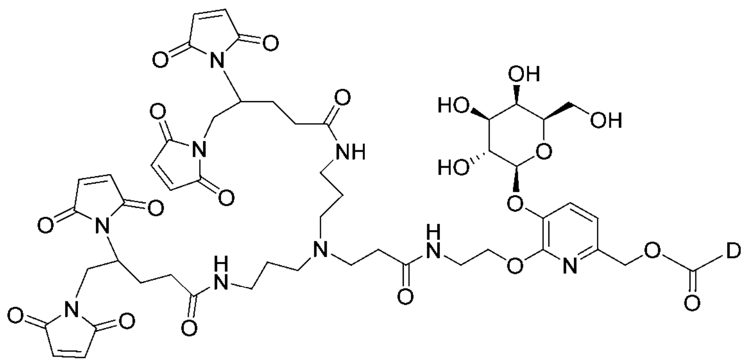

,

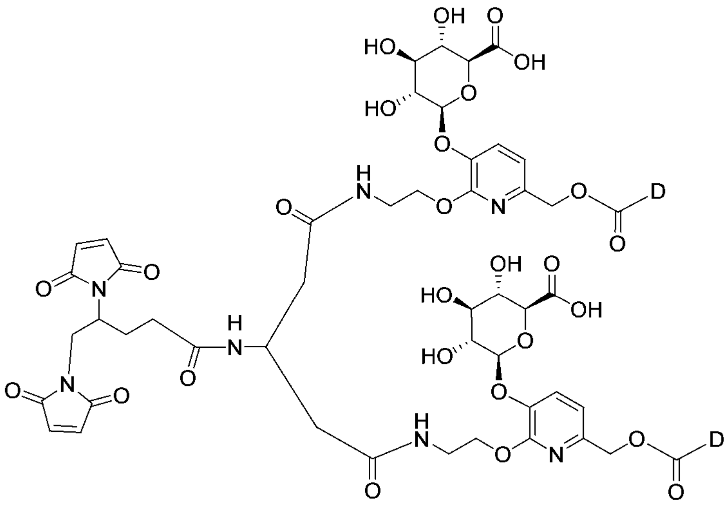

,

wherein, $R^5$, $R^6$ and D are as defined in claim 17.

**23.** The compound of formula (A) or the pharmaceutically acceptable salt thereof according to any one of claims 20 to 22, wherein, D is an antitumor drug, a drug for treating autoimmune diseases, or an anti-inflammatory drug; the antitumor drug is, for example, a tubulin inhibitor, a DNA alkylating agent, a DNA minor groove binder, or a DNA topoisomerase inhibitor; preferably, D is Monomethyl Auristatin E (MMAE), SN-38, or Exatecan.

**24.** The compound of formula (A) or the pharmaceutically acceptable salt thereof according to any one of claims 20 to 23, which is selected from the group consisting of the following structures:

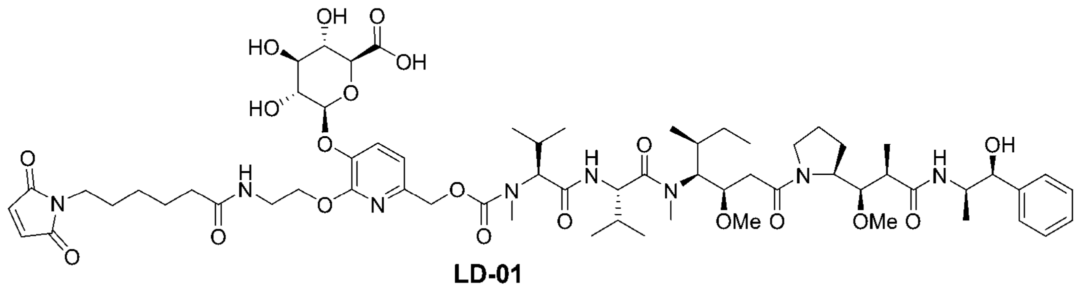

**LD-01**

,

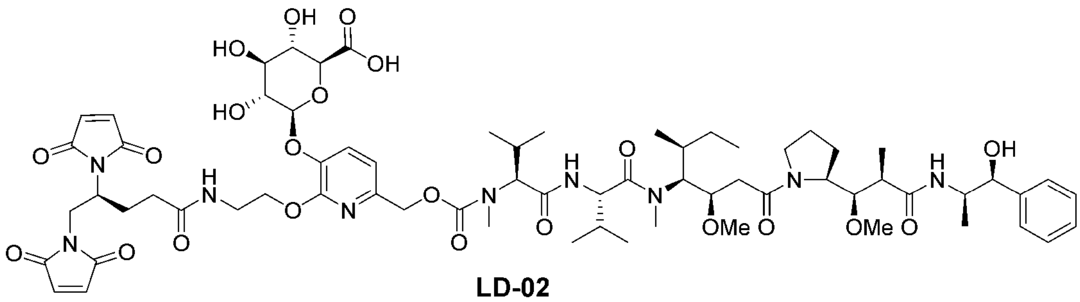

**LD-02**

.

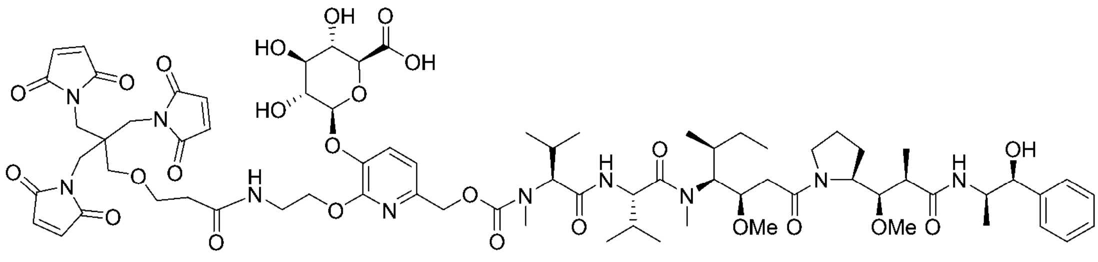

**LD-03**

,

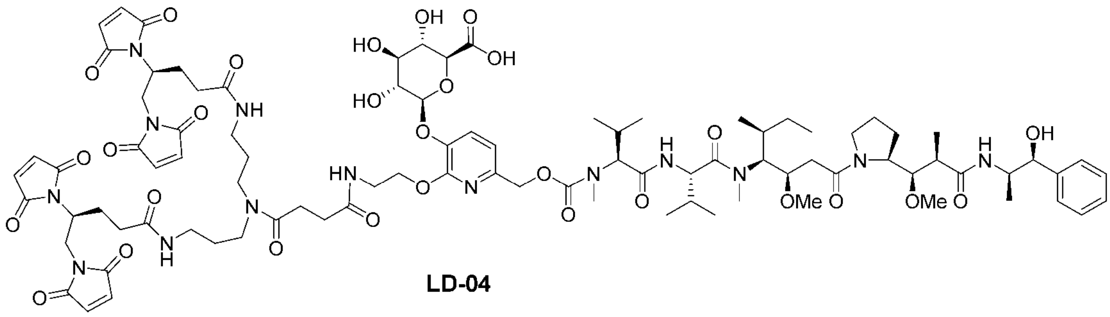

**LD-04**

,

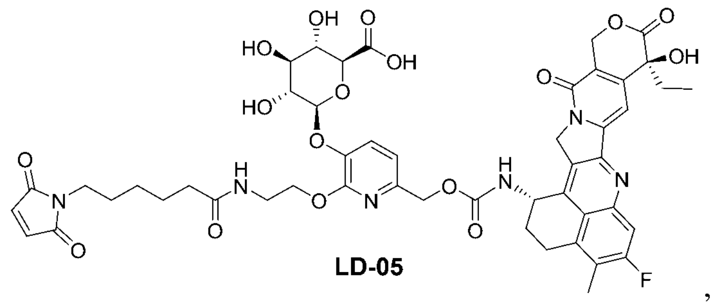

**LD-05**

,

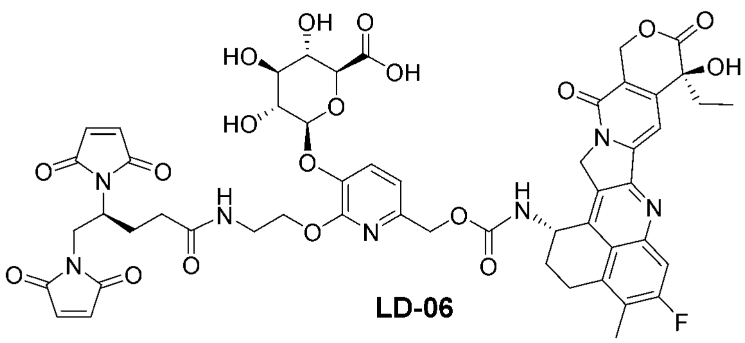

**LD-06**

,

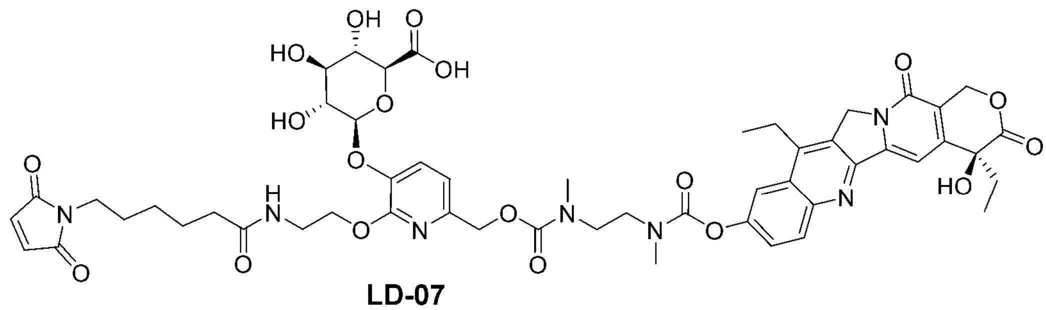

**LD-07**

,

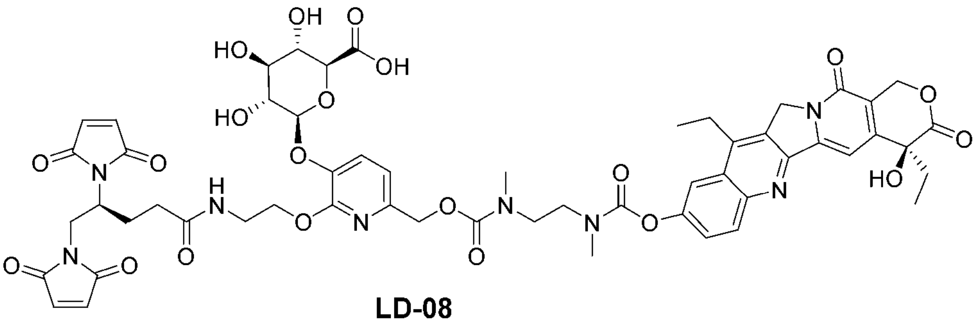

**LD-08**

,

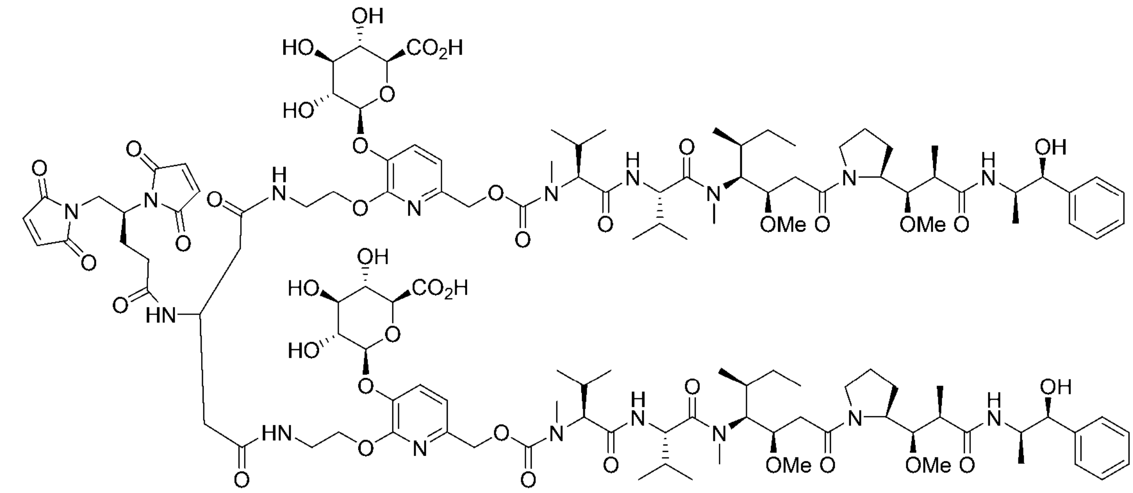

**LD-09**

,

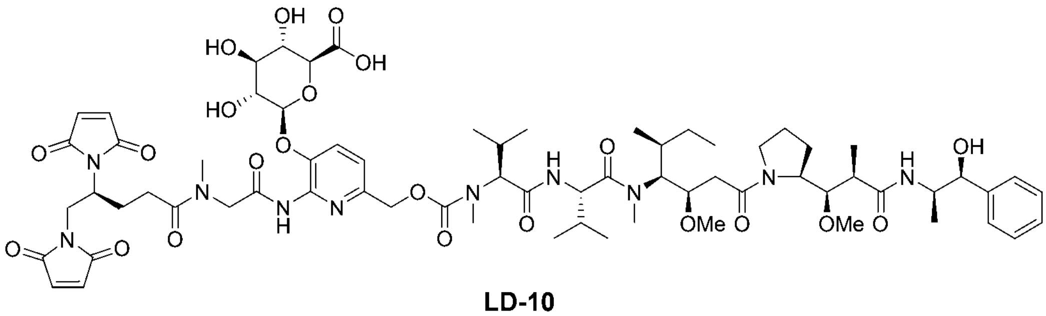

**LD-10**

,

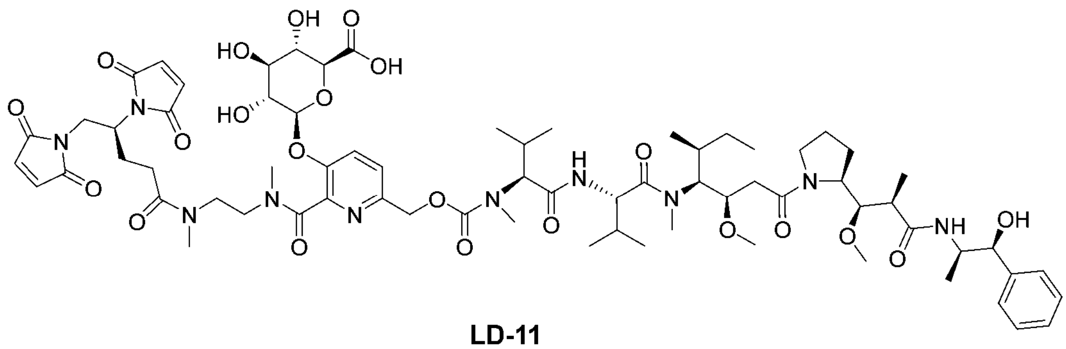

**LD-11**

,

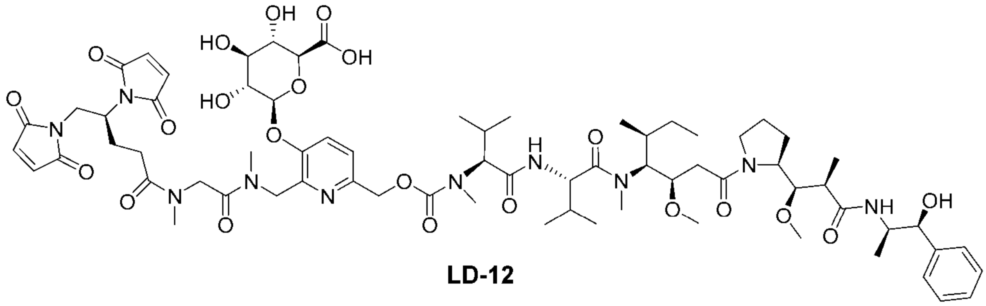

LD-12 ,

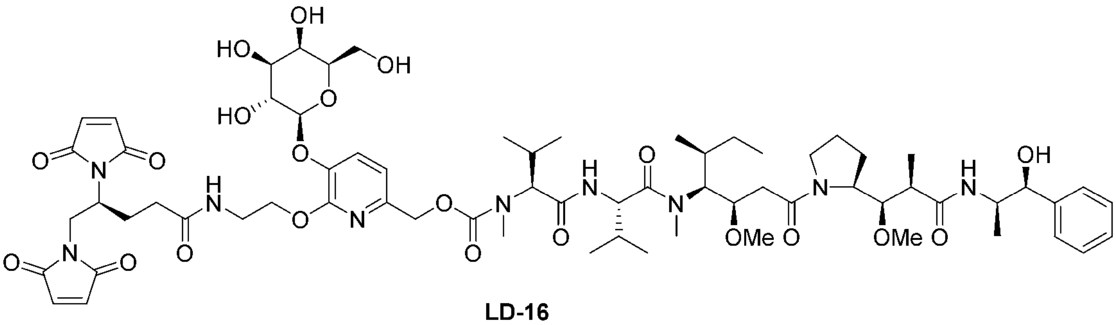

LD-16 ,

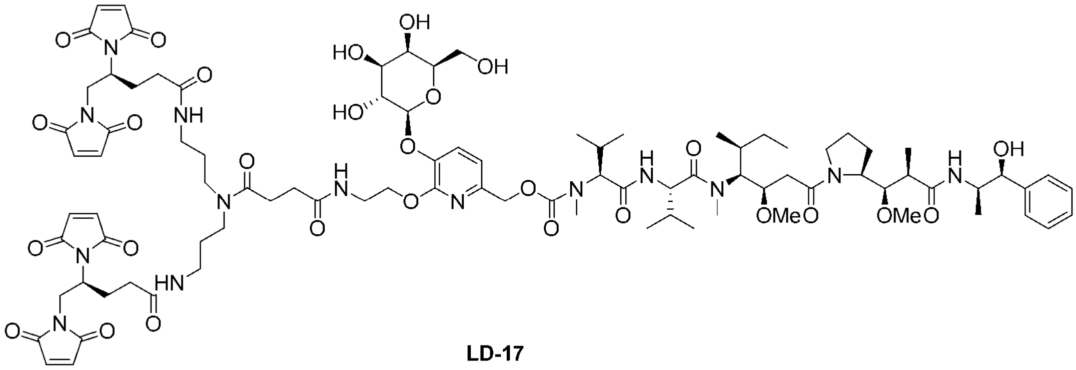

LD-17 .

**25.** A pharmaceutical composition comprising the antibody-drug conjugate of formula I according to any one of claims 1 to 19 and a pharmaceutically acceptable carrier.

**26.** Use of the antibody-drug conjugate of formula I according to any one of claims 1 to 19 or the pharmaceutical composition according to claim 25 in the preparation of a medicament for treating cancer, autoimmune diseases, and inflammatory diseases.

Figure 1A

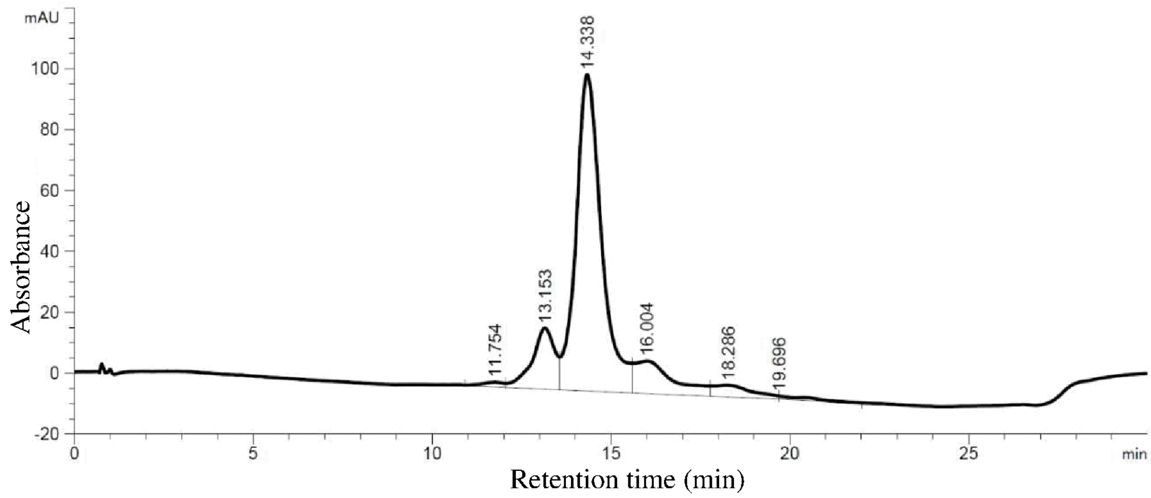


Figure 1B

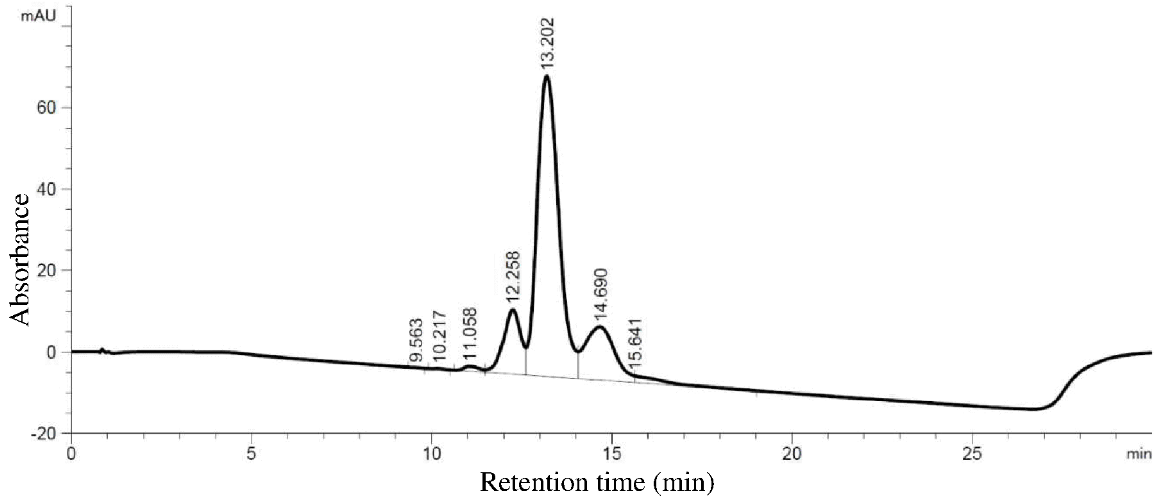


Figure 1C

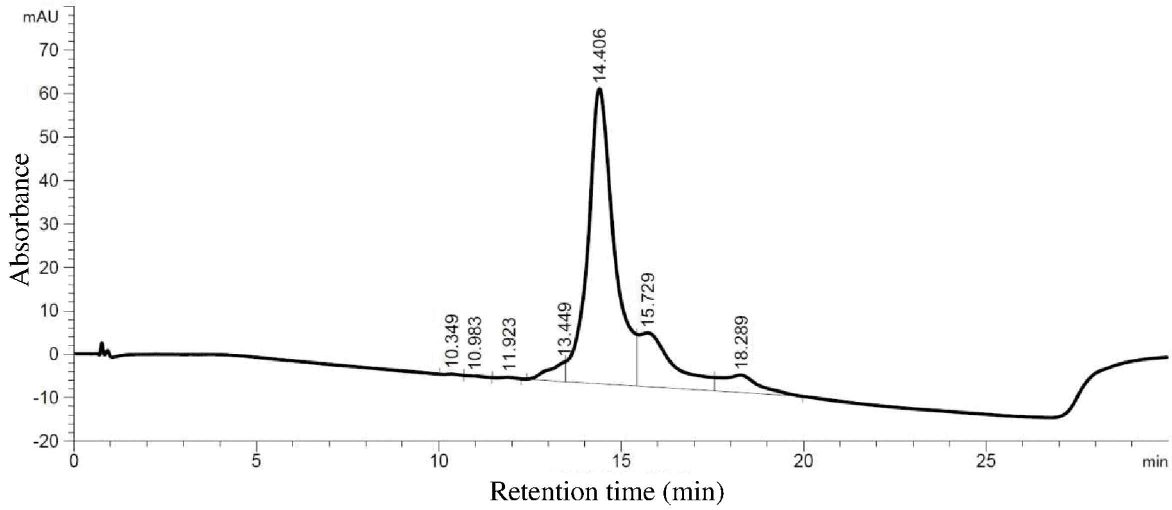

Figure 1D

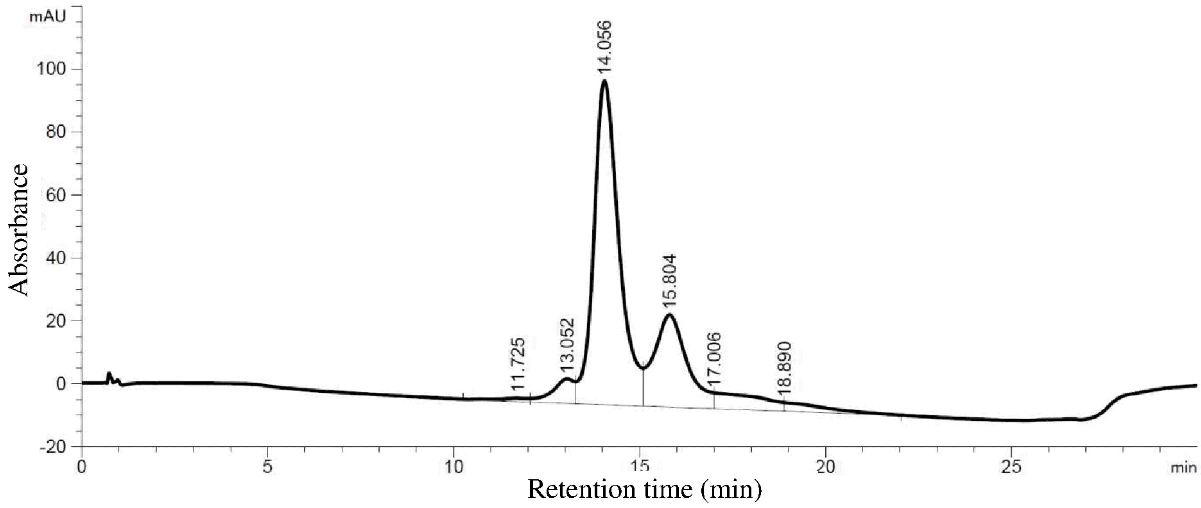


Figure 1E

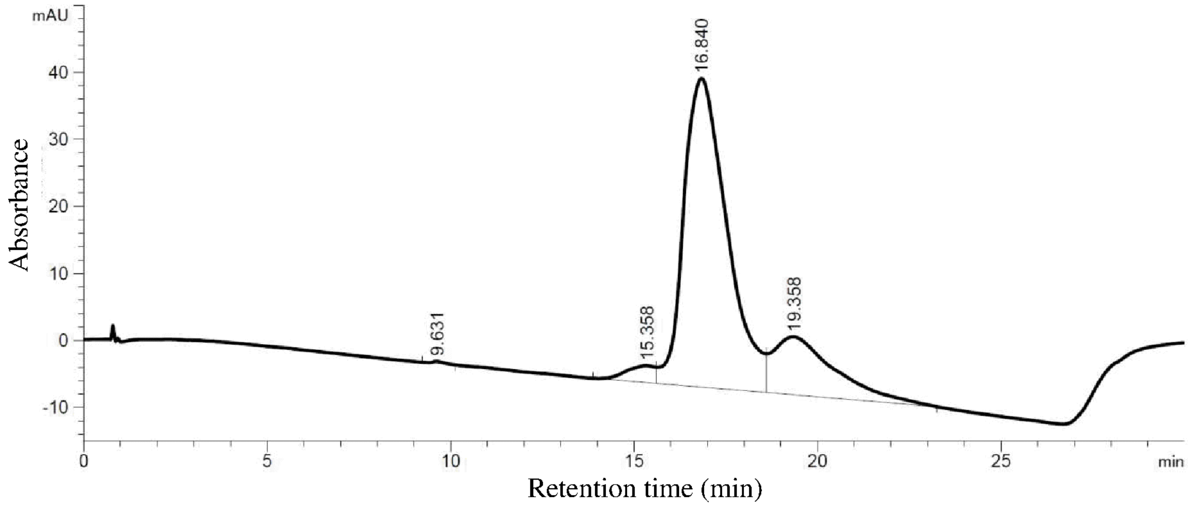


Figure 1F

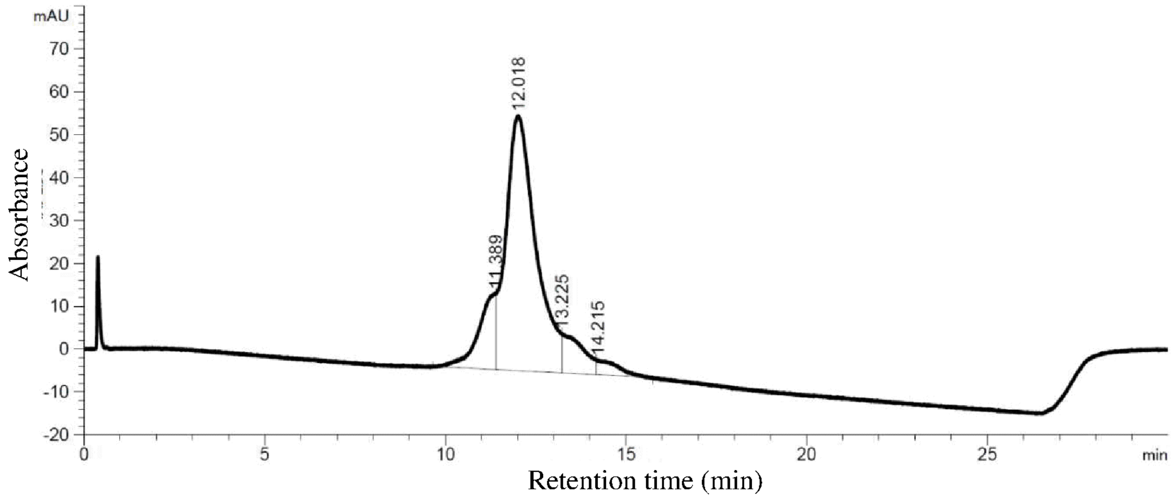

Figure 1G

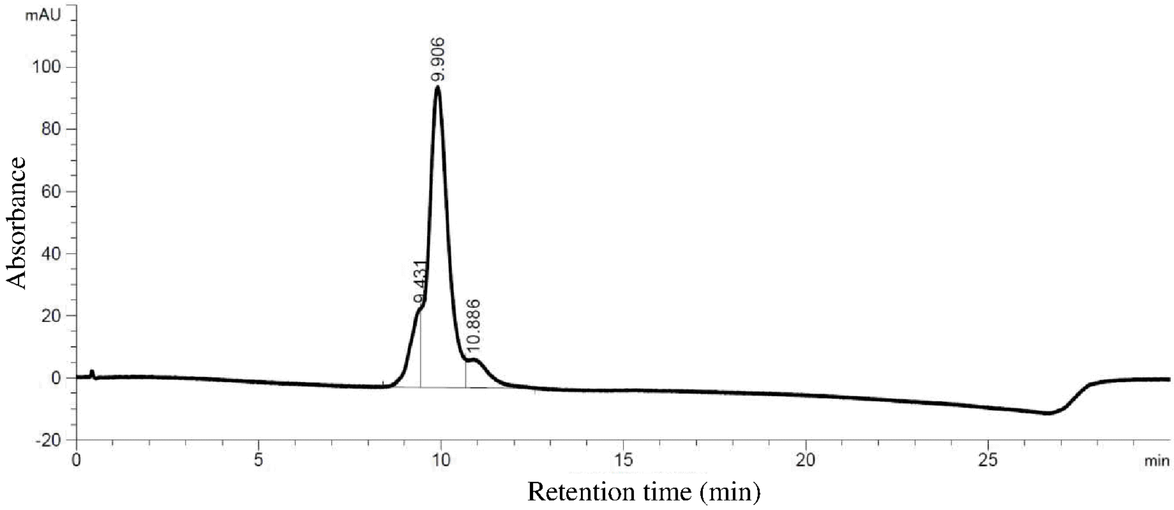


Figure 1H

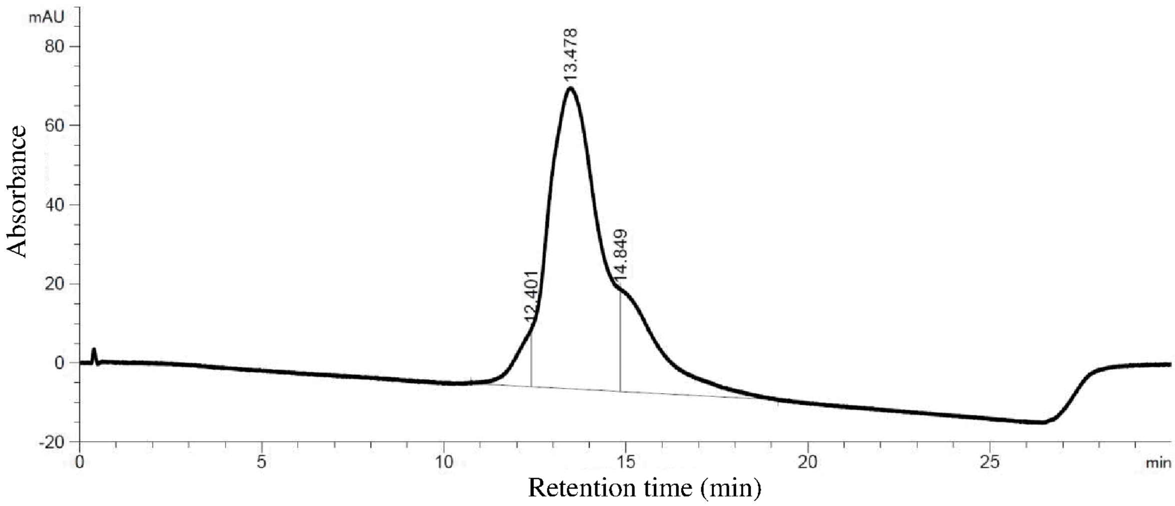


Figure 1I

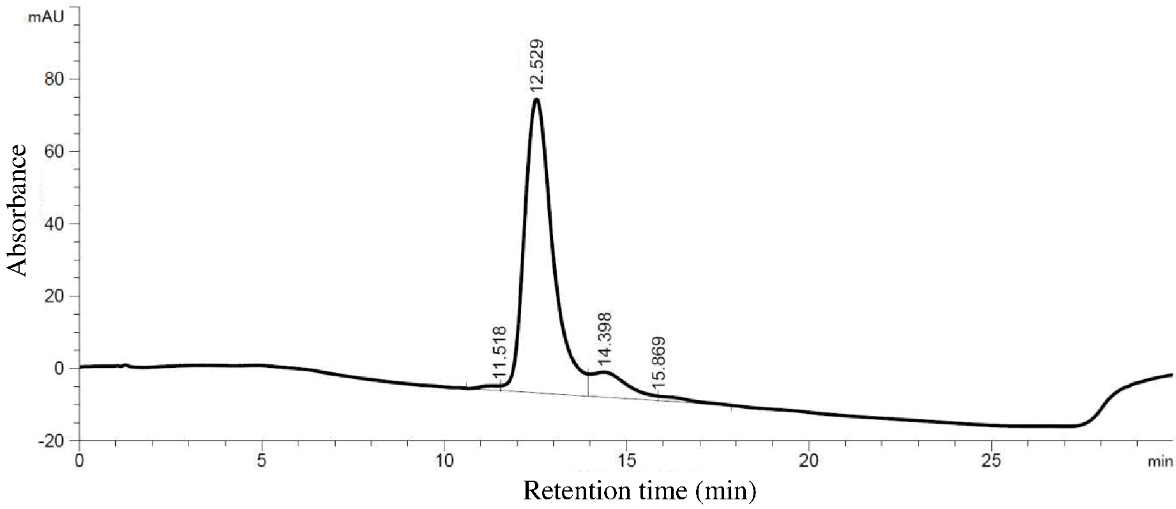

Figure 1J

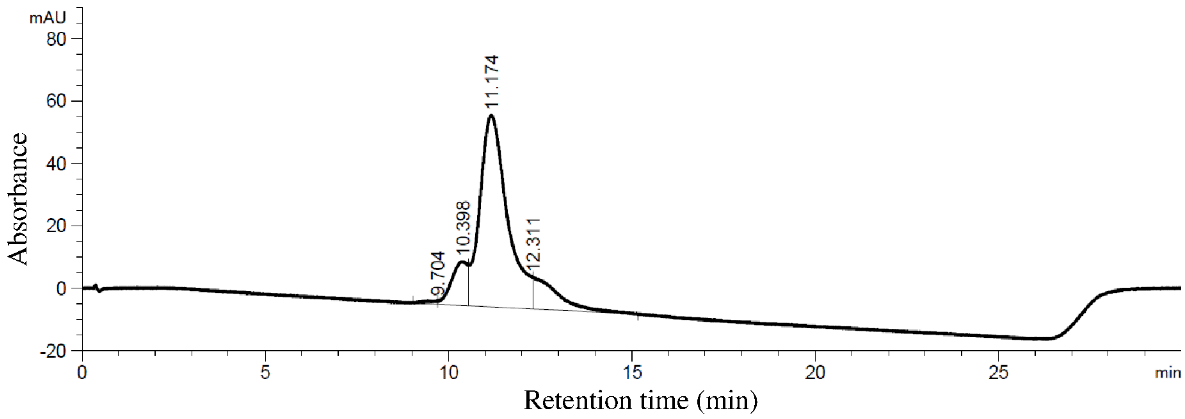


Figure 1K

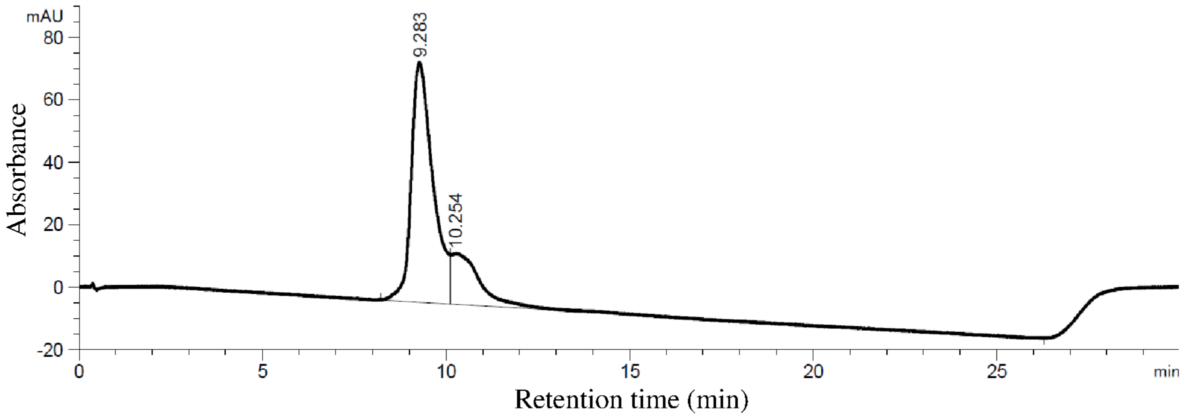

# INTERNATIONAL SEARCH REPORT

International application No.
**PCT/CN2024/098950**

## A. CLASSIFICATION OF SUBJECT MATTER

A61K47/68(2017.01)i; A61P35/00(2006.01)i

According to International Patent Classification (IPC) or to both national classification and IPC

## B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNABS; CNTXT; DWPI; ENTXT; ENTXTC; CNKI; STNext REGISTRY/CAPLUS: 抗体药物偶联物; 连接子; 糖基吡啶; ADC; linker; glycosylpyridine

## C. DOCUMENTS CONSIDERED TO BE RELEVANT

| Category* | Citation of document, with indication, where appropriate, of the relevant passages | Relevant to claim No. |
|---|---|---|
| X | CN 106999605 A (ANTIKOR BIOPHARMA LTD.) 01 August 2017 (2017-08-01) description, paragraphs 0895-0897, and claims 40-43 | 1-14, 18-26 |
| X | CN 112512592 A (SEATTLE GENETICS INC.) 16 March 2021 (2021-03-16) claims 31-34 | 1-14, 18-26 |
| Y | CN 106999605 A (ANTIKOR BIOPHARMA LTD.) 01 August 2017 (2017-08-01) description, paragraphs 0895-0897, and claims 40-43 | 12-16, 18-19, 21-26 |
| Y | CN 112512592 A (SEATTLE GENETICS INC.) 16 March 2021 (2021-03-16) claims 31-34 | 12-16, 18-19, 21-26 |
| Y | CN 111116745 A (SHANGHAI XINLINIAN BIOPHARMACEUTICAL TECHNOLOGY CO., LTD.) 08 May 2020 (2020-05-08) description, paragraphs 0120, 0122, and 0317-0365, and SEQ ID NOs: 33 & 34 | 12-16, 18-19, 21-26 |
| X | CN 105813653 A (SEATTLE GENETICS INC.) 27 July 2016 (2016-07-27) claims 1-73 | 1-14, 18-26 |
| X | CN 111712520 A (SEATTLE GENETICS INC.) 25 September 2020 (2020-09-25) claims 1-59 | 1-14, 18-26 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 September 2024** | **23 September 2024** |

| Name and mailing address of the ISA/CN | Authorized officer |
|---|---|
| **China National Intellectual Property Administration (ISA/CN)<br>China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/098950**

**C. DOCUMENTS CONSIDERED TO BE RELEVANT**

| Category* | Citation of document, with indication, where appropriate, of the relevant passages | Relevant to claim No. |
|---|---|---|
| X | CN 113599533 A (LEGOCHEM BIOSCIENCES, INC.) 05 November 2021 (2021-11-05) claims 1-222 | 1-14, 18-26 |
| A | WO 2018103739 A1 (XDCEXPLORER (SHANGHAI) CO., LTD.) 14 June 2018 (2018-06-14) entire document | 1-26 |

Form PCT/ISA/210 (second sheet) (July 2022)

**INTERNATIONAL SEARCH REPORT**
**Information on patent family members**

International application No.
**PCT/CN2024/098950**

| Patent document cited in search report | Publication date (day/month/year) | Patent family member(s) | Publication date (day/month/year) |
|---|---|---|---|
| CN 106999605 A | 01 August 2017 | JP 2021063124 A | 22 April 2021 |
| | | US 2022233706 A1 | 28 July 2022 |
| | | GB 201416960 D0 | 12 November 2014 |
| | | JP 2018502047 A | 25 January 2018 |
| | | JP 6947630 B2 | 13 October 2021 |
| | | EP 3197501 A1 | 02 August 2017 |
| | | EP 3197501 B1 | 10 April 2024 |
| | | WO 2016046574 A1 | 31 March 2016 |
| | | AU 2015323539 A1 | 06 April 2017 |
| | | AU 2015323539 B2 | 21 January 2021 |
| | | CA 2999384 A1 | 31 March 2016 |
| | | US 2017360951 A1 | 21 December 2017 |
| | | US 11202837 B2 | 21 December 2021 |
| CN 112512592 A | 16 March 2021 | JP 2021527040 A | 11 October 2021 |
| | | JP 7527978 B2 | 05 August 2024 |
| | | EP 3801633 A1 | 14 April 2021 |
| | | JP 2024023543 A | 21 February 2024 |
| | | MX 2020013169 A | 29 March 2021 |
| | | IL 278990 A | 31 January 2021 |
| | | IL 278990 B1 | 01 April 2024 |
| | | IL 278990 B2 | 01 August 2024 |
| | | AU 2019282767 A1 | 07 January 2021 |
| | | CA 3101866 A1 | 12 December 2019 |
| | | WO 2019236954 A1 | 12 December 2019 |
| | | TW 202015740 A | 01 May 2020 |
| | | BR 112020024915 A2 | 09 March 2021 |
| | | SG 11202011915 TA | 30 December 2020 |
| | | IL 311437 A | 01 May 2024 |
| | | US 2023036256 A1 | 02 February 2023 |
| | | KR 20210053871 A | 12 May 2021 |
| CN 111116745 A | 08 May 2020 | EP 3873931 A1 | 08 September 2021 |
| | | EP 3873931 A4 | 29 June 2022 |
| | | CA 3113864 A1 | 07 May 2020 |
| | | JP 2022506310 A | 17 January 2022 |
| | | KR 20210087044 A | 09 July 2021 |
| | | WO 2020088587 A1 | 07 May 2020 |
| | | AU 2019370755 A1 | 20 May 2021 |
| | | BR 112021006919 A2 | 20 July 2021 |
| | | US 2021388082 A1 | 16 December 2021 |
| CN 105813653 A | 27 July 2016 | JP 2020152733 A | 24 September 2020 |
| | | JP 7229202 B2 | 27 February 2023 |
| | | IL 268595 A | 31 October 2019 |
| | | IL 268595 B | 31 May 2021 |
| | | MX 2016007364 A | 08 September 2016 |
| | | IL 246015 A0 | 31 July 2016 |
| | | IL 246015 B | 31 March 2020 |
| | | CA 2932647 A1 | 25 June 2015 |
| | | CA 2932647 C | 14 June 2022 |
| | | JP 2017500299 A | 05 January 2017 |
| | | JP 6701077 B2 | 27 May 2020 |

Form PCT/ISA/210 (patent family annex) (July 2022)

**INTERNATIONAL SEARCH REPORT**
**Information on patent family members**

International application No.
**PCT/CN2024/098950**

| Patent document cited in search report | Publication date (day/month/year) | Patent family member(s) | Publication date (day/month/year) |
|---|---|---|---|
| | | IL 290330 A | 01 April 2022 |
| | | IL 290330 B1 | 01 May 2023 |
| | | IL 290330 B2 | 01 September 2023 |
| | | JP 2023055973 A | 18 April 2023 |
| | | IL 301674 A | 01 May 2023 |
| | | KR 20240010081 A | 23 January 2024 |
| | | US 2016303254 A1 | 20 October 2016 |
| | | US 11116847 B2 | 14 September 2021 |
| | | MX 2021010192 A | 21 September 2021 |
| | | US 2022054650 A1 | 24 February 2022 |
| | | AU 2020281140 A1 | 07 January 2021 |
| | | AU 2020281140 B2 | 08 September 2022 |
| | | KR 20160098303 A | 18 August 2016 |
| | | KR 102442906 B1 | 14 September 2022 |
| | | KR 20220127364 A | 19 September 2022 |
| | | TW 201532616 A | 01 September 2015 |
| | | TWI 727919 B | 21 May 2021 |
| | | ZA 202004099 B | 20 December 2023 |
| | | WO 2015095755 A1 | 25 June 2015 |
| | | NZ 721877 A | 29 July 2022 |
| | | AU 2014369019 A1 | 21 July 2016 |
| | | AU 2014369019 B2 | 17 September 2020 |
| | | ZA 201604564 B | 28 September 2022 |
| | | JP 2019081813 A | 30 May 2019 |
| | | JP 6784790 B2 | 11 November 2020 |
| | | IL 282581 A | 30 June 2021 |
| | | IL 282581 B | 01 March 2022 |
| | | SG 11201610465 PA | 27 January 2017 |
| | | AU 2022283652 A1 | 02 February 2023 |
| CN 111712520 A | 25 September 2020 | AR 114112 A1 | 22 July 2020 |
| | | KR 20200121317 A | 23 October 2020 |
| | | US 2021361776 A1 | 25 November 2021 |
| | | US 11819553 B2 | 21 November 2023 |
| | | SG 11202007073 SA | 28 August 2020 |
| | | IL 276514 A | 30 September 2020 |
| | | IL 276514 B1 | 01 August 2024 |
| | | MX 2020008030 A | 10 September 2020 |
| | | US 2024173427 A1 | 30 May 2024 |
| | | JP 2023089195 A | 27 June 2023 |
| | | EP 3752534 A1 | 23 December 2020 |
| | | EP 3752534 A4 | 01 December 2021 |
| | | CA 3090251 A1 | 22 August 2019 |
| | | WO 2019161174 A1 | 22 August 2019 |
| | | JP 2021513844 A | 03 June 2021 |
| | | JP 7402807 B2 | 21 December 2023 |
| | | TW 201938587 A | 01 October 2019 |
| | | AU 2019220700 A1 | 24 September 2020 |
| CN 113599533 A | 05 November 2021 | AU 2016359235 A1 | 31 May 2018 |
| | | AU 2016359235 B2 | 15 September 2022 |
| | | US 2019151465 A1 | 23 May 2019 |

Form PCT/ISA/210 (patent family annex) (July 2022)

**INTERNATIONAL SEARCH REPORT**
**Information on patent family members**

International application No.
**PCT/CN2024/098950**

| Patent document cited in search report | Publication date (day/month/year) | Patent family member(s) | Publication date (day/month/year) |
|---|---|---|---|
| | | US 11173214 B2 | 16 November 2021 |
| | | EP 3380126 A1 | 03 October 2018 |
| | | EP 3380126 A4 | 24 July 2019 |
| | | CA 3006249 A1 | 01 June 2017 |
| | | WO 2017089895 A1 | 01 June 2017 |
| | | KR 20180078329 A | 09 July 2018 |
| | | JP 2019503981 A | 14 February 2019 |
| | | JP 7327899 B2 | 16 August 2023 |
| | | JP 2022046650 A | 23 March 2022 |
| | | US 2022218840 A1 | 14 July 2022 |
| | | US 11975076 B2 | 07 May 2024 |
| | | TW 201720466 A | 16 June 2017 |
| | | TWI 808931 B | 21 July 2023 |
| WO 2018103739 A1 | 14 June 2018 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5208020 A
- WO 2012061590 A
- US 20120121615 A
- US 7498298 B
- US 20110021568 A
- US 20130224228 A
- WO 2011130616 A
- WO 201751249 A
- US 2019367488 A
- WO 2016192528 A
- WO 2014114207 A
- WO 2019033773 A


### Non-patent literature cited in the description


- *Science*, 1975, vol. 189, 1002-1005
- *Biochemistry*, 2008, vol. 47, 11818-11829
- *Bioconjugate Chem.*, 2010, vol. 21, 5-13